# EUROPÄISCHE PATENTANMELDUNG

(11) **EP 3 995 525 A1**
(43) Veröffentlichungstag der Anmeldung: **11.05.2022**
(21) Anmeldenummer: 20206589.2
(22) Anmeldetag: 10.11.2020
(51) Int. Cl.: C08G 61/08, C08F 220/20, C07C 1/20, F16B 13/00, E21D 20/00, C08K 3/00

(54) **MONOMER MIT ZWEI POLYMERISIERBAREN GRUPPEN (FÜR ROMP UND FÜR RADIKALISCHE POLYMERISATION) UND DESSEN VERWENDUNG**

(71) Anmelder: Hilti Aktiengesellschaft, 9494 Schaan (LI)
(72) Erfinder: Pfeil, Armin, 86899 Landsberg am Lech (DE); Kumru, Memet-Emin, 86199 Augsburg (DE); Slugovc, Christian, 8010 Graz (AT); Wappl, Christina, 8010 Graz (AT)
(74) Vertreter: Hilti Aktiengesellschaft Corporate Intellectual Property

(57) **Zusammenfassung**

Die vorliegende Erfindung betrifft eine Verbindung, welche zwei polymerisierbare Gruppen beinhaltet: eine Gruppe, welche durch ringöffnende Metathese-Polymerisation (ROMP) polymerisiert werden kann, und eine weitere Gruppe, welche durch radikalische Polymerisation polymerisiert werden kann. Die vorliegende Erfindung betrifft auch die Verwendung dieser Verbindung als Monomer in der Polymerisation, beispielsweise als Bestandteil eines Zweikomponenten-Systems für die chemische Befestigung eines Verankerungsmittels in einem Bohrloch, mittels Single Cure Härtung oder mittels Dual Cure Härtung. Die Single Cure Härtung ist eine radikalische Polymerisation, die Dual Cure Härtung ist eine Kombination aus radikalischer Polymerisation und ringöffnender Metathese-Polymerisation (ROMP).

## Beschreibung

Die vorliegende Erfindung betrifft eine Verbindung, welche zwei polymerisierbare Gruppen beinhaltet: eine Gruppe, welche durch ringöffnende Metathese-Polymerisation (ROMP) polymerisiert werden kann, und eine weitere Gruppe, welche durch radikalische Polymerisation polymerisiert werden kann. Die vorliegende Erfindung betrifft auch die Verwendung dieser Verbindung als Monomer in der Polymerisation, beispielsweise als Bestandteil eines Zweikomponenten-Systems für die chemische Befestigung eines Verankerungsmittels in einem Bohrloch, mittels Single Cure Härtung oder mittels Dual Cure Härtung. Die Single Cure Härtung ist eine radikalische Polymerisation, die Dual Cure Härtung ist eine Kombination aus radikalischer Polymerisation und ringöffnender Metathese-Polymerisation (ROMP).

### Hintergrund

Die Verwendung von chemischen Befestigungsmitteln auf Basis radikalisch härtbarer Polymere ist seit langem bekannt. Im Bereich der Befestigungstechnik hat sich die Verwendung von Polymeren als organisches Bindemittel für die chemische Befestigungstechnik, z.B. als Bestandteil einer Dübelmasse ("chemischer Dübel"), durchgesetzt. Es handelt sich bei solchen Dübelmassen um Verbundmassen, die als Mehrkomponenten-Systeme, üblicherweise Zweikomponenten-Systeme ("2K-Systeme") konfektioniert sind, wobei eine Komponente das radikalisch härtbare Monomer und die andere Komponente einen Initiator (für die Radikalbildung) enthält. Andere, übliche Bestandteile, wie beispielsweise Additive, Füllstoffe, Beschleuniger, Inhibitoren, Lösungsmittel und Reaktivverdünner, können in der einen und/oder der anderen Komponente enthalten sein. Durch Vermischen der beiden Komponenten wird dann durch Radikalbildung die Härtungsreaktion, d.h. die Polymerisation, in Gang gebracht und die Mischung zum Duromeren gehärtet.

Als chemische Dübel werden üblicherweise entweder Epoxid-Amin-Systeme oder Methacrylat-basierte Systeme verwendet. Erstere haben einen geringen Polymerisationsschrumpf, härten aber bei tiefen Umgebungstemperaturen, wie sie beispielsweise auf Baustellen vorkommen, nur sehr langsam und oft mit ungenügenden Aushärtegraden aus. Außerdem haben sie bei niedrigen Temperaturen eine hohe Viskosität, was ihre Anwendung auf Baustellen erschwert. Dazu kommt, dass sie kritisch bezüglich der Anwendersicherheit und der Umwelt sind, da sie ätzende Polyaminhärter enthalten und Epoxidharze sensibilisierend und wassergefährdend sein können. Daher werden auf Baustellen in der Regel Methacrylat-basierte Systeme eingesetzt. Als Initiator für die radikalische Härtung von Methacrylat-basierten chemischen Dübeln werden typischerweise Peroxide, wie Diacetylperoxid, Hydroperoxide oder Peroxyester eingesetzt.

Der Schwachpunkt von Methacrylat-basierten chemischen Dübeln ist der signifikante Schrumpf bei der Polymerisation, der zu Scherspannungen und Schwächung des chemischen Dübels führen kann. Polymerisationsschrumpf infolge der dichteren Packung der Atome im Polymer im Vergleich zu den Monomeren schwächt die Bindungsstärke des chemischen Dübels entweder, weil das Polymer sich durch das Schrumpfen von der Bohrlochwand löst, und/oder weil das Polymer trotz des Schrumpfs sowohl an der Bohrlochwand als auch an der zu befestigenden Ankerstange haften bleibt und somit durch das Schrumpfen Spannungen entstehen.

Aufgabe der Erfindung ist es, ein Monomer für die Polymerisation bereitzustellen, welches nur einen geringen Schrumpf verursacht. Ein solche Monomer sollte insbesondere als Monomer für ein Zweikomponenten-System zur Verwendung für die chemische Befestigung eines Verankerungsmittels in einem Bohrloch geeignet sein.

Diese Aufgabe wird durch die Gegenstände der Ansprüche gelöst.

### Beschreibung der Erfindung

Die Erfindung betrifft eine Verbindung, welche zwei polymerisierbare Gruppen umfasst: ein Strukturelement, welches ein gespanntes Cycloolefin ist, und ein weiteres Strukturelement, welches ein Ester einer α,β-ungesättigten Carbonsäure ist. Das erste dieser beiden Strukturelemente kann typischerweise durch ringöffnende Metathese-Polymerisation (Ring Opening Metathesis Polymerisation; ROMP) polymerisiert werden, und das zweite dieser Strukturelemente kann typischerweise durch radikalische Polymerisation polymerisiert werden. Die erfindungsgemäße Verbindung wird daher im Folgenden auch als RadP-ROMP-Monomer bezeichnet. Allerdings sei hier schon festgehalten, dass das gespannte Cycloolefin-Strukturelement vermutlich auch bei einer reinen radikalischen Polymerisation zumindest teilweise an der Polymerisationsreaktion teilnimmt.

Die vorliegende Erfindung betrifft auch die Verwendung der erfindungsgemäßen Verbindung als Monomer in der Polymerisation, beispielsweise als Bestandteil eines Zweikomponenten-Systems für die chemische Befestigung eines Verankerungsmittels in einem Bohrloch.

Die vorliegende Erfindung betrifft des Weiteren ein Zweikomponenten-System, welches eine erfindungsgemäße Verbindung enthält.

Die erfindungsgemäße Verbindung vereint in sich den Vorteil des schnellen Härtens und der hervorragenden Reaktivität auch bei niedriger Härtungstemperatur von radikalinitiierten Systemen auf (Meth)acrylatbasis mit dem Vorteil des geringen Schrumpfs von durch ROMP polymerisierten Systemen. Denn die Kombination der radikalischen Polymerisation mit ROMP führt zu kovalent vernetzten duroplastischen Materialien mit einem Polymerisationsschrumpf, welcher im Vergleich zur radikalischen Polymerisation von (Meth)acrylat-haltigen Monomeren als einzigem Monomer, beispielsweise von Methylmethacrylat, reduziert ist. Dieser Vorteil eines reduzierten Schrumpfs kann sogar erzielt werden, wenn die erfindungsgemäße Verbindung Bestandteil eines Zweikomponenten-Systems ist, welches alleine aufgrund der Anwesenheit eines Radikalinitiators polymerisiert, in welchem also kein ROMP-Initiator vorhanden ist.

Der reduzierte Schrumpf ist insbesondere für die Verankerung von Gewindestangen oder anderen Befestigungselementen in Bohrlöchern (beispielsweise in Beton) vor Vorteil, da durch den reduzierten Schrumpf eine gegenüber reinen Methacrylaten bessere dauerhafte Anbindung an den Untergrund erfolgt.

Des Weiteren erfolgt die schrumpfarme Aushärtung nach Mischen der Komponenten eines erfindungsgemäßen 2K-Systems in der Regel bei Umgebungstemperatur.

Ein erfindungsgemäßes System ist ein Single Cure System, welches auf der durch Vermischen der Komponenten des Systems ausgelösten Initiierung von einer einzigen Polymerisationsreaktion beruht. Erfindungsgemäß ist dies eine Radikal-initiierte Polymerisation (RadP).

Die RadP-Polymerisation erfordert ein Monomer, das radikalisch polymerisiert werden kann, und einen Radikalinitiator, welcher typischerweise mit einem Radikalbeschleuniger kombiniert wird. Der Radikalinitiator sollte im erfindungsgemäßen Single Cure System vorteilhafterweise vom radikalisch polymerisierbaren Monomer getrennt vorliegen, damit erst bei Vermischung der Systemkomponenten die Polymerisation startet.

Ein Gegenstand der Erfindung ist folglich ein Single **Cure-Zweikomponenten-System** umfassend:
eine **Komponente A** umfassend ein erfindungsgemäßes Monomer (RadP-ROMP-Monomer), und optional einen Radikalbeschleuniger (beispielsweise DMpT), und
eine **Komponente B** umfassend einen Radikalinitiator (beispielsweise BPO).

Ein weiteres erfindungsgemäßes System ist ein Dual Cure System. Das erfindungsgemäße Dual Cure System beruht auf der durch Vermischen der Komponenten des Systems ausgelösten Initiierung von zwei verschiedenen Polymerisationsreaktionen: einer Radikalinitiierten Polymerisation (RadP) und einer Ring Opening Metathesis Polymerisation (ROMP). Das erfindungsgemäße Dual Cure System wird daher im Folgenden auch als RadP-ROMP-Dual Cure System bezeichnet.

Die ROMP-Polymerisation erfordert ein ROMP-Monomer, also ein Monomer umfassend ein gespanntes Cycloolefin als Strukturelement, und einen ROMP-Initiator; und die RadP-Polymerisation erfordert ein (Meth)acrylat-haltiges Monomer (bevorzugt ein Methacrylathaltiges Monomer), das radikalisch polymerisiert werden kann, und einen Radikalinitiator, welcher typischerweise mit einem Radikalbeschleuniger kombiniert wird.

Das erfindungsgemäße RadP-ROMP-Dual Cure System erfordert also die Verwendung zweier verschiedener Monomere:
a) eines Monomers umfassend ein gespanntes Cycloolefin als Strukturelement, beispielsweise eines Norbornens; und
b) eines (Meth)acrylat-haltigen Monomers (bevorzugt eines Methacrylat-haltigen Monomers), beispielsweise von Methylmethacrylat.
Im erfindungsgemäßen RadP-ROMP-Monomer liegen die gespannte Cycloolefingruppe und die (Meth)acrylatgruppe als Strukturelemente in einem einzigen Molekül vor, die Verwendung zweier getrennter Monomer (a) und (b) ist daher bei einem erfindungsgemäßen Dual Cure-Zweikomponenten-System, welches das erfindungsgemäße RadP-ROMP-Monomer enthält, nicht zwingend erforderlich. Jedoch können optional neben dem erfindungsgemäßen RadP-ROMP-Monomer noch ein oder mehrere weitere polymerisierbare Monomer vorhanden sein, insbesondere ein wie hierin definiertes ROMP-Monomer und/oder ein wie hierin definiertes (Meth)acrylat.

Das erfindungsgemäße RadP-ROMP-Dual Cure System erfordert des Weiteren die Verwendung zweier verschiedener Initiatoren:
a) eines ROMP-Initiators, beispielsweise eines Grubbs-Katalysators; und
b) eines Radikalinitiators, typischerweise in Kombination mit einem Beschleuniger, beispielsweise von Dibenzoylperoxid (BPO) in Kombination mit *N,N*-Dimethyl-para-toluidin (DMpT).
Diese beiden Initiatoren sollten im RadP-ROMP-Dual Cure System getrennt von den dazu passenden Monomeren vorliegen, damit erst bei Vermischung der Systemkomponenten die Polymerisation startet. Des Weiteren sollte der Radikalinitiator getrennt vom Radikalbeschleuniger vorliegen, damit erst bei Vermischung der Systemkomponenten die Polymerisation startet.

Ein weiterer Gegenstand der Erfindung ist also ein Dual **Cure-Zweikomponenten-System** umfassend:
eine **Komponente A** umfassend ein erfindungsgemäßes RadP-ROMP-Monomer und
einen Radikalinitiator (beispielsweise BPO), und
eine **Komponente B** umfassend einen Initiator für Ring Opening Metathese Polymerisation (ROMP-Initiator), und einen Radikalbeschleuniger (beispielsweise DMpT), und optional ein (Meth)acrylat, bevorzugt ein Methacrylat (beispielsweise Methylmethacrylat oder BDDMA).

Optional kann jedes der erfindungsgemäßen Zweikomponenten-Systeme mit den Komponenten A und B Teil eines Mehrkomponentensystems sein, welches noch weitere Komponenten umfasst. In diesem Fall können Bestandteile der Komponenten A und B auch auf eine oder mehrere der weiteren Komponenten verteilt sein.

Das erfindungsgemäße Zweikomponenten-System bildet bei Vermischen der beiden Komponenten A und B ein Polymer. Es dient vorzugsweise der chemischen Befestigung eines Verankerungsmittels in einem Bohrloch und ist daher in einer bevorzugten Ausführungsform ein chemischer Dübel.

Ein weiterer Gegenstand der Erfindung ist ein Verfahren zur chemischen Befestigung eines Verankerungsmittels in einem Bohrloch, gekennzeichnet dadurch, dass zur Befestigung ein erfindungsgemäßes Zweikomponenten-System verwendet wird.

Ein weiterer Gegenstand der Erfindung ist die Verwendung eines erfindungsgemäßen Zweikomponenten-Systems zur chemischen Befestigung eines Verankerungsmittels in einem Bohrloch, also als chemischer Dübel oder Bestandteil eines chemischen Dübels.

Zum besseren Verständnis der Erfindung werden die folgenden Erläuterungen zur hierin verwendeten **Terminologie** als sinnvoll erachtet.

Die verwendeten **Abkürzungen** bedeuten (weitere Abkürzungen sind ggf. direkt im Text erläutert):
BDDMA: Butan-1,4-diol-dimethylacrylat; BHT: 2,6-Di-tert-butyl-4-methylphenol (butyliertes Hydroxytoluol); BPO: Dibenzoylperoxid; Cp: Cyclopentadien; Cy: Cyclohexan bzw. Cyclohexyl; DCM: Dichlormethan; DiPpT: *N,N*-Diisopropyl-para-toluidin; DMpT: *N,N*-Dimethyl-para-toluidin; eq: Äquivalente; EtOAc: Ethylacetat; HEMA: 2-Hydroxyethylmethacrylat; M:I: Monomer-Initiator-Verhältnis; MES: Mesityl, d.h. 2,4,6-Trimethylphenyl; MMA: Methylmethacrylat; SIMes: 1,3-Bis(2,4,6-trimethylphenyl)-2-imidazolidinyliden; Tempol: 4-Hydroxy-2,2,6,6-tetramethylpiperidinyloxyl; THF: Tetrahydrofuran; TLC: Dünnschichtchromatographie; vol%: Volumenprozent; v/v: Volumenanteil; Gew.-%: Gewichtsprozent; w/w: Gewichtsanteil.

Im Sinne der Erfindung bedeuten die verwendeten Begriffe:
- *"Ring Opening Metathesis Polymerisation"* oder *"ringöffnende Metathese-Polymerisation"* oder *"ROMP"* oder *"ROMP-Polymerisation"* oder *"ringöffnende Olefinmetathese-Polymerisation"* eine Polymerisationsreaktion, der eine Olefinmetathese ausgehend von einer gespannten Cycloolefin-Struktur zugrunde liegt. Dabei dient ein ROMP-Initiator, typischerweise ein Metall-Carben-Komplex als Initiator der Polymerisation. Das gespannte Cycloolefin enthält mindestens eine Doppelbindung im Ring. Die Doppelbindung im Ring erfährt eine Umverteilung mit Hilfe eines Metallacyclobutan-Intermediats, dann findet eine Ringöffnung und Polymerisation statt. Die Triebkraft der Polymerisation ist die Auflösung der Ringspannung. Siehe das folgende Schema: 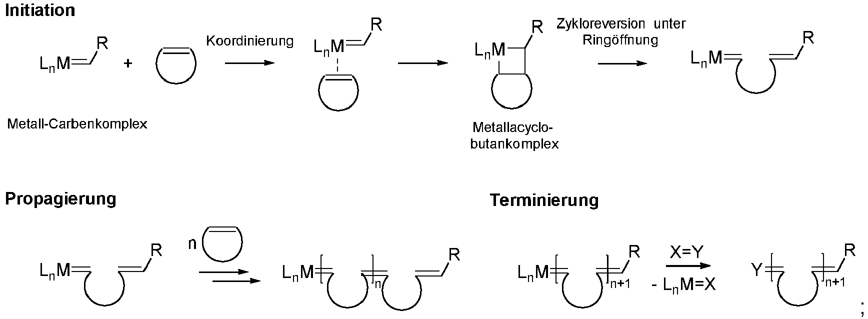
- *"RadP"* oder *"RadP-Polymerisation"* eine radikalisch initiierte Polymerisation. Eine radikalisch initiierte Polymerisation erfordert die Bildung eines Radikals, um die Polymerisation zu starten. Eine typische RadP-Polymerisation ist eine Polymerisation, welche durch Radikalbildung aus BPO als Radikalinitiator in Anwesenheit eines tertiären Amins wie beispielsweise DMpT als Radikalbeschleuniger gestartet wird;
- *"Initiator"* ein Reagenz, das eine Polymerisation in Gang setzt;
- *"Radikalinitiator"* oder *"RadP-Initiator"* ein Reagenz, das (üblicherweise in Kombination mit einem Radikalbeschleuniger) Radikale bildet, welche eine radikalische Polymerisation in Gang setzen;
- *"ROMP-Initiator"* ein Reagenz, das eine ringöffnende Metathese-Polymerisation (ROMP) in Gang setzt und katalysiert, also einen Katalysator für eine ringöffnende Olefinmetathese-Polymerisation; synonym wird hier der Begriff *"ROMP-Katalysator"* verwendet;
- *"Beschleuniger"* oder *"Radikalbeschleuniger"* ein Reagenz, welches mit einem Radikalinitiator eine Reaktion eingeht, so dass bereits bei niedrigen Temperaturen durch den Radikalinitiator größere Mengen an Radikalen erzeugt werden, oder welches die Zerfallsreaktion des Radikalinitiators katalysiert;
- *"Inhibitor"* einen Stoff, der eine unerwünschte radikalische Polymerisation während der Synthese oder der Lagerung einer Monomer-haltigen-Zusammensetzung unterdrückt (diese Stoffe werden in Fachkreisen auch als *"Stabilisator"* bezeichnet) bzw. der eine radikalische Polymerisation nach Zugabe eines Radikalinitiators (üblicherweise in Verbindung mit einem Radikalbeschleuniger) zeitlich verzögert (diese Stoffe werden in Fachkreisen auch als *"Inhibitor"* bezeichnet - die jeweilige Bedeutung des Begriffes erschließt sich aus dem Kontext);
- *"Reaktivverdünner"* flüssige oder niedrigviskose Monomere, welche andere Monomere verdünnen und dadurch die zu deren Applikation notwendige Viskosität verleihen, zur Reaktion befähigte funktionelle Gruppen enthalten und bei der Polymerisation (Härtung) zum überwiegenden Teil Bestandteil der gehärteten Masse (z.B. des Mörtels) werden; Reaktivverdünner werden auch co-polymerisierbares Monomer genannt;
- *"Zweikomponenten-System"* bzw. *"2K-System"* ein System zur Herstellung eines Polymers, das zwei voneinander getrennt gelagerte Komponenten, eine Komponente A und eine Komponente B, umfasst, so dass eine Härtung der in den Komponenten enthaltenen Monomere erst nach dem Vermischen der beiden Komponenten erfolgt;
- *"Mehrkomponenten-System"* ein System zur Herstellung eines Polymers, das mehrere voneinander getrennt gelagerte Komponenten umfasst, so dass eine Härtung der in den Komponenten enthaltenen Monomere erst nach dem Vermischen aller Komponenten erfolgt;
- *"Methacrylat"* eine organische Verbindung, welche eine Methacrylsäureester-Gruppe enthält (beispielsweise Methylmethacrylat, also Methacrylsäuremethylester);
- *"(Meth)acryl...*/*...(meth)acryl...",* dass sowohl die "Methacryl.../...methacryl..."- als auch die "Acryl.../...acryl..."-Verbindungen gemeint sein sollen; bevorzugt sind in der vorliegenden Erfindung "Methacryl.../...methacryl..."-Verbindungen gemeint;
- *"Alkyl"* einen gesättigten Kohlenwasserstoffrest, der verzweigt oder unverzweigt sein kann; bevorzugt ein C₁-C₂₀-Alkyl, bevorzugter ein C₁-C₉-Alkyl. Die Gruppe der C₁-C₂₀-Alkyle umfasst unter anderem Methyl, Ethyl, n-Propyl, iso-Propyl, n-Butyl, 2-Butyl, iso-Butyl, tert-Butyl, n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, n-Pentyl, iso-Pentyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, 2-Ethylhexyl, n-Octyl, iso-Decyl, 2-Propyl-heptyl, Cyclohexyl, iso-Nonyl, iso-Tridecyl, 3,5,5-Trimethyl-1-hexyl und 2-Isopropyl-5-methylhexyl. Besonders bevorzugt ist ein C₁-C₄-Alkyl, also ein Alkyl ausgewählt aus der Gruppe bestehend aus Methyl, Ethyl, n-Propyl, iso-Propyl, n-Butyl, iso-Butyl, und tert-Butyl; Methyl, Ethyl und tert-Butyl sind besonders bevorzugt, und ganz besonders bevorzugt ist Methyl;
- *"Substituent", soweit nicht anders angegeben, einen* Substituenten, welcher keine Kohlenwasserstoffgruppe ist, insbesondere einen Substituenten ausgewählt aus der Gruppe bestehend aus Halogenyl, --OH, =O, -CF₃, -CF₂-CF₃, -SiAlkyl₃, bevorzugt aus der Gruppe bestehend aus -F, -Cl, -OH, =O und -CF₃; *"substituiert",* soweit nicht anders angegeben, dass ein Substituent oder mehrere Substituenten vorhanden sind, bevorzugt nur ein Substituent;
- *"Hydroxyalkyl"* ein Alkyl, das mindestens eine, bevorzugt eine oder zwei Hydroxylgruppe(n), noch bevorzugter nur eine Hydroxylgruppe als Substituenten trägt, bevorzugt an einem Kohlenstoffatom ohne weitere Substituenten;
- *"Alkenyl"* einen ungesättigten Kohlenwasserstoffrest mit mindestens einer, bevorzugt nur einer Doppelbindung, der verzweigt oder unverzweigt (jedoch nicht cyklisch) sein kann; bevorzugt ein C₂-C₂₀-Alkenyl, besonders bevorzugt ein C₂-C₆-Alkenyl, also ein Alkenyl ausgewählt aus der Gruppe bestehend aus Ethenyl, Propenyl, Butenyl, Pentenyl und Hexenyl; Ethenyl, Propenyl und Butenyl sind besonders bevorzugt, und ganz besonders bevorzugt ist Ethenyl;
- *"Hydroxyalkenyl"* ein Alkenyl, das mindestens eine, bevorzugt eine oder zwei Hydroxylgruppe(n) als Substituenten trägt, bevorzugt an einem Kohlenstoffatom ohne weitere Substituenten und ohne Doppelbindung zu einem weiteren Kohlenstoff;
- *"Alkinyl"* einen ungesättigten Kohlenwasserstoffrest mit mindestens einer, bevorzugt nur einer Dreifachbindung, der verzweigt oder unverzweigt sein kann; bevorzugt ein C₂-C₂₀-Alkinyl, besonders bevorzugt ein C₂-C₆-Alkinyl, also ein Alkinyl ausgewählt aus der Gruppe bestehend aus Ethinyl, Propinyl, Butinyl, Pentinyl und Hexinyl; Ethinyl, Propinyl und Butinyl sind besonders bevorzugt, und ganz besonders bevorzugt ist Ethinyl;
- *"Hydroxyalkinyl"* ein Alkinyl, das mindestens eine, bevorzugt eine oder zwei Hydroxylgruppe(n) als Substituenten trägt, bevorzugt an einem Kohlenstoffatom ohne weitere Substituenten und ohne Dreifachbindung zu einem weiteren Kohlenstoff;
- *"Aryl"* bzw. *"Ar"* eine mono- oder bizyklische aromatische Gruppe, bevorzugt mit 6 bis 10 Kohlenstoffatomen. Beispielhafte C₆-C₁₀-Arylgruppen umfassen Phenyl und Naphthyl. Bevorzugt ist das Aryl Phenyl. Die Arylgruppe kann optional mit einem oder mehreren Substituenten unabhängig voneinander ausgewählt aus der Gruppe bestehend aus -F, -Cl, -OH und -CF₃, -C₁-C₆-Alkyl oder -O-C₁-C₆-Alkyl substituiert sein;
- *"Heteroaryl"* eine mono- oder bizyklische aromatische Gruppe, bevorzugt mit 5 bis 10 Ringatomen, bevorzugter mit 5 oder 6 Ringatomen, welche mindestens ein, bevorzugt nur ein oder zwei, besonders bevorzugt nur ein Heteroatom ausgewählt aus S, O, N, bevorzugt N, enthält. Beispielhafte Heteroarylgruppen umfassen Pyrrol, Pyridinyl und Pyrimidinyl. Die Heteroarylgruppe kann optional mit einem oder mehreren Substituenten unabhängig voneinander ausgewählt aus der Gruppe bestehend aus -F, -Cl, -OH und -CF₃, -C₁-C₆-Alkyl oder -O-C₁-C₆-Alkyl substituiert sein;
- *"Arylalkyl"* eine Gruppe aus mindestens einem wie hier weiter oben definierten Aryl mit mindestens einem wie hier weiter oben definierten Alkyl, die über eine Einfachbindung miteinander verknüpft sind. Bevorzugt ist ein Arylalkyl ein -(Alkyl)ₙ-Aryl-(Alkyl)ₙ mit n = 0 oder 1, besonders bevorzugt ist es -(CH₂)ₙ-Phenyl-(CH₂)ₙ mit n = 0 oder 1;
- *"ein", "eine", "einer"* als Artikel vor einer chemischen Verbindungsklasse, z.B. vor dem Wort "Methacrylat", dass eine oder mehrere unter diese chemische Verbindungsklasse fallende Verbindungen, z.B. verschiedene Methacrylate, gemeint sein können. In einer bevorzugten Ausführungsform ist mit diesem Artikel nur eine einzelne Verbindung gemeint;
- *"mindestens ein", "mindestens eine", "mindestens einer"* zahlenmäßig *"ein oder mehrere".* In einer bevorzugten Ausführungsform ist mit diesem Begriff zahlenmäßig *"ein", "eine", "einer"* gemeint;
- *"enthalten", "umfassen"* und *"beinhalten",* dass neben den genannten Bestandteilen noch weitere vorhanden sein können. Diese Begriffe sind einschließlich gemeint und umfassen daher auch *"bestehen aus". "Bestehen aus"* ist abschließend gemeint und bedeutet, dass keine weiteren Bestandteile vorhanden sein können. In einer bevorzugten Ausführungsform bedeuten die Begriffe *"enthalten", "umfassen"* und *"beinhalten"* den Begriff *"bestehen aus";*
- *"etwa"* oder *"circa"* oder *"ca."* vor einem Zahlenwert einen Bereich von ± 10% dieses Wertes, bevorzugt ± 5% dieses Wertes, bevorzugter ± 2% dieses Wertes, bevorzugter ± 1% dieses Wertes, besonders bevorzugt ± 0% dieses Wertes (also genau diesen Wert);
- ein durch Zahlen begrenzter Bereich, z.B. *"von 10 % bis 20 %",* dass die beiden Eckwerte und jeder Wert innerhalb dieses Bereichs einzeln offenbart sind.

Falls eine hierin beschriebene Verbindung mehr als ein Stereoisomer in Form von Tautomeren, Diastereomeren, Enantiomeren, E/Z-Isomeren hat, beispielsweise aufgrund eines asymmetrischen Zentrums oder aufgrund der Möglichkeit einer endo- oder exo-Konfiguration, so sind alle möglichen Stereoisomere und auch Mischungen gemeint, soweit nicht anders angegeben. So kann ein zweifach substituiertes Norbornen beispielsweise in endo/endo, endo/exo, oder exo/exo-Konfiguration vorliegen. Bei Verbindungen, bei denen eine endo/endo, endo/exo, oder exo/exo-Konfiguration möglich ist, ist die endo/exo-Konfiguration bevorzugt. Bei Verbindungen, bei denen eine E- oder Z-Konfiguration möglich ist, insbesondere bei nichtcyklischen Olefinen, welche als eines der Edukte für die Herstellung eines gespannten Cycloolefins mittels Diels-Alder-Reaktion (insbesondere mit einem Cycloalk-1,3-dien) verwendet werden sollen, ist die E-Konfiguration bevorzugt.

Falls in diesem Text Normen (z.B. DIN-Normen) genannt werden, bezieht sich dies auf die zum Anmeldetag dieser Anmeldung aktuelle Ausgabe der jeweiligen Norm.

Im Folgenden wird das erfindungsgemäße Molekül näher erläutert. Anschließend werden die erfindungsgemäßen 2K-Systeme näher erläutert, und darauffolgend die einzelnen Bestandteile der Komponenten A und B der erfindungsgemäßen 2K-Systeme.

### Erfindungsgemäße Verbindung: RadP-ROMP-Monomer

Die erfindungsgemäße Verbindung umfasst zwei polymerisierbare Gruppen: ein Strukturelement, welches ein gespanntes Cycloolefin ist, und ein weiteres Strukturelement, welches ein Ester einer α,β-ungesättigten Carbonsäure ist.

Das erste dieser beiden Strukturelemente kann typischerweise durch ringöffnende Metathese-Polymerisation (Ring Opening Metathesis Polymerisation; ROMP) polymerisiert werden, und das zweite dieser Strukturelemente kann typischerweise durch radikalische Polymerisation polymerisiert werden. Die erfindungsgemäße Verbindung wird daher im Folgenden auch als RadP-ROMP-Monomer bezeichnet.

Das erfindungsgemäße RadP-ROMP-Monomer erlaubt zwei verschiedene Arten der Polymerisation mit demselben Monomermolekül, nämlich Ring Opening Metathesis Polymerisation (ROMP) und Radikalpolymerisation (RadP). Die Herstellung und die Reaktionsmöglichkeiten eines RadP-ROMP-Monomers sind im folgenden Schema beispielhaft für die Verbindung "Mon13" dargestellt. 2-(Methacryloyloxy)ethyl-endo,exo-5-norbornencarboxylat ("Mon13") wird darin in Schritt B hergestellt aus 2 Hydroxyethyl-endo,exo-5-norbornencarboxylat ("Mon9"), hergestellt durch Veresterung des Diels-Alder-Produkts aus 2-Hydroxyethylacrylat (HEA) und Cyclopentadien (Cp) (Schritt A). Anschließend werden ROMP (Schritt C - z.B. unter Verwendung eines Grubbs-Katalysators) und Radikalpolymerisation (RadP) (Schritt D - z.B. unter Verwendung von Benzoylperoxid) gleichzeitig gestartet:

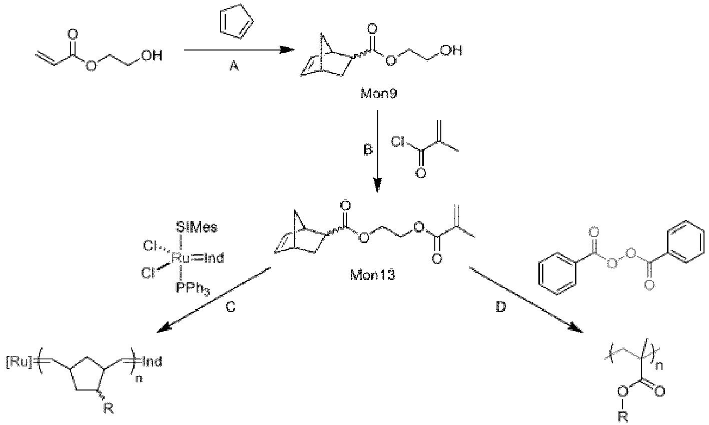

Durch die zwei verschiedenen im Molekül vorhandenen funktionellen Gruppen wird Crosslinking erreicht, wie hier beispielhaft für zwei in den Beispielen beschriebenen RadP-ROMP-Monomere, nämlich Mon13 und Mon14, dargestellt:

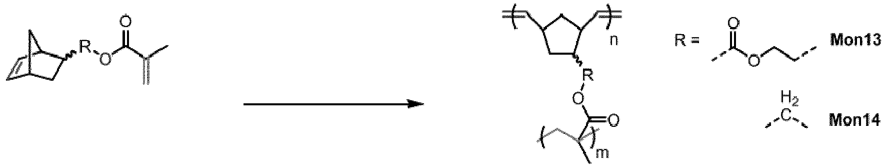

Im Rahmen der vorliegenden Erfindung konnte zudem gezeigt werden, dass das gespannte Cycloolefin anscheinend zumindest teilweise auch an einer radikalischen Polymerisation als Reaktionspartner beteiligt ist.

Die α,β-ungesättigte Carbonsäure, welche im RadP-ROMP-Monomer verestert ist, ist bevorzugt ausgewählt aus der Gruppe bestehend aus verzweigten und unverzweigten C₃-C₂₀-α,β-ungesättigten Carbonsäuren, bevorzugter aus der Gruppe bestehend aus verzweigten und unverzweigten C₄-C₁₀-α,β-ungesättigten Carbonsäuren, noch bevorzugter aus der Gruppe bestehend aus verzweigten und unverzweigten C₄-C₆-α,β-ungesättigten Carbonsäuren. Besonders bevorzugt ist sie ausgewählt aus der Gruppe bestehend aus Tiglinsäure ((E)-2,3-Dimethylacrylsäure), Sorbinsäure (Hexadiensäure), Crotonsäure (trans-Butensäure), und Methacrylsäure. Noch bevorzugter ist sie Methacrylsäure.

Mit "gespanntes Cycloolefin" sind im Rahmen der vorliegenden Erfindung alle Cycloolefine mit Ausnahme des Cyclohexens bezeichnet. Cyclohexen kann nicht mit einer ringöffnenden Metathese polymerisiert werden. Gespannte Cycloolefine, welche für vorliegende Erfindung geeignet sind, werden beispielsweise in EP 0 771 830 A2 und in US 7,691,919 B2, Spalten 11 bis 13, gezeigt.

Das gespannte Cycloolefin-Strukturelement hat mindestens eine Doppelbindung im Ring, bevorzugt eine oder zwei Doppelbindungen, bevorzugter nur eine Doppelbindung. Umfasst das gespannte Cycloolefin-Strukturelement zwei Doppelbindungen, so haben diese kein gemeinsames Kohlenstoffatom und sind bevorzugt nicht konjugiert, noch bevorzugter Teil verschiedener Ringe, oder im Falle von verbrückten Systemen Teil verschiedener Brücken, innerhalb des gespannten Cycloolefin-Strukturelements.

Das gespannte Cycloolefin-Strukturelement kann mindestens ein Heteroatom, bevorzugt nur ein Heteroatom, ausgewählt aus N, O, Si, P und S, bevorzugt aus N, O und S, bevorzugter O, als Ringatom enthalten. Bevorzugt sind jedoch alle Ringatome Kohlenstoffatome oder nur eines der Ringatome ist durch O ersetzt (Oxa-Derivat), noch bevorzugter sind alle Ringatome Kohlenstoffatome. Im Falles eines Oxa-Derivats ersetzt -O- ein -CH₂-, im Falle eines Sulfa-Derivats ersetzt -S- ein -CH₂-. N, Si und P können mit zwei oder mehr Kohlenstoff-Ringatomen verknüpft sein, die verbleibenden Valenzen sind dann mit H-Atomen oder Substituenten verbunden (z.B. -NH- oder -N(CH₃)-).

Das gespannte Cycloolefin-Strukturelement ist entweder monocyklisch oder polycyklisch mit zwei oder mehr, bevorzugt zwei bis vier kondensierten, anellierten und/oder verbrückten Ringen. Es umfasst bevorzugt 3 bis 16 Ringatome, bevorzugter 3 bis 14 Ringatome, noch bevorzugter 5 bis 12, noch bevorzugter 5, 7 oder 8 Ringatome, noch bevorzugter 5 oder 7 Ringatome. Falls das gespannte Cycloolefin(-Strukturelement) monocyclisch ist, umfasst es bevorzugt 3 bis 8 Ringatome, bevorzugter 5 oder 7 Ringatome; falls es polycyclisch, insbesondere bicyklisch ist, umfasst es bevorzugt 7 bis 14 Ringatome, bevorzugter 7 bis 12 Ringatome, noch bevorzugter 7 bis 9 Ringatome. Bei polycyklischen gespannten Cycloolefin(-Strukturelement)en sind Bicyclo[x,y,z]-olefine, Tricyclo[w.x.y.z]-olefine und Tetracyclo[v.w.x.y.z]-olefine bevorzugt, in denen v, w, x, y und z unabhängig voneinander eine ganze Zahl von 0 bis 6 sind, bevorzugt eine ganze Zahl von 1 bis 3 sind, bevorzugter eine ganze Zahl von 1 bis 2 sind. Von diesen sind Bicyclo[x,y,z]-olefine besonders bevorzugt, insbesondere für x = 2, y = 2, z = 1 oder 2.

Außerdem kann mindestens ein Arylring, bevorzugt ein Benzolring, an das gespannte Cycloolefin-Strukturelement ankondensiert sein.

Das gespannte Cycloolefin-Strukturelement ist bevorzugt monocyklisch oder bicyklisch, bevorzugt ein monocyklisches (beispielsweise Cyclopenten) oder verbrücktes bicyklisches gespanntes Cycloolefin (beispielsweise Norbornen). Ein bicyklisches gespanntes Cycloolefin ist bevorzugt ein bicyklisches gespanntes Cycloolefin welches durch Diels-Alder-Reaktion eines monocyklischen 1,3-Diens mit einem Olefin gebildet wird. Ist dieses Olefin zweifach substituiert, so hat es bevorzugt E-Konfiguration, und das resultierende gespannte Cycloolefin hat endo/exo-Konfiguration, wie in der folgenden beispielhaften Reaktion gezeigt:

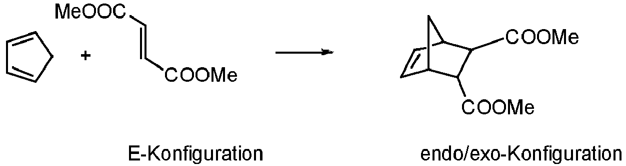

Durch Diels-Alder-Reaktion eines Cycloolefins mit zwei Doppelbindungen im Ring (beispielsweise Cyclopentadien) mit einem Cycloolefin entstehende weitere gespannte Cycloolefine (beispielsweise das aus Cyclopentadien und Norbornen entstehende Tetracyclododecen, oder Dicyclopentadien) fallen ebenfalls in die Gruppe der bevorzugten gespannten Cycloolefin-Strukturelemente. Hierzu gehören insbesondere: 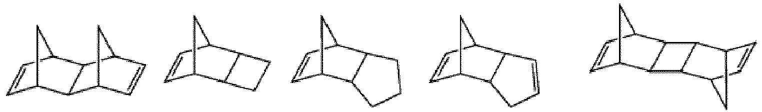 und deren Oxa-Derivate.

Das gespannte Cycloolefin-Strukturelement ist in einer Ausführungsform ausgewählt aus der Gruppe bestehend aus Tricyclo[5.2.1.0^{2,6}]deca-3,8-dien (Dicyclopentadien), Tetracyclo[6.2.1.1.0]-dodecen, Bicyclo[3.3.2]-decen, Bicyclo[3.2.2]-nonen, Bicyclo[2.2.2]-octen, Bicyclo[2.2.1]-heptadien (Norbornadien), Bicyclo[2.2.1]-hepten (Norbornen) und Bicyclo[2.2.0]-hexen, und aus deren Oxa-Derivaten.

Bevorzugt ist das gespannte Cycloolefin-Strukturelement ein Cyclopenten, ein Norbornen, ein Norbornadien, ein Cycloocten, ein Tricyclo[5.2.1.0^{2,6}]deca-3,8-dien (Dicyclopentadien) oder ein Tetracyclo[6.2.1.1.0]-dodecen, oder ein Oxa-Derivat davon, bevorzugter ein Norbornen, ein Norbornadien, ein Tricyclo[5.2.1.0^{2,6}]deca-3,8-dien (Dicyclopentadien), oder ein Tetracyclo[6.2.1.1.0]-dodecen, oder ein Oxa-Derivat davon, noch bevorzugter ein Norbornen oder Norbornadien, oder ein Oxa-Derivat davon, noch bevorzugter ein Norbornen.

Das RadP-ROMP-Monomer hat in einer bevorzugten Ausführungsform die folgende Formel (III): 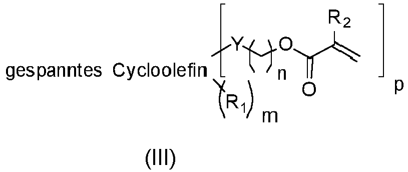 worin:
- gespanntes Cycloolefin ein wie oben definiertes gespanntes Cycloolefin-Strukturelement bedeutet;
- Y ausgewählt ist aus der Gruppe bestehend aus einer Einfachbindung, -C₁-C₂₀-Alkandiyl-, -O-C₁-C₂₀-Alkandiyl-, -C₁-C₂₀-Alkandiyl-O-, -O-C₁-C₂₀-Alkandiyl-O-, -O-,-Si(Alkyl)₂-, -C₁-C₂₀-Alkandiyl-Si(Alkyl)₂-, -C₁-C₂₀-Alkandiyl-Si(Alkyl)₂--C₁-C₂₀-Alkandiyl-,-C₁-C₂₀-Alkandiyl-O-C₁-C₂₀-Alkandiyl-, -C(=O)-O-, -C₁-C₂₀-Alkandiyl--C(=O)-O-,-C(=O)-O-C₁-C₂₀-Alkandiyl-, -C(=O)-O-C₁-C₂₀-Alkandiyl-O-C(=O)-, -C₁-C₂₀-Alkandiyl-C(=O)-O-C₁-C₂₀-Alkandiyl-, -O-C(=O)-, -O-C(=O)-O-, -C₁-C₂₀-Alkandiyl-O-C(=O)-,-O-C(=O)-C₁-C₂₀-Alkandiyl-, -O-C(=O)-C₁-C₂₀-Alkandiyl-C(=O)-O-, -Aryldiyl-, -O-Aryldiyl-, -O-Aryldiyl-O-, -Aryldiyl-O-Aryldiyl-,-C(=O)-O-Aryldiyl-, -C(=O)-O-Aryldiyl-O-C(=O)-, -Aryldiyl-C(=O)-O-Aryldiyl-, -O-C(=O)-Aryldiyl-, -O-C(=O)-Aryldiyl-C(=O)-O-, -C₆-C₂₀-Arylalkandiyl-O-C₆-C₂₀-Arylalkandiyl-, -O-C₆-C₂₀-Arylalkandiyl-O-, -C₆-C₂₀-Arylalkandiyl-O-C₆-C₂₀-Arylalkandiyl-, -C(=O)-O-C₆-C₂₀-Arylalkandiyl-, -C(=O)-O-C₃-C₂₀-Arylalkandiyl-O-C(=O)-, -C₆-C₂₀-Arylalkandiyl-C(=O)-O-C₆-C₂₀-Arylalkandiyl-, -O-C(=O)-C₆-C₂₀-Arylalkandiyl-, -O-C(=O)-C₆-C₂₀-Arylalkandiyl-C(=O)-O-, -(CH₂)_{q}-C=C-(CH₂)_{q}-, worin q unabhängig voneinander eine ganze Zahl von 1 bis 20, bevorzugt von 1 bis 10, noch bevorzugter von 1 bis 2 sind, -(CH₂)_{q}-O-(CH₂)_{q}-, worin q unabhängig voneinander eine ganze Zahl von 1 bis 20, bevorzugt von 1 bis 10, noch bevorzugter von 1 bis sind, -(CH₂)_{q}-NR₄-(CH₂)_{q}-, worin q unabhängig voneinander eine ganze Zahl von 1 bis 20, bevorzugt von 1 bis 10, noch bevorzugter von 1 bis sind, und R₄ H oder ein substituiertes oder unsubstituiertes C₁-C₂₀-Alkyl ist, bevorzugter H oder ein unsubstituiertes C₁-C₂₀-Alkyl ist, noch bevorzugter H ist.

Bevorzugt ist Y ausgewählt aus der Gruppe bestehend aus einer Einfachbindung, -C₁-C₈-Alkandiyl-, -O-C₁-C₈-Alkandiyl-, -C₁-C₈-Alkandiyl-O-, -O-C₁-C₈-Alkandiyl-O-, -O-,-SiMe₂-, -C₁-C₈-Alkandiyl-SiMe₂-, -C₁-C₈-Alkandiyl-SiMe₂--C₁-C₈-Alkandiyl-, -C₁-C₈-Alkandiyl-O-C₁-C₈-Alkandiyl-, -C(=O)-O-, -C₁-C₈-Alkandiyl-C(=O)-O-, -C(=O)-O-C₁-C₈-Alkandiyl-, -C(=O)-O-C₁-C₈-Alkandiyl-O-C(=O)-, -C₁-C₈-Alkandiyl-C(=O)-O-C₁-C₈-Alkandiyl-, -O-C(=O)-, -O-C(=O)-O-, -C₁-C₈-Alkandiyl-O-C(=O)-, -O-C(=O)-C₁-C₈-Alkandiyl-, -O-C(=O)-C₁-C₈-Alkandiyl-C(=O)-O-, -C₆-C₁₀-Aryldiyl-, -O-C₆-C₁₀-Aryldiyl-, -O-C₆-C₁₀-Aryldiyl-O-, -C₆-C₁₀-Aryldiyl-O-C₆-C₁₀-Aryldiyl-,-C(=O)-O-C₆-C₁₀-Aryldiyl-,-C(=O)-O-C₆-C₁₀-Aryldiyl-O-C(=O)-, -C₆-C₁₀-Aryldiyl-C(=O)-O-C₆-C₁₀-Aryldiyl-,-O-C(=O)-C₆-C₁₀-Aryldiyl-, -O-C(=O)-C₆-C₁₀-Aryldiyl-C(=O)-O-, -C₇-C₁₂-Arylalkandiyl-O-C₇-C₁₂-Arylalkandiyl-, -O-C₇-C₁₂-ArylAlkandiyl-O-, -C₇-C₁₂-ArylAlkandiyl-O-C₇-C₁₂-Arylalkandiyl-,-C(=O)-O-C₇-C₁₂-Arylalkandiyl-, -C(=O)-O-C₇-C₁₂-Arylalkandiyl-O-C(=O)-, -C₇-C₁₂-Arylalkandiyl-C(=O)-O-C₇-C₁₂-Arylalkandiyl-, -O-C(=O)-C₇-C₁₂-Arylalkandiyl-, -O-C(=O)-C₇-C₁₂-Arylalkandiyl-C(=O)-O-, -(CH₂)_{q}-C=C-(CH₂)_{q}-, worin q unabhängig voneinander eine ganze Zahl von 1 bis 20, bevorzugt von 1 bis 10, noch bevorzugter von 1 bis 2 sind, -(CH₂)_{q}-O-(CH₂)_{q}-, worin q unabhängig voneinander eine ganze Zahl von 1 bis 20, bevorzugt von 1 bis 10, noch bevorzugter von 1 bis sind,-(CH₂)_{q}-NR₄-(CH₂)_{q}-, worin q unabhängig voneinander eine ganze Zahl von 1 bis 20, bevorzugt von 1 bis 10, noch bevorzugter von 1 bis sind, und R₄ H oder ein substituiertes oder unsubstituiertes C₁-C₂₀-Alkyl ist, bevorzugter H oder ein unsubstituiertes C₁-C₂₀-Alkyl ist, noch bevorzugter H ist.

Bevorzugter ist Y ausgewählt aus der Gruppe bestehend aus einer Einfachbindung,-C₁-C₈-Alkandiyl-, -O-C₁-C₈-Alkandiyl-, -C₁-C₈-Alkandiyl-O-, -O-C₁-C₈-Alkandiyl-O-,-C₁-C₈-Alkandiyl-O-C₁-C₈-Alkandiyl-, -C(=O)-O-, -C₁-C₈-Alkandiyl-C(=O)-O-, -C(=O)-O-C₁-C₈-Alkandiyl-, -C(=O)-O-C₁-C₈-Alkandiyl-O-C(=O)-, -C₁-C₈-Alkandiyl-C(=O)-O-C₁-C₈-Alkandiyl-, -O-C(=O)-, -C₁-C₈-Alkandiyl-O-C(=O)-, -O-C(=O)-C₁-C₈-Alkandiyl-,-O-C(=O)-C₁-C₈-Alkandiyl-C(=O)-O-, -C₆-C₁₀-Aryldiyl-, -O-C₆-C₁₀-Aryldiyl-.

Noch bevorzugter ist Y ausgewählt aus der Gruppe bestehend aus -C₁-C₈-Alkandiyl-,-C₁-C₈-Alkandiyl-O-, -C(=O)-O-, -C₁-C₈-Alkandiyl--C(=O)-O-, -C(=O)-O-C₁-C₈-Alkandiyl-, -O-C(=O)-, -C₁-C₈-Alkandiyl-O-C(=O)-.

Noch bevorzugter ist Y ausgewählt aus der Gruppe bestehend aus -C(=O)-O-,-C₁-C₂-Alkandiyl-, -C₁-C₂-Alkandiyl-O-, -C(=O)-O-C₁-C₂-Alkandiyl-, -C₁-C₂-Alkandiyl-C(=O)-O-, -C₁-C₂-Alkandiyl-O-C(=O)-. Besonders bevorzugt ist Y -CH₂-, -CH₂-O-, -C(=O)-O-, -CH₂-C(=O)-O-, -CH₂-O-C(=O)-, oder -C(=O)-O-CH₂-. Ganz besonders bevorzugt ist Y -CH₂-O-, -C(=O)-O-, -CH₂-C(=O)-O-, oder -CH₂-O-C(=O);
- m eine ganze Zahl von 0 bis 2 ist, bevorzugt 0 oder 1 ist; wenn m 2 ist, dann sind die beiden R₁ bevorzugt gleich;
- n eine ganze Zahl von 1 bis 10 ist, bevorzugt von 2 bis 6 ist, bevorzugter 2 ist;
- p eine ganze Zahl von 1 bis 2 ist, bevorzugt 1 ist;
- R₁ unabhängig voneinander ausgewählt sind aus der Gruppe bestehend aus -C(=O)-OH, -C(=O)-O-C₁-C₂₀-Alkyl, -O-C(=O)-C₁-C₂₀-Alkyl, -C(=O)-O-Aryl, -O-C(=O)-Aryl,-C₁-C₂₀-Alkyl, -Aryl, -C₁-C₂₀-Hydroxyalkyl, -C₁-C₂₀-Alkandiyl-O-C₁-C₂₀-Alkyl, Halogenyl, -CF₃, -CF₂-CF₃, und -SiAlkyl₃. Bevorzugt ist R₁ ein -C(=O)-OH, -C(=O)-O-C₁-C₈-Alkyl, -O-C(=O)-C₁-C₈-Alkyl, -C₁-C₈-Alkyl, -C₁-C₈-Hydroxyalkyl, oder -C₁-C₈-Alkandiyl-O-C₁-C₈-Alkyl, noch bevorzugter ein -C(=O)-OH, -C(=O)-O-C₁-C₄-Alkyl, -O-C(=O)-C₁-C₄-Alkyl, -C₁-C₄-Alkyl, -C₁-C₄-Hydroxyalkyl, oder -C₁-C₄-Alkandiyl-O-C₁-C₄-Alkyl, noch bevorzugter ein -C(=O)-OH, -C(=O)-O-C₁-C₄-Alkyl, -O-C(=O)-C₁-C₄-Alkyl, -C₁-C₄-Alkyl, -CH₂-OH, oder -CH₂-O-C₁-C₄-Alkyl. Noch bevorzugter ist R₁ ausgewählt aus der Gruppe bestehend aus -C(=O)-OH, -C(=O)-O-Methyl, -O-C(=O)-Methyl, Methyl, Ethyl, n-Propyl, iso-Propyl, n-Butyl, iso-Butyl, und tert-Butyl, -CH₂-OH, -CH₂-O-Methyl, 2-Hydroxyethyl, 3-Hydroxy-n-propyl, 2-Hydroxy-isopropyl, 4-Hydroxy-n-Butyl, 3-Hydroxy-isobutyl, und 2-Hydroxy-tert-butyl. Methyl, Ethyl, -C(=O)-O-Methyl, -O-C(=O)-Methyl, -CH₂-OH und 2-Hydroxyethyl sind besonders bevorzugt, und ganz besonders bevorzugt ist Methyl oder -CH₂-OH.

Zwei an nebeneinanderliegenden Kohlenstoffatome gebundene R₁ können auch miteinander verknüpft sein und so mit den Kohlenstoffatomen, an welche sie gebunden sind, einen Ring bilden, bevorzugt einen Ring mit 5 bis 7 Ringatomen. Beispielsweise kann so ein Cyclopentanring entstehen, oder ein Lactonring.

Alternativ bilden zwei R₁ zusammen eine Brücke der Formel -(CH₂)_{z}- welche zwei Cycloolefin-Strukturelemente miteinander verbindet, worin z eine ganze Zahl von 1 bis 4, bevorzugt von 1 bis 3, bevorzugter von 1 bis 2, besonders bevorzugt 1 ist, oder eine Brücke der Formel -(CH₂)_{q}-Ar-(CH₂)_{q}- welche zwei Cycloolefin-Strukturelemente miteinander verbindet, worin Ar ein Aromat ist, bevorzugt Phenyl, q unabhängig voneinander eine ganze Zahl von 0 bis 2, bevorzugt von 0 bis 1, noch bevorzugter beide 0 sind, oder eine Brücke der Formel -(CH₂)_{q}-C=C-(CH₂)_{q}- welche zwei Cycloolefin-Strukturelemente miteinander verbindet, worin q unabhängig voneinander eine ganze Zahl von 0 bis 2, bevorzugt von 0 bis 1, noch bevorzugter beide 0 sind, oder eine Brücke der Formel -(CH₂)_{q}-O-(CH₂)_{q}- welche zwei Cycloolefin-Strukturelemente miteinander verbindet, worin q unabhängig voneinander eine ganze Zahl von 0 bis 2, bevorzugt von 0 bis 1, noch bevorzugter beide 0 sind, oder eine Brücke der Formel -(CH₂)_{q}-NR₄-(CH₂)_{q}- welche zwei Cycloolefin-Strukturelemente miteinander verbindet, worin q unabhängig voneinander eine ganze Zahl von 0 bis 2, bevorzugt von 0 bis 1, noch bevorzugter beide 0 sind, und R₄ H oder ein substituiertes oder unsubstituiertes C₁-C₂₀-Alkyl ist, bevorzugter H oder ein unsubstituiertes C₁-C₂₀-Alkyl ist, noch bevorzugter H ist, oder eine Brücke der Formel -C(=O)-O-(CH₂)_{z}-O-(O=)-C- welche zwei Cycloolefin-Strukturelemente miteinander verbindet, worin z eine ganze Zahl von 1 bis 4, bevorzugt von 1 bis 3, bevorzugter von 1 bis 2, besonders bevorzugt 2 ist, oder eine Brücke der Formel -C(=O)-O-, oder eine Brücke der Formel -C(=O)-O-Ar-O-(O=)-C- welche zwei Cycloolefin-Strukturelemente miteinander verbindet, worin Ar ein Aromat, bevorzugt Phenyl ist, z eine ganze Zahl von 1 bis 4, bevorzugt von 1 bis 3, bevorzugter von 1 bis 2, besonders bevorzugt 1 ist, oder eine Brücke der Formel -(CH₂)_{q}-Si(R₄)₂-(CH₂)_{q}- welche zwei Cycloolefin-Strukturelemente miteinander verbindet, worin q unabhängig voneinander eine ganze Zahl von 0 bis 2, bevorzugt von 0 bis 1, noch bevorzugter mindestens einmal 1 sind, und R₄ H oder ein substituiertes oder unsubstituiertes C₁-C₂₀-Alkyl ist, bevorzugter H oder ein unsubstituiertes C₁-C₂₀-Alkyl ist, noch bevorzugter ein unsubstituiertes C₁-C₄-Alkyl ist;
- R₂ ein C₁-C₁₇-Alkyl oder C₁-C₁₇-Hydroxyalkyl ist. Bevorzugt ist R₂ ein C₁-C₈-Alkyl oder C₂-C₈-Hydroxyalkyl, noch bevorzugter ein C₁-C₄-Alkyl oder C₂-C₄-Hydroxyalkyl. Noch bevorzugter ist R₂ ausgewählt aus der Gruppe bestehend aus Methyl, Ethyl, n-Propyl, iso-Propyl, n-Butyl, iso-Butyl, und tert-Butyl, 2-Hydroxyethyl, 3-Hydroxy-n-propyl, 2-Hydroxy-isopropyl, 4-Hydroxy-n-Butyl, 3-Hydroxy-isobutyl, und 2-Hydroxy-tert-butyl; Methyl, Ethyl und 2-Hydroxyethyl sind besonders bevorzugt, und ganz besonders bevorzugt ist Methyl.

In einer bevorzugten Ausführungsform (Variante) hat das RadP-ROMP-Monomer die Formel (III): 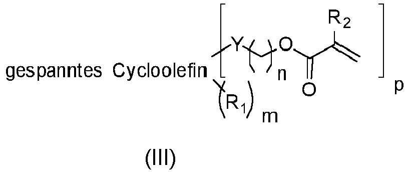 worin:
- gespanntes Cycloolefin ausgewählt ist aus der Gruppe bestehend aus Cyclopenten, Norbornen, Norbornadien, Cycloocten, Tetracyclo[6.2.1.1.0]-dodecen und aus deren Oxa-Derivaten, in welchen ein C-Atom im gespannten Cycloolefin-Strukturelement durch O ersetzt ist. Bevorzugt ist es ausgewählt aus der Gruppe bestehend aus Norbornen, Norbornadien, Tricyclo[5.2.1.0^{2,6}]deca-3,8-dien (Dicyclopentadien), Tetracyclo[6.2.1.1.0]-dodecen, und aus deren Oxa-Derivaten, in welchen ein C-Atom im gespannten Cycloolefin-Strukturelement durch O ersetzt ist;
- Y ausgewählt ist aus der Gruppe bestehend aus einer Einfachbindung, -C₁-C₈-Alkandiyl-, -O-C₁-C₈-Alkandiyl-, -C₁-C₈-Alkandiyl-O-, -O-C₁-C₈-Alkandiyl-O-, -C₁-C₈-Alkandiyl-O-C₁-C₈-Alkandiyl-, -C(=O)-O-, -C₁-C₈-Alkandiyl-C(=O)-O-, -C(=O)-O-C₁-C₈-Alkandiyl-, -C(=O)-O-C₁-C₈-Alkandiyl-O-C(=O)-, -C₁-C₈-Alkandiyl-C(=O)-O-C₁-C₈-Alkandiyl-, -O-C(=O)-, -C₁-C₈-Alkandiyl-O-C(=O)-, -O-C(=O)-C₁-C₈-Alkandiyl-, -O-C(=O)-C₁-C₈-Alkandiyl-C(=O)-O-, -C₆-C₁₀-Aryldiyl-, -O-C₆-C₁₀-Aryldiyl-.
   Noch bevorzugter ist Y ausgewählt aus der Gruppe bestehend aus -C₁-C₈-Alkandiyl-,-C₁-C₈-Alkandiyl-O-, -C(=O)-O-, -C₁-C₈-Alkandiyl--C(=O)-O-, -C(=O)-O-C₁-C₈-Alkandiyl-, -O-C(=O)-, -C₁-C₈-Alkandiyl-O-C(=O)-.
   Noch bevorzugter ist Y ausgewählt aus der Gruppe bestehend aus -C(=O)-O-,-C₁-C₂-Alkandiyl-, -C₁-C₂-Alkandiyl-O-, -C(=O)-O-C₁-C₂-Alkandiyl-, -C₁-C₂-Alkandiyl-C(=O)-O-, -C₁-C₂-Alkandiyl-O-C(=O)-. Besonders bevorzugt ist Y -CH₂-, -CH₂-O-, -C(=O)-O-, -CH₂-C(=O)-O-, -CH₂-O-C(=O)-, oder -C(=O)-O-CH₂-. Ganz besonders bevorzugt ist Y -CH₂-O-, -C(=O)-O-, -CH₂-C(=O)-O-, oder -CH₂-O-C(=O);
- m 0 oder 1 ist;
- n eine ganze Zahl von 2 bis 6 ist, bevorzugter 2 ist;
- p eine ganze Zahl von 1 bis 2 ist, bevorzugt 1 ist;
- R₁ ausgewählt ist aus der Gruppe bestehend aus -C(=O)-OH, -C(=O)-O-C₁-C₄-Alkyl, -O-C(=O)-C₁-C₄-Alkyl, -C₁-C₄-Alkyl, -CH₂-OH, oder -CH₂-O-C₁-C₄-Alkyl, bevorzugt ausgewählt ist aus der Gruppe bestehend aus -C(=O)-OH, -C(=O)-O-Methyl, -O-C(=O)-Methyl, Methyl, Ethyl, n-Propyl, iso-Propyl, n-Butyl, iso-Butyl, und tert-Butyl,-CH₂-OH, -CH₂-O-Methyl, 2-Hydroxyethyl, 3-Hydroxy-n-propyl, 2-Hydroxy-isopropyl, 4-Hydroxy-n-Butyl, 3-Hydroxy-isobutyl, und 2-Hydroxy-tert-butyl. Methyl, Ethyl, -CH₂-OH und 2-Hydroxyethyl sind besonders bevorzugt, und ganz besonders bevorzugt ist Methyl. Alternativ bilden zwei R₁ zusammen eine Brücke der Formel -(CH₂)_{z}- welche zwei Cycloolefin-Strukturelemente miteinander verbindet, worin z eine ganze Zahl von 1 bis 2, bevorzugt 1 ist, oder eine Brücke der Formel -(CH₂)_{q}-C=C-(CH₂)_{q}- welche zwei Cycloolefin-Strukturelemente miteinander verbindet,, worin q unabhängig voneinander eine ganze Zahl von 0 bis 1, bevorzugter beide 0 sind, oder eine Brücke der Formel-(CH₂)_{q}-O-(CH₂)_{q}- welche zwei Cycloolefin-Strukturelemente miteinander verbindet, worin q unabhängig voneinander eine ganze Zahl von 0 bis 2, bevorzugt von 0 bis 1, noch bevorzugter beide 0 sind, oder eine Brücke der Formel -C(=O)-O-(CH₂)_{z}-O-(O=)-C- welche zwei Cycloolefin-Strukturelemente miteinander verbindet, worin z eine ganze Zahl von 1 bis 4, bevorzugt von 1 bis 3, bevorzugter von 1 bis 2, besonders bevorzugt 1 ist, oder eine Brücke der Formel -C(=O)-O-Phenyl-O-(O=)-C- welche zwei Cycloolefin-Strukturelemente miteinander verbindet, oder eine Brücke der Formel-(CH₂)_{q}-Si(R₄)₂-(CH₂)_{q}- welche zwei Cycloolefin-Strukturelemente miteinander verbindet, worin q unabhängig voneinander eine ganze Zahl von 0 bis 1, noch bevorzugter mindestens einmal 1 sind, und R₄ H oder ein substituiertes oder unsubstituiertes C₁-C₄-Alkyl ist, bevorzugter H oder ein unsubstituiertes C₁-C₄-Alkyl ist, noch bevorzugter ein unsubstituiertes C₁-C₄-Alkyl ist;
- R₂ ausgewählt ist aus der Gruppe bestehend aus Methyl, Ethyl, n-Propyl, iso-Propyl, n-Butyl, iso-Butyl, und tert-Butyl, 2-Hydroxyethyl, 3-Hydroxy-n-propyl, 2-Hydroxy-isopropyl, 4-Hydroxy-n-Butyl, 3-Hydroxy-isobutyl, und 2-Hydroxy-tert-butyl; Methyl, Ethyl und 2-Hydroxyethyl sind besonders bevorzugt, und ganz besonders bevorzugt ist Methyl.

Das RadP-ROMP-Monomer hat in einer bevorzugteren Ausführungsform die folgende Formel (IV), (V), oder (VI): 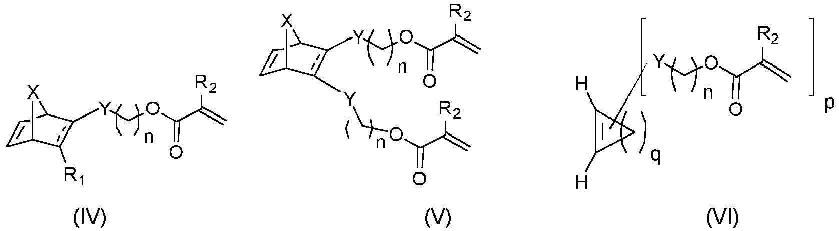 worin:
- --- eine Einfach- oder Doppelbindung, bevorzugt eine Einfachbindung ist;
- Y, n, R₁ und p wie oben für Formel (III) und bevorzugt wie für die oben definierte bevorzugte Variante von Formel (III) definiert sind;
- X -CH₂- oder -O-, bevorzugt -CH₂- ist;
- q eine ganze Zahl ausgewählt aus der Gruppe bestehend aus 1, 2, 3, 5 und 6, bevorzugt ausgewählt aus 1, 3 und 5, besonders bevorzugt 3 ist, wobei p kleiner oder gleich q ist.

Das RadP-ROMP-Monomer ist in einer noch bevorzugteren Ausführungsform ausgewählt aus der folgenden Gruppe bestehend aus den Formeln (VII) bis (IX): 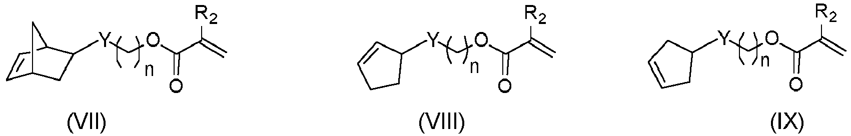 worin: Y, n und R₂ wie oben für Formel (III) und bevorzugt wie für die oben definierte bevorzugte Variante von Formel (III) definiert sind.

In einer besonders bevorzugten Ausführungsform ist das RadP-ROMP-Monomer ein Molekül, welches sowohl eine Norbornengruppe als auch eine Methacrylatgruppe umfasst, also ein Molekül der Formel (VII), worin R₂ Methyl ist. Das RadP-ROMP-Monomer hat somit in dieser Ausführungsform die folgende Formel (X): 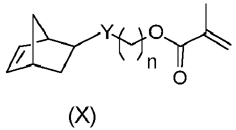 worin:
Y und n wie oben für Formel (III) und bevorzugt wie für die oben definierte bevorzugte Variante von Formel (III) definiert sind.

Am bevorzugtesten ist das RadP-ROMP-Monomer ein Molekül mit der Formel Mon13 oder Mon14, ganz besonders bevorzugt ein Molekül mit der Formel Mon13:

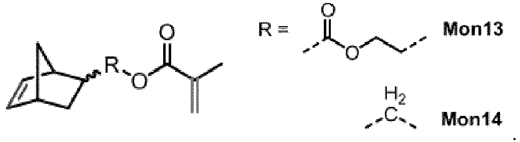

Der prozentuale Anteil (in Gew.-%) von RadP-ROMP-Monomer in Komponente A beträgt vorteilhafterweise mehr als etwa 5%, bevorzugt mehr als etwa 15%, und besonders bevorzugt mehr als etwa 20% bezogen auf das Gesamtgewicht der Komponente A. Der prozentuale Anteil (in Gew.-%) von RadP-ROMP-Monomer in Komponente A beträgt vorteilhafterweise von etwa 5% bis etwa 90%, bevorzugt von etwa 8% bis etwa 80%, bevorzugter von etwa 10% bis etwa 60%, bevorzugter von etwa 20% bis etwa 55%, noch bevorzugter von etwa 25% bis etwa 55%, besonders bevorzugt von etwa 25% bis etwa 50% und ganz besonders bevorzugt von etwa 28% bis etwa 45% bezogen auf das Gesamtgewicht der Komponente A.

Am bevorzugtesten sind die in den Beispielen verwendeten ROMP-Monomere, bevorzugt etwa in den in den Beispielen angegebenen Mengen und/oder Relationen zur Gesamtmenge der Komponente A und/oder Relationen zur Gesamtmenge des gesamten 2K-Systems.

### Erfindungsgemäße Zweikomponenten-Systeme

Die erfindungsgemäße Verbindung findet in einer Ausführungsform Verwendung als Monomer in einem Zweikomponenten-System. Dieses erfindungsgemäße 2K-System ist entweder ein Single Cure-2K-System oder ein Dual Cure-2K-System.

Optional kann ein erfindungsgemäßes 2K-System auch als Mehrkomponentensystem mit mehr als 2 Komponenten ausgestaltet sein.

Die erfindungsgemäße Verbindung ist in einer Ausführungsform in einem erfindungsgemäßen 2K-System das einzige polymerisierbare Monomer. In einer weiteren, bevorzugten Ausführungsform ist jedoch mindestens ein weiteres polymerisierbares Monomer in dem 2K-System vorhanden. Bevorzugt ist dieses weitere Monomer ausgewählt aus der Gruppe bestehend aus ROMP-Monomeren, (Meth)acrylaten, und Kombinationen davon. Im erfindungsgemäßen Single Cure-2K-System ist beispielsweise bevorzugt zusätzlich zur erfindungsgemäßen Verbindung noch ein (Meth)acrylat enthalten. Im erfindungsgemäßen Dual Cure-2K-System ist beispielsweise bevorzugt zusätzlich zur erfindungsgemäßen Verbindung noch ein ROMP-Monomer und ein (Meth)acrylat enthalten. Dies wird im Folgenden detailliert beschrieben.

### Erfindungsgemäßes Single Cure-System

Das erfindungsgemäße Single Cure-System ist typischerweise ein Zweikomponenten-System. Jedoch kann es durch Aufteilung der Inhaltsstoffe auch als Mehrkomponenten-System mit mehr als zwei Komponenten, z.B. drei Komponenten, konfektioniert werden. Diese drei oder mehr Komponenten werden dann gleichzeitig oder nacheinander miteinander vermischt, um die Polymerisation zu starten. Ein Zweikomponenten-System wie hierin beschrieben ist jedoch bevorzugt.

Das erfindungsgemäße Single Cure-Zweikomponenten-System besteht aus einer Komponente A und einer Komponente B. Jede dieser Komponenten kann das erfindungsgemäße RadP-ROMP-Monomer enthalten, typischerweise ist es jedoch nur in Komponente A enthalten. Des Weiteren kann auch jede dieser Komponenten optional mindestens ein weiteres polymerisierbares Monomer enthalten (was auch zwei oder mehr weitere polymerisierbare Monomere einschließt). Bevorzugt ist, wenn es vorhanden ist, das mindestens eine weitere polymerisierbare Monomer ebenfalls in Komponente A enthalten. Das mindestens eine weitere polymerisierbare Monomer ist bevorzugt ein radikalisch polymerisierbares Monomer, insbesondere ein (Meth)acrylat.

Das erfindungsgemäße Single Cure-2K-System ist ein System, bei dem die Polymerisation radikalisch erfolgt. Der Radikalinitiator befindet sich dabei typischerweise in derjenigen Komponente, in welcher sich das erfindungsgemäße RadP-ROMP-Monomer nicht befindet, also typischerweise in Komponente B. Folglich befindet sich der Radikalbeschleuniger, wenn vorhanden, in der anderen Komponente, also in Komponente A.

In einem typischen erfindungsgemäßen Single Cure-Zweikomponenten-System enthält die Komponente (A):
- das wie oben definierte erfindungsgemäße RadP-ROMP-Monomer, bevorzugt eine wie oben beschriebene bevorzugte Ausführungsform davon;
- optional einen Radikalbeschleuniger, bevorzugt eine wie hierin beschriebene bevorzugte Ausführungsform davon.

In einer bevorzugten Ausführungsform enthält die Komponente (A):
- das wie oben definierte erfindungsgemäße RadP-ROMP-Monomer, bevorzugt eine wie oben beschriebene bevorzugte Ausführungsform davon;
- mindestens einen Radikalbeschleuniger, bevorzugt eine wie hierin beschriebene bevorzugte Ausführungsform davon;
- mindestens einen wie hierin definierten Füllstoff, bevorzugt eine wie hierin beschriebene bevorzugte Ausführungsform davon.

In einer bevorzugteren Ausführungsform enthält die Komponente (A):
- das wie oben definierte erfindungsgemäße RadP-ROMP-Monomer, bevorzugt eine wie oben beschriebene bevorzugte Ausführungsform davon;
- einen Radikalbeschleuniger, bevorzugt eine wie hierin beschriebene bevorzugte Ausführungsform davon;
- mindestens einen wie hierin definierten Füllstoff, bevorzugt eine wie hierin beschriebene bevorzugte Ausführungsform davon, bevorzugter einen Füllstoff ausgewählt aus (optional nachbehandelter) pyrogener Kieselsäure, Quarzsand und Quarzmehl, und Mischungen davon;
- mindestens ein (Meth)acrylat, bevorzugt HPMA und/oder BDDMA.

Die Komponente (B) enthält typischerweise:
- mindestens einen Radikalinitiator, bevorzugt Benzoylperoxid (BPO) oder tert-Butylperoxybenzoat.

In einer bevorzugten Ausführungsform enthält die Komponente (B):
- mindestens einen Radikalinitiator, bevorzugt Benzoylperoxid (BPO) oder tert-Butylperoxybenzoat; und
- mindestens einen wie hierin definierten Füllstoff, bevorzugt eine wie hierin beschriebene bevorzugte Ausführungsform davon.

In einer bevorzugteren Ausführungsform enthält die Komponente (B):
- Benzoylperoxid (BPO) oder tert-Butylperoxybenzoat; und
- einen Füllstoff ausgewählt aus (optional nachbehandelter) pyrogener Kieselsäure, Quarzsand und Quarzmehl, und Mischungen davon.

Typischerweise enthält die Komponente (B) neben den genannten Bestandteilen auch Wasser.

Die Komponenten A und die Komponenten B aus jeder dieser Ausführungsformen sind in beliebigen Kombinationen A + B miteinander zu einem Single Cure 2K-System kombinierbar.

In einer besonders bevorzugten Ausführungsform sind die Bestandteile des Single Cure 2K-Systems eines oder mehrere der Bestandteile, welche in den erfindungsgemäßen Beispielen genannt werden. Ganz besonders bevorzugt sind Single Cure 2K-Systeme, welche dieselben Bestandteile enthalten oder aus denselben Bestandteilen bestehen, wie sie in den einzelnen erfindungsgemäßen Beispielen genannt werden, und zwar bevorzugt in etwa in den dort genannten Anteilen.

In einer ganz besonders bevorzugten Ausführungsform des erfindungsgemäßen Single Cure 2K-Systems enthält das Single Cure 2K-System die Bestandteile der Komponenten A und/oder B, bevorzugt A und B, wie in Beispiel 5 und 6, bevorzugt in Beispiel 5 für diese Komponenten beschrieben. Am bevorzugtesten enthält das Single Cure 2K-System die Komponenten A und/oder B, bevorzugt A und B, wie in Beispiel 5 oder 6, bevorzugt in Beispiel 5 für diese Komponenten beschrieben, in etwa den Mengenverhältnissen, wie sie in diesen Beispielen beschrieben sind.

### Erfindungsgemäßes Dual Cure System

Das erfindungsgemäße Dual Cure-System ist typischerweise ein Zweikomponenten-System. Jedoch kann es durch Aufteilung der Inhaltsstoffe auch als Mehrkomponenten-System mit mehr als zwei Komponenten, z.B. drei Komponenten, konfektioniert werden. Diese drei oder mehr Komponenten werden dann gleichzeitig oder nacheinander miteinander vermischt, um die Polymerisation zu starten. Ein Zweikomponenten-System wie hierin beschrieben ist jedoch bevorzugt.

Sollte ein Mehrkomponenten-System verwendet werden, so sollte der Radikalinitiator sich vorteilhafterweise in einer anderen Komponente als der ROMP-Initiator befinden. Des Weiteren muss sich der Radikalinitiator in einer anderen Komponente als das Methacrylat befinden, und der ROMP-Initiator in einer anderen Komponente als das RadP-ROMP-Monomer.

Das erfindungsgemäße Dual Cure-Zweikomponenten-System umfasst:
eine **Komponente A** umfassend das erfindungsgemäße RadP-ROMP-Monomer und
einen Radikalinitiator (beispielsweise BPO), und optional mindestens ein weiteres Monomer, welches ein gespanntes Cycloolefin als Strukturelement enthält (ROMP-Monomer); und
eine **Komponente B** umfassend mindestens einen Initiator für Ring Opening Metathese Polymerisation (ROMP-Initiator), und mindestens einen Radikalbeschleuniger (beispielsweise DMpT), und optional mindestens ein (Meth)acrylat, bevorzugt ein Methacrylat (beispielsweise Methylmethacrylat oder BDDMA).

In einer Ausführungsform des erfindungsgemäßen Dual Cure 2K-Systems enthält die Komponente A:
- das wie oben definierte RadP-ROMP-Monomer, bevorzugt eine wie oben beschriebene bevorzugte Ausführungsform davon;
- optional mindestens ein weiteres Monomer, welches ein gespanntes Cycloolefin als Strukturelement enthält (also ein wie hierin definiertes ROMP-Monomer), bevorzugt eine wie hierin beschriebene bevorzugte Ausführungsform davon; und
- mindestens einen wie hierin definierten Radikalinitiator, bevorzugt eine wie hierin beschriebene bevorzugte Ausführungsform davon;
und die Komponenten B:
- mindestens einen wie hierin definierten ROMP-Initiator, bevorzugt eine wie hierin beschriebene bevorzugte Ausführungsform davon; und
- mindestens einen wie hierin definierten Radikalbeschleuniger, bevorzugt eine wie hierin beschriebene bevorzugte Ausführungsform davon.

In einer bevorzugteren Ausführungsform des erfindungsgemäßen Dual Cure 2K-Systems enthält die Komponente A:
- das wie oben definierte RadP-ROMP-Monomer, bevorzugt eine wie oben beschriebene bevorzugte Ausführungsform davon;
- optional mindestens ein weiteres Monomer, welches ein gespanntes Cycloolefin als Strukturelement enthält (ROMP-Monomer), bevorzugt eine wie hierin beschriebene bevorzugte Ausführungsform davon; und
- mindestens einen wie hierin definierten Radikalinitiator, bevorzugt eine wie hierin beschriebene bevorzugte Ausführungsform davon;
und die Komponenten B:
- das wie hierin definierte (Meth)acrylat, bevorzugt eine wie hierin beschriebene bevorzugte Ausführungsform davon;
- mindestens einen wie hierin definierten ROMP-Initiator, bevorzugt eine wie hierin beschriebene bevorzugte Ausführungsform davon;
- mindestens einen wie hierin definierten Radikalbeschleuniger, bevorzugt eine wie hierin beschriebene bevorzugte Ausführungsform davon; und
- mindestens einen wie hierin definierten Inhibitor, bevorzugt eine wie hierin beschriebene bevorzugte Ausführungsform davon.

In einer bevorzugteren Ausführungsform des erfindungsgemäßen Dual Cure 2K-Systems enthält die Komponente A:
- das wie oben definierte RadP-ROMP-Monomer, bevorzugt eine wie oben beschriebene bevorzugte Ausführungsform davon;
- optional mindestens ein weiteres Monomer, welches ein gespanntes Cycloolefin als Strukturelement enthält (ROMP-Monomer), bevorzugt eine wie hierin beschriebene bevorzugte Ausführungsform davon;
- mindestens einen wie hierin definierten Radikalinitiator, bevorzugt eine wie hierin beschriebene bevorzugte Ausführungsform davon; und
- mindestens einen wie hierin definierten Füllstoff, bevorzugt eine wie hierin beschriebene bevorzugte Ausführungsform davon;
und die Komponenten B:
- das wie hierin definierte Methacrylat, bevorzugt eine wie hierin beschriebene bevorzugte Ausführungsform davon;
- mindestens einen wie hierin definierten ROMP-Initiator, bevorzugt eine wie hierin beschriebene bevorzugte Ausführungsform davon;
- mindestens einen wie hierin definierten Radikalbeschleuniger, bevorzugt eine wie hierin beschriebene bevorzugte Ausführungsform davon; und
- mindestens einen wie hierin definierten Inhibitor, bevorzugt eine wie hierin beschriebene bevorzugte Ausführungsform davon.

In einer bevorzugteren Ausführungsform des erfindungsgemäßen Dual Cure 2K-Systems enthält die Komponente A:
- das wie oben definierte RadP-ROMP-Monomer, bevorzugt eine wie oben beschriebene bevorzugte Ausführungsform davon;
- optional mindestens ein weiteres Monomer, welches ein gespanntes Cycloolefin als Strukturelement enthält (ROMP-Monomer), bevorzugt eine wie hierin beschriebene bevorzugte Ausführungsform davon;
- mindestens einen wie hierin definierten Radikalinitiator, bevorzugt eine wie hierin beschriebene bevorzugte Ausführungsform davon; und
- mindestens einen wie hierin definierten Füllstoff, bevorzugt eine wie hierin beschriebene bevorzugte Ausführungsform davon;
und die Komponenten B:
- das wie hierin definierte Methacrylat, bevorzugt eine wie hierin beschriebene bevorzugte Ausführungsform davon;
- mindestens einen wie hierin definierten ROMP-Initiator, bevorzugt eine wie hierin beschriebene bevorzugte Ausführungsform davon;
- mindestens einen wie hierin definierten Radikalbeschleuniger, bevorzugt eine wie hierin beschriebene bevorzugte Ausführungsform davon;
- mindestens einen wie hierin definierten Inhibitor, bevorzugt eine wie hierin beschriebene bevorzugte Ausführungsform davon; und
- mindestens einen wie hierin definierten Füllstoff, bevorzugt eine wie hierin beschriebene bevorzugte Ausführungsform davon.

In der Komponente A können ein oder mehrere ROMP-Monomere neben dem erfindungsgemäßen RadP-ROMP-Monomer vorhanden sein, und/oder in der Komponenten B können ein oder mehrere weitere (Meth)acrylate vorhanden sein.

Die Komponenten A und die Komponenten B aus jeder dieser Ausführungsformen sind in beliebigen Kombinationen A + B miteinander zu einem Dual Cure 2K-System kombinierbar.

In einer besonders bevorzugten Ausführungsform sind die Bestandteile des Dual Cure 2K-Systems eines oder mehrere der Bestandteile, welche in den erfindungsgemäßen Beispielen genannt werden. Ganz besonders bevorzugt sind Dual Cure 2K-Systeme, welche dieselben Bestandteile enthalten oder aus denselben Bestandteilen bestehen, wie sie in den einzelnen erfindungsgemäßen Beispielen genannt werden, und zwar bevorzugt in etwa in den dort genannten Anteilen.

In einer ganz besonders bevorzugten Ausführungsform des erfindungsgemäßen Dual Cure 2K-Systems enthält das Dual Cure 2K-System die Bestandteile der Komponenten A und/oder B, bevorzugt A und B, wie in Beispiel 4 oder 5, bevorzugt in Beispiel 5 für diese Komponenten beschrieben. Am bevorzugtesten enthält das Dual Cure 2K-System die Komponenten A und/oder B, bevorzugt A und B, wie in Beispiel 4 oder 5, bevorzugt in Beispiel 5 für diese Komponenten beschrieben, in etwa den Mengenverhältnissen, wie sie in diesen Beispielen beschrieben sind..

### Bestandteile der erfindungsgemäßen 2K-Systeme

Im folgende werden die einzelnen, wie oben aufgeführten Bestandteile der erfindungsgemäßen 2K-Systeme näher beschrieben, die zusätzlich zum erfindungsgemäßen RadP-ROMP-Monomer in den 2K-Systemen enthalten sind oder sein können. Soweit nicht anders angegeben gelten die folgenden Beschreibungen für die jeweils beschriebenen Bestandteile gleichermaßen für das erfindungsgemäße Single Cure-2K-System und das erfindungsgemäße Dual Cure-2K-System.

### ROMP-Monomer

Monomere, die für ROMP geeignet sind, sind dem Fachmann grundsätzlich bekannt, beispielsweise aus Leitgeb A.; Wappel J.; Slugovc C., The ROMP toolbox upgraded, Polymer 2010, 51, 2927-2946; aus Slugovc C., The Ring Opening Metathesis Polymerisation Toolbox, Macromol. Rapid Commun. 2004, 25,1283-1297; aus EP 0 771 830 A2, und aus US 7,691,919 B2, Spalten 11 bis 13. Die Beschreibung der ROMP-Monomere, welche in diesen Dokumenten gegeben wird, wird hiermit durch Referenz in die vorliegende Beschreibung aufgenommen. ROMP-Monomer kann grundsätzlich jede Verbindung sein, welche als Strukturelement mindestens ein wie oben definiertes gespanntes Cycloolefin umfasst, oder welche ein solches wie oben definiertes gespanntes Cycloolefin ist.
Das ROMP-Monomer besteht entweder aus dem unsubstituierten gespannten Cycloolefin-Strukturelement, ist also ein gespanntes Cycloolefin, oder das gespannte Cycloolefin-Strukturelement kann einen oder mehrere, bevorzugt einen oder zwei Substituenten tragen. Außerdem kann mindestens ein Arylring, bevorzugt ein Benzolring, an das gespannte Cycloolefin-Strukturelement ankondensiert sein.

In einer bevorzugten Ausführungsform ist das ROMP-Monomer das unsubstituierte gespannte Cycloolefin-Strukturelement, also ein unsubstituiertes mono- oder polycyklisches gespanntes Cycloolefin ohne ankondensiertes Aryl, das ROMP-Monomer ist also beispielsweise Cyclopenten oder Norbornen.

In einer weiteren bevorzugten Ausführungsform ist das ROMP-Monomer das substituierte gespannte Cycloolefin-Strukturelement, also ein substituiertes gespanntes Cycloolefin. *"Substituiertes gespanntes Cycloolefin"* bedeutet ein gespanntes Cycloolefin-Strukturelement welches substituiert ist mit einem oder mehreren, bevorzugt einem Substituenten ausgewählt aus der Gruppe bestehend aus Halogen, Pseudohalogen, C₁-C₂₀-Alkyl, Hydroxy-C₁-C₂₀-Alkyl, C₂-C₂₀-Alkenyl, Hydroxy-C₂-C₂₀-Alkenyl, C₂-C₂₀-Alkinyl, Hydroxy-C₂-C₂₀-Alkinyl, -O-C₁-C₂₀-Alkyl, -O-C₂-C₂₀-Alkenyl, -O-C₂-C₂₀-Alkinyl, -O-C(=O)-C₁-C₂₀-Alkyl, -O-C(=O)-C₂-C₂₀-Alkenyl,-O-C(=O)-C₂-C₂₀-Alkinyl, -C(=O)-O-C₁-C₂₀-Alkyl, -C(=O)-O-C₂-C₂₀-Alkenyl, -C(=O)-O-C₂-C₂₀-Alkinyl, -C(=O)-O-C₁-C₂₀-Hydroxyalkyl, -C(=O)-O-C₂-C₂₀-Hydroxyalkenyl, -C(=O)-O-C₂-C₂₀-Hydroxyalkinyl, -O-C₁-C₂₀-Alkandiyl-C(=O)-O-C₁-C₂₀-Hydroxyalkyl, -O-C₁-C₂₀-Alkandiyl-C(=O)-O-C₂-C₂₀-Hydroxyalkenyl, -O-C₁-C₂₀-Alkandiyl-C(=O)-O-C₂-C₂₀-Hydroxyalkinyl, -O-C₁-C₂₀-Alkenyldiyl-C(=O)-O-C₁-C₂₀-Hydroxyalkyl, -O-C₁-C₂₀-Alkenyldiyl-C(=O)-O-C₂-C₂₀-Hydroxyalkenyl, -O-C₁-C₂₀-Alkenyldiyl-C(=O)-O-C₂-C₂₀-Hydroxyalkinyl, -O-C₁-C₂₀-Alkandiyl-O-C(=O)-C₁-C₂₀-Alkyl, -O-C₁-C₂₀-Alkandiyl-O-C(=O)-C₂-C₂₀-Alkenyl, -O-C₁-C₂₀-Alkandiyl-O-C(=O)-C₂-C₂₀-Alkinyl, Heteroaryl, -O-Heteroaryl, -O-C₁-C₂₀-Alkandiyl-Heteroaryl, -O-C(=O)-Heteroaryl, -O-C₁-C₂₀-Alkandiyl-O-C(=O)-Heteroaryl, Aryl, -O-Aryl, -O-C₁-C₂₀-Alkandiyl-Aryl,-O-C(=O)-Aryl, -O-C₁-C₂₀-Alkandiyl-O-C(=O)-Aryl, oder Kombinationen davon.

Der Substituent ist bevorzugt ausgewählt aus der Gruppe bestehend aus Halogen, C₁-C₂₀-Alkyl, Hydroxy-C₁-C₂₀-Alkyl, C₂-C₂₀-Alkenyl, Hydroxy-C₂-C₂₀-Alkenyl,. -O-C₁-C₂₀-Alkyl, -O-C₂-C₂₀-Alkenyl, -O-C₂-C₂₀-Alkinyl, -O-C(=O)-C₁-C₂₀-Alkyl, -O-C(=O)-C₂-C₂₀-Alkenyl, -O-C(=O)-C₂-C₂₀-Alkinyl, -O-C₁-C₂₀-Alkandiyl-O-C(=O)-C₁-C₂₀-Alkyl, und -O-C₁-C₂₀-Alkandiyl-O-C(=O)-C₂-C₂₀-Alkenyl. Besonders bevorzugt ist der Substituent ein -O-C(=O)-C₂-C₂₀-Alkenyl oder O-C₁-C₂₀-Alkandiyl-O-C(=O)-C₂-C₂₀-Alkenyl.

Der Substituent ist bevorzugt an ein C-Atom des gespannten Cycloolefin-Strukturelements gebunden, welches nicht Teil der C=C-Doppelbindung im Ring und auch (im Falle eines Bicyklus') kein Brückenkopf-C-Atom ist. In einer weiteren bevorzugten Ausführungsform befindet sich der Substituent bei einem polycyklischen gespannten Cycloolefin an einem Ring ohne Doppelbindung im Ring, oder im Falle von verbrückten Systemen an einer anderen Brücke als derjenigen, in welcher sich die Doppelbindung befindet.

In einer bevorzugten Ausführungsform ist der Substituent ein Strukturelement, welches ein Ester einer Carbonsäure ist. Handelt es sich bei der Carbonsäure um eine α,β-ungesättigten Carbonsäure, so wäre das resultierende Monomer das oben beschriebene erfindungsgemäße RadP-ROMP-Monomer; dieses soll jedoch für die Zwecke der vorliegenden Beschreibung nicht vom Begriff "ROMP-Monomer" umfasst sein, da der Begriff "ROMP-Monomer" hier nur Monomere bezeichnen soll, welche zusätzlich zum erfindungsgemäßen RadP-ROMP-Monomer in einem erfindungsgemäßen 2K-System vorhanden sein können.

Die verestere Gruppe ist in einer bevorzugten Ausführungsform ein Alkyl oder ein Alkenyl, oder ein Hydroxyalkyl oder Hydroxyalkenyl, also eine Gruppe, die neben dem -O- in der Estergruppe noch eine oder mehrere, bevorzugt eine, freie OH-Gruppe trägt. In einer weiteren bevorzugten Ausführungsform ist die Carbonsäuregruppe mit dem gespannten Cycloolefin-Element verestert, entweder direkt oder indirekt. Indirekt bedeutet, dass sich zwischen der Estergruppe und dem gespannten Cycloolefin-Element noch weitere Atome befinden, beispielsweise eine verzweigte oder unverzweigte -C₁-C₂₀-Alkandiylgruppe, -C₁-C₂₀-Alkandiyl-O-Gruppe, -C(=O)-O-Alkandiylgruppe, -Alkandiyl-C(=O)-O-Alkandiylgruppe, -Alkandiyl-O-C(=O)-Alkandiylgruppe, -O-C(=O)-Alkandiylgruppe oder -C₂-C₂₀-Alkenyldiylgruppe.

In einer bevorzugten Ausführungsform ist das ROMP-Monomer eine Verbindung mit der Formel (I) 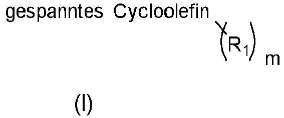 worin:
- gespanntes Cycloolefin ein wie oben definiertes gespanntes Cycloolefin-Strukturelement bedeutet;
- m eine ganze Zahl von 0 bis 2 ist, bevorzugt 0 oder 1 ist; wenn m 2 ist, dann sind die beiden R₁ bevorzugt gleich;
- R₁ unabhängig voneinander ausgewählt sind aus der Gruppe bestehend aus -C(=O)-OH, -C(=O)-O-C₁-C₂₀-Alkyl, -O-C(=O)-C₁-C₂₀-Alkyl, -C(=O)-O-Aryl, -O-C(=O)-Aryl,-C₁-C₂₀-Alkyl, -Aryl, -C₁-C₂₀-Hydroxyalkyl, -C₁-C₂₀-Alkandiyl-O-C₁-C₂₀-Alkyl, Halogenyl, -CF₃, -CF₂-CF₃, und -SiAlkyl₃. Bevorzugt ist R₁ ein -C(=O)-OH, -C(=O)-O-C₁-C₈-Alkyl, -O-C(=O)-C₁-C₈-Alkyl, -C₁-C₈-Alkyl, -C₁-C₈-Hydroxyalkyl, oder -C₁-C₈-Alkandiyl-O-C₁-C₈-Alkyl, noch bevorzugter ein -C(=O)-OH, -C(=O)-O-C₁-C₄-Alkyl, -O-C(=O)-C₁-C₄-Alkyl, -C₁-C₄-Alkyl, -C₁-C₄-Hydroxyalkyl, oder -C₁-C₄-Alkandiyl-O-C₁-C₄-Alkyl, noch bevorzugter ein -C(=O)-OH, -C(=O)-O-C₁-C₄-Alkyl, -O-C(=O)-C₁-C₄-Alkyl, -C₁-C₄-Alkyl, -CH₂-OH, oder -CH₂-O-C₁-C₄-Alkyl. Noch bevorzugter ist R₁ ausgewählt aus der Gruppe bestehend aus -C(=O)-OH, -C(=O)-O-Methyl, -O-C(=O)-Methyl, Methyl, Ethyl, n-Propyl, iso-Propyl, n-Butyl, iso-Butyl, und tert-Butyl, -CH₂-OH, -CH₂-O-Methyl, 2-Hydroxyethyl, 3-Hydroxy-n-propyl, 2-Hydroxy-isopropyl, 4-Hydroxy-n-Butyl, 3-Hydroxy-isobutyl, und 2-Hydroxy-tert-butyl. Methyl, Ethyl, -C(=O)-O-Methyl, -O-C(=O)-Methyl, -CH₂-OH und 2-Hydroxyethyl sind besonders bevorzugt, und ganz besonders bevorzugt ist Methyl oder -CH₂-OH.
   Zwei an nebeneinanderliegenden Kohlenstoffatome gebundene R₁ können auch miteinander verknüpft sein und so mit den Kohlenstoffatomen, an welche sie gebunden sind, einen Ring bilden, bevorzugt einen Ring mit 5 bis 7 Ringatomen. Beispielsweise kann so ein Cyclopentanring entstehen, oder ein Lactonring.
   Alternativ bilden zwei R₁ zusammen eine Brücke der Formel -(CH₂)_{z}- welche zwei Cycloolefin-Strukturelemente miteinander verbindet, worin z eine ganze Zahl von 1 bis 4, bevorzugt von 1 bis 3, bevorzugter von 1 bis 2, besonders bevorzugt 1 ist, oder eine Brücke der Formel -(CH₂)_{q}-Ar-(CH₂)_{q}- welche zwei Cycloolefin-Strukturelemente miteinander verbindet, worin Ar ein Aromat ist, bevorzugt Phenyl, q unabhängig voneinander eine ganze Zahl von 0 bis 2, bevorzugt von 0 bis 1, noch bevorzugter beide 0 sind, oder eine Brücke der Formel -(CH₂)_{q}-C=C-(CH₂)_{q}- welche zwei Cycloolefin-Strukturelemente miteinander verbindet, worin q unabhängig voneinander eine ganze Zahl von 0 bis 2, bevorzugt von 0 bis 1, noch bevorzugter beide 0 sind, oder eine Brücke der Formel -(CH₂)_{q}-O-(CH₂)_{q}- welche zwei Cycloolefin-Strukturelemente miteinander verbindet, worin q unabhängig voneinander eine ganze Zahl von 0 bis 2, bevorzugt von 0 bis 1, noch bevorzugter beide 0 sind, oder eine Brücke der Formel -(CH₂)_{q}-NR₄-(CH₂)_{q}- welche zwei Cycloolefin-Strukturelemente miteinander verbindet, worin q unabhängig voneinander eine ganze Zahl von 0 bis 2, bevorzugt von 0 bis 1, noch bevorzugter beide 0 sind, und R₄ H oder ein substituiertes oder unsubstituiertes C₁-C₂₀-Alkyl ist, bevorzugter H oder ein unsubstituiertes C₁-C₂₀-Alkyl ist, noch bevorzugter H ist, oder eine Brücke der Formel -C(=O)-O-(CH₂)_{z}-O-(O=)-C- welche zwei Cycloolefin-Strukturelemente miteinander verbindet, worin z eine ganze Zahl von 1 bis 4, bevorzugt von 1 bis 3, bevorzugter von 1 bis 2, besonders bevorzugt 2 ist, oder eine Brücke der Formel -C(=O)-O-, oder eine Brücke der Formel -C(=O)-O-Ar-O-(O=)-C- welche zwei Cycloolefin-Strukturelemente miteinander verbindet, worin Ar ein Aromat, bevorzugt Phenyl ist, z eine ganze Zahl von 1 bis 4, bevorzugt von 1 bis 3, bevorzugter von 1 bis 2, besonders bevorzugt 1 ist, oder eine Brücke der Formel -(CH₂)_{q}-Si(R₄)₂-(CH₂)_{q}- welche zwei Cycloolefin-Strukturelemente miteinander verbindet, worin q unabhängig voneinander eine ganze Zahl von 0 bis 2, bevorzugt von 0 bis 1, noch bevorzugter mindestens einmal 1 sind, und R₄ H oder ein substituiertes oder unsubstituiertes C₁-C₂₀-Alkyl ist, bevorzugter H oder ein unsubstituiertes C₁-C₂₀-Alkyl ist, noch bevorzugter ein unsubstituiertes C₁-C₄-Alkyl ist.

In einer bevorzugten Ausführungsform (Variante) hat das ROMP-Monomer die Formel (I): 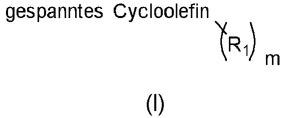 worin:
- gespanntes Cycloolefin ausgewählt ist aus der Gruppe bestehend aus Cyclopenten, Norbornen, Norbornadien, Cycloocten, Tetracyclo[6.2.1.1.0]-dodecen und aus deren Oxa-Derivaten, in welchen ein C-Atom im gespannten Cycloolefin-Strukturelement durch O ersetzt ist. Bevorzugt ist es ausgewählt aus der Gruppe bestehend aus Norbornen, Norbornadien, Tricyclo[5.2.1.0^{2,6}]deca-3,8-dien (Dicyclopentadien), Tetracyclo[6.2.1.1.0]-dodecen, und aus deren Oxa-Derivaten, in welchen ein C-Atom im gespannten Cycloolefin-Strukturelement durch O ersetzt ist;
- m 0 oder 1 ist;
- R₁ ausgewählt ist aus der Gruppe bestehend aus -C(=O)-OH, -C(=O)-O-C₁-C₄-Alkyl, -O-C(=O)-C₁-C₄-Alkyl, -C₁-C₄-Alkyl, -CH₂-OH, oder -CH₂-O-C₁-C₄-Alkyl, bevorzugt ausgewählt ist aus der Gruppe bestehend aus -C(=O)-OH, -C(=O)-O-Methyl, -O-C(=O)-Methyl, Methyl, Ethyl, n-Propyl, iso-Propyl, n-Butyl, iso-Butyl, und tert-Butyl,-CH₂-OH, -CH₂-O-Methyl, 2-Hydroxyethyl, 3-Hydroxy-n-propyl, 2-Hydroxy-isopropyl, 4-Hydroxy-n-Butyl, 3-Hydroxy-isobutyl, und 2-Hydroxy-tert-butyl. Methyl, Ethyl, -CH₂-OH und 2-Hydroxyethyl sind besonders bevorzugt, und ganz besonders bevorzugt ist Methyl. Alternativ bilden zwei R₁ zusammen eine Brücke der Formel -(CH₂)_{z}- welche zwei Cycloolefin-Strukturelemente miteinander verbindet, worin z eine ganze Zahl von 1 bis 2, bevorzugt 1 ist, oder eine Brücke der Formel -(CH₂)_{q}-C=C-(CH₂)_{q}- welche zwei Cycloolefin-Strukturelemente miteinander verbindet,, worin q unabhängig voneinander eine ganze Zahl von 0 bis 1, bevorzugter beide 0 sind, oder eine Brücke der Formel-(CH₂)_{q}-O-(CH₂)_{q}- welche zwei Cycloolefin-Strukturelemente miteinander verbindet, worin q unabhängig voneinander eine ganze Zahl von 0 bis 2, bevorzugt von 0 bis 1, noch bevorzugter beide 0 sind, oder eine Brücke der Formel -C(=O)-O-(CH₂)_{z}-O-(O=)-C- welche zwei Cycloolefin-Strukturelemente miteinander verbindet, worin z eine ganze Zahl von 1 bis 4, bevorzugt von 1 bis 3, bevorzugter von 1 bis 2, besonders bevorzugt 1 ist, oder eine Brücke der Formel -C(=O)-O-Phenyl-O-(O=)-C- welche zwei Cycloolefin-Strukturelemente miteinander verbindet, oder eine Brücke der Formel - (CH₂)_{q}-Si(R₄)₂-(CH₂)_{q}- welche zwei Cycloolefin-Strukturelemente miteinander verbindet, worin q unabhängig voneinander eine ganze Zahl von 0 bis 1, noch bevorzugter mindestens einmal 1 sind, und R₄ H oder ein substituiertes oder unsubstituiertes C₁-C₄-Alkyl ist, bevorzugter H oder ein unsubstituiertes C₁-C₄-Alkyl ist, noch bevorzugter ein unsubstituiertes C₁-C₄-Alkyl ist.

Das ROMP-Monomer hat in einer bevorzugteren Ausführungsform die folgende Formel (II A) oder (II B): 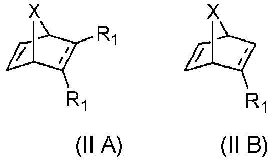 worin:
- --- eine Einfach- oder Doppelbindung, bevorzugt eine Einfachbindung ist;
- R₁ wie oben für Formel (I) und bevorzugt wie für die oben definierte bevorzugte Variante von Formel (I) definiert ist, wobei die R1 in Formel (II A) gleich oder verschieden sein können, jedoch bevorzugt gleich sind;
- X -CH₂- oder -O-, bevorzugt -CH₂- ist;
- q eine ganze Zahl ausgewählt aus der Gruppe bestehend aus 1, 2, 3, 5 und 6, bevorzugt ausgewählt aus 1, 3 und 5, besonders bevorzugt 3 ist.

Ganz besonders bevorzugte ROMP-Monomere sind ausgewählt aus der Gruppe bestehend aus 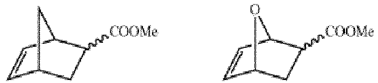 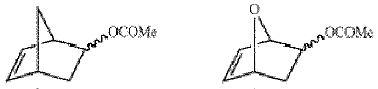 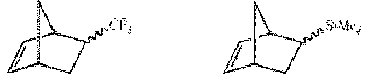 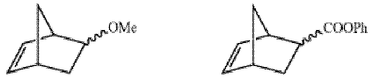 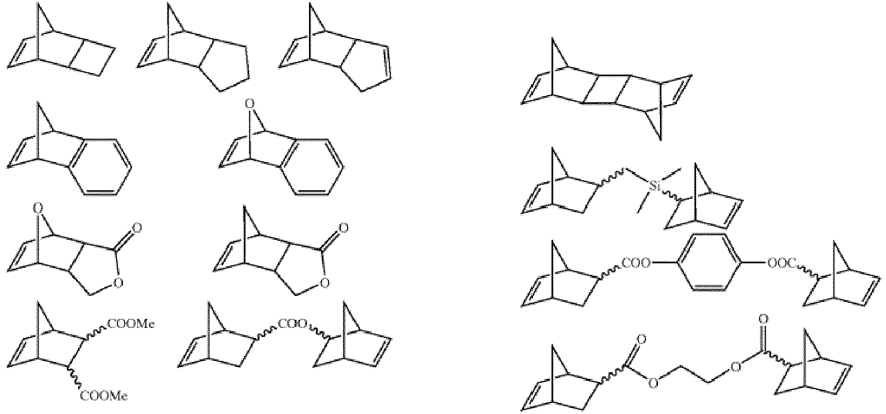 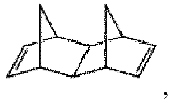 Bicyclo[3.3.2]-decen, Bicyclo[3.2.2]-nonen, Bicyclo[2.2.2]-octen, Bicyclo[2.2.1]-hepten (Norbornen), Bicyclo[2.2.0]-hexen, Cyclopenten, Cycloocten, Tetracyclo[6.2.1.1.0]-dodecen, Tricyclo[5.2.1.0^{2,6}]deca-3,8-dien (Dicyclopentadien), und Bicyclo[2.2.1]-heptadien (Norbornadien) und aus deren Oxa-Derivaten, in welchen ein C-Atom im gespannten Cycloolefin-Strukturelement durch O ersetzt ist. Aus dieser Gruppe noch weiter bevorzugt ist die Gruppe bestehend aus 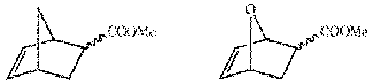 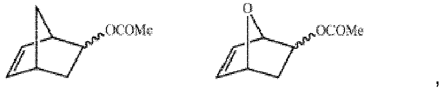 Cyclopenten, Norbornen, Norbornadien, Cycloocten, Tetracyclo[6.2.1.1.0]-dodecen und aus deren Oxa-Derivaten, in welchen ein C-Atom im gespannten Cycloolefin-Strukturelement durch O ersetzt ist. Noch bevorzugter ist die Gruppe bestehend aus Norbornen, Norbornadien, Tricyclo[5.2.1.0^{2,6}]deca-3,8-dien (Dicyclopentadien), Tetracyclo[6.2.1.1.0]-dodecen, und aus deren Oxa-Derivaten, in welchen ein C-Atom im gespannten Cycloolefin-Strukturelement durch O ersetzt ist. Am bevorzugtesten ist ein Norbornen oder ein ROMP-Monomer, welches ein Norbornen-Strukturelement umfasst, oder eines von deren Oxa-Derivaten, in welchen ein C-Atom im gespannten Cycloolefin-Strukturelement durch O ersetzt ist. Am besonders bevorzugtesten ist ein Norbornen oder ein ROMP-Monomer, welches ein Norbornen-Strukturelement umfasst.

In einer Ausführungsform sind die ROMP-Monomere aus dieser ganz besonders bevorzugten Gruppe mit einem der oben definierten Substituenten substituiert. In einer anderen Ausführungsform sind sie nicht weiter substituiert.

Im Kontext der vorliegenden Erfindung ist das in einem erfindungsgemäßen 2K-System nur optional vorhandene ROMP-Monomer kein wie oben definiertes RadP-ROMP-Monomer.

In der erfindungsgemäßen Verbindung sind die beiden für ein RadP-ROMP-Dual Cure System erforderlichen Monomerenelemente, also ein gespanntes Cycloolefin-Monomer und ein (Meth)acrylat-Monomer, in einem einzigen Monomer (dem RadP-ROMP-Monomer) bereits miteinander verbunden. Deswegen ist bei Verwendung dieses (bifunktionellen) erfindungsgemäßen RadP-ROMP-Monomers die Anwesenheit eines weiteren, separaten Monomers nicht zwingend erforderlich. In einer bevorzugten Ausführungsform ist jedoch neben dem RadP-ROMP-Monomer auch noch ein (Meth)acrylat als separates Monomer im erfindungsgemäßen 2K-System vorhanden, typischerweise in der Komponente B. In einer anderen bevorzugten Ausführungsform ist neben dem RadP-ROMP-Monomer auch noch ein ROMP-Monomer als separates Monomer im erfindungsgemäßen 2K-System vorhanden, typischerweise in der Komponente A. In einer weiteren bevorzugten Ausführungsform ist neben dem RadP-ROMP-Monomer auch ein (Meth)acrylat als separates Monomer im erfindungsgemäßen 2K-System vorhanden, typischerweise in der Komponente B, und zusätzlich auch noch ein ROMP-Monomer, typischerweise in der Komponente A.

Der prozentuale Anteil (in Gew.-%) der Gesamtmenge von ROMP-Monomer und RadP-ROMP-Monomer in Komponente A beträgt vorteilhafterweise mehr als etwa 5%, bevorzugt mehr als etwa 15%, und besonders bevorzugt mehr als etwa 20% bezogen auf das Gesamtgewicht der Komponente A. Der prozentuale Anteil (in Gew.-%) von der Gesamtmenge von ROMP-Monomer und RadP-ROMP-Monomer in Komponente A beträgt vorteilhafterweise von etwa 5% bis etwa 90%, bevorzugt von etwa 8% bis etwa 80%, bevorzugter von etwa 10% bis etwa 60%, bevorzugter von etwa 20% bis etwa 55%, noch bevorzugter von etwa 25% bis etwa 55%, besonders bevorzugt von etwa 25% bis etwa 50% und ganz besonders bevorzugt von etwa 28% bis etwa 45% bezogen auf das Gesamtgewicht der Komponente A.

Am bevorzugtesten sind die in den Beispielen verwendeten ROMP-Monomere, bevorzugt etwa in den in den Beispielen angegebenen Mengen und/oder Relationen zur Gesamtmenge der Komponente A und/oder Relationen zur Gesamtmenge des gesamten 2K-Systems.

### Radikalinitiator

Grundsätzlich kann jeder Radikalinitiator verwendet werden, welcher der Fachwelt für die Polymerisation von Methacrylaten bekannt ist.

Bevorzugt wird ein Peroxid als Radikalinitiator verwendet. Alle dem Fachmann bekannten Peroxide, die zum Härten von Methacrylaten verwendet werden, können eingesetzt werden. Derartige Peroxide umfassen organische und anorganische Peroxide, entweder flüssig oder fest, wobei auch Wasserstoffperoxid verwendet werden kann. Beispiele geeigneter Peroxide sind Peroxycarbonate (der Formel -OC(O)OO-), Peroxyester (der Formel -C(O)OO-), Diacylperoxide (der Formel -C(O)OOC(O)-), Dialkylperoxide (der Formel -OO-), Hydroperoxide (der Formel -OOH) und dergleichen. Diese können als Oligomer oder Polymer vorliegen. Eine umfassende Reihe an Beispielen für geeignete Peroxide ist zum Beispiel in der Anmeldung US 2002/0091214 A1, Absatz [0018], beschrieben.

Bevorzugt sind die Peroxide aus der Gruppe der organischen Peroxide ausgewählt. Geeignete organische Peroxide sind: tertiäre Alkylhydroperoxide, wie tert-Butylhydroperoxid, und andere Hydroperoxide, wie Cumenhydroperoxid, Peroxyester oder Persäuren, wie tert-Butylperester (z.B. tert-Butylperoxybenzoat), Benzoylperoxid (BPO), Peracetate und Perbenzoate, Lauroylperoxid, einschließlich (Di)peroxyester, Perether, wie Peroxydiethylether, Perketone, wie Methylethylketonperoxid. Die als Radikalinitiator verwendeten organischen Peroxide sind oft tertiäre Perester oder tertiäre Hydroperoxide, d.h. Peroxid-Verbindungen mit tertiären Kohlenstoffatomen, die direkt an eine -O-O-acyl- oder -OOH-Gruppe gebunden sind. Aber auch Gemische dieser Peroxide mit anderen Peroxiden können erfindungsgemäß eingesetzt werden. Die Peroxide können auch gemischte Peroxide sein, d.h. Peroxide, die zwei verschiedene Peroxid-tragende Einheiten in einem Molekül aufweisen. In einer bevorzugten Ausführungsform wird Benzoylperoxid (BPO) oder tert-Butylperoxybenzoat, ganz besonders bevorzugt wird BPO verwendet.

Das Peroxid kann in seiner Reinform, auf einem Trägermaterial, oder als Bestandteil einer Mischung verwendet werden. Typischerweise wird es als Bestandteil einer Mischung verwendet, oder auf einem Trägermaterial, beispielsweise auf einem Trägermaterial wie etwa bei Perkadox 20S. Das in den Beispielen verwendete Peroxid BPO und seine trägergebundene Variante Perkadox 20S sind besonders bevorzugt, am bevorzugtesten ist Perkadox 20S oder ein vergleichbares trägergebundenes BPO.

Der Radikalinitiator wird in einer Menge verwendet, welche auf seine Reaktivität abgestimmt ist. Typischerweise ist dies beispielsweise bei BPO eine Menge von etwa 1,0 Gew.-% bis etwa 25 Gew.-% bezogen auf alle Monomere mit einer Methacrylatgruppe, bevorzugter von etwa 2,5 Gew.-% bis etwa 12,5 Gew.-%, noch bevorzugter von etwa 3,5 Gew.-% bis etwa 7,5 Gew.-%, noch bevorzugter von etwa 5,0 Gew.-% bis etwa 6,0 Gew.-%, beispielsweise etwa 5,0 Gew.-% bezogen auf alle Monomere mit einer Methacrylatgruppe. Bei trägergebundenem BPO wird die verwendete Menge entsprechend der korrespondierenden Menge an reinem BPO angepasst. Typischerweise ist die Menge des Radikalinitiators demzufolge beispielsweise bei Perkadox 20S eine Menge von etwa 5 Gew.-% bis etwa 60 Gew.-% bezogen auf alle Monomere mit einer Methacrylatgruppe, bevorzugter von etwa 10 Gew.-% bis etwa 50 Gew.-%, noch bevorzugter von etwa 20 Gew.-% bis etwa 40 Gew.-%, noch bevorzugter von etwa 25 Gew.-% bis etwa 30 Gew.-%, beispielsweise etwa 30 Gew.-% bezogen auf alle Monomere mit einer Methacrylatgruppe.

Die bevorzugtesten Radikalinitiatoren sind die in den Beispielen verwendeten Radikalinitiatoren, bevorzugt etwa in den in den Beispielen angegebenen Mengen und/oder Relationen zu den entsprechenden Methacrylat-Monomeren.

### Füllstoffe

Die Komponente A umfasst in einer bevorzugten Ausführungsform auch einen oder mehrere Füllstoffe. Optional kann zusätzlich auch die Komponente B Füllstoffe umfassen. In einer bevorzugten Ausführungsform umfassen beide Komponenten Füllstoffe, insbesondere dieselben Füllstoffe, bevorzugt in denselben Gew.-% bezogen auf die Gesamtmenge der jeweiligen Komponente.

Es sei darauf hingewiesen, dass einige Stoffe sowohl als Füllstoff und, ggf. in modifizierter Form, auch als Additiv verwendet werden können. Beispielsweise dient pyrogene Kieselsäure in ihrer polaren, nicht nachbehandelten Form eher als Füllstoff und in ihrer apolaren, nachbehandelten Form eher als Additiv. In Fällen, in denen genau derselbe Stoff als Füllstoff oder Additiv verwendet werden kann, soll seine Gesamtmenge (ggf. in Summe mit weiteren Füllstoffen) die hierin festgelegte Obergrenze für Füllstoffe vorteilhafterweise nicht überschreiten.

Zur Herstellung eines Zweikomponenten-Systems für bauliche Anwendungen, insbesondere für die chemische Befestigung, können üblicherweise für solche Anwendungen verwendete Füllstoffe zugegeben werden. Diese Füllstoffe sind typischerweise anorganische Füllstoffe, wie beispielsweise weiter unten beschrieben.

Zur Berechnung des Füllstoffanteils gilt: Der Anteil der gesamten Füllstoffe (und ggf. Additive) an der Gesamtmenge der jeweiligen Komponente beträgt von 0 Gew.-% bis etwa 90 Gew.-%, bevorzugt von etwa 20 bis etwa 80 Gew.-%, bevorzugter von etwa 30 bis etwa 70 Gew.-%, und noch bevorzugter von etwa 50 bis etwa 65 Gew.-%. Diese Gew.-% Bereiche sind auch für den Anteil der gesamten Füllstoffe an der Gesamtmenge des 2K-Systems die bevorzugten Bereiche.

Als Füllstoffe finden übliche Füllstoffe, vorzugsweise mineralische oder mineralähnliche Füllstoffe, wie Quarz, Glas, Sand, Quarzsand, Quarzmehl, Porzellan, Korund, Keramik, Talkum, Kieselsäure (z.B. pyrogene Kieselsäure, insbesondere apolar nachbehandelte pyrogene Kieselsäure), Silikate, Aluminiumoxide (z.B. Tonerde), Ton, Titandioxid, Kreide, Schwerspat, Feldspat, Basalt, Aluminiumhydroxid, Granit oder Sandstein, polymere Füllstoffe, wie Duroplaste, hydraulisch härtbare Füllstoffe, wie Gips, Branntkalk oder Zement (z.B. Aluminatzement (oft auch als Tonerdezement bezeichnet) oder Portlandzement), Metalle, wie Aluminium, Ruß, ferner Holz, mineralische oder organische Fasern, oder dergleichen, oder Gemische von zwei oder mehr davon Verwendung.

Die Füllstoffe können in beliebigen Formen vorliegen, beispielsweise als Pulver oder Mehl, oder als Formkörper, z.B. in Zylinder-, Ring-, Kugel-, Plättchen-, Stäbchen-, Sattel- oder Kristallform, oder ferner in Faserform (fibrilläre Füllstoffe), und die entsprechenden Grundteilchen haben vorzugsweise einen maximalen Durchmesser von etwa 10 mm und einen Minimaldurchmesser von etwa 1 nm. Das heißt, der Durchmesser ist etwa 10 mm oder irgendein Wert von weniger als etwa 10 mm, aber mehr als etwa 1 nm. Bevorzugt ist der maximale Durchmesser ein Durchmesser von etwa 5 mm, bevorzugter von etwa 3 mm, noch bevorzugter von etwa 0,7 mm. Ganz besonders bevorzugt ist ein maximaler Durchmesser von etwa 0,5 mm. Der bevorzugtere Minimaldurchmesser ist etwa 10 nm, noch bevorzugter etwa 50 nm, ganz besonders bevorzugt etwa 100 nm. Durchmesserbereiche, die sich durch Kombination dieser maximalen Durchmesser und Minimaldurchmesser ergeben, sind besonders bevorzugt. Bevorzugt und deutlicher verstärkend wirken sich jedoch die globulären, inerten Stoffe (Kugelform) aus. Auch Core-Shell-Partikel, bevorzugt in Kugelform, können als Füllstoffe verwendet werden.

Bevorzugte Füllstoffe sind ausgewählt aus der Gruppe bestehend aus Zement, Kieselsäure, Quarz, Quarzsand, Quarzmehl, Tonerde, Korund und Mischungen aus zwei oder mehreren davon. Besonders bevorzugt sind Füllstoffe ausgewählt aus der Gruppe bestehend aus pyrogener Kieselsäure, insbesondere apolar nachbehandelter pyrogener Kieselsäure, Quarzsand, Quarzmehl, Korund und Mischungen aus zwei oder mehreren davon. Ganz besonders bevorzugt sind Füllstoffe ausgewählt aus der Gruppe bestehend aus apolar nachbehandelter pyrogener Kieselsäure, Quarzsand und Quarzmehl und Mischungen aus zwei oder mehr davon. Eine Mischung aus einer apolar nachbehandelten pyrogenen Kieselsäure, z.B. einer mit Polydimethylsiloxan (PDMS) nachbehandelten pyrogenen Kieselsäure (z.B. Cab-O-Sil TS-720), mit Quarzsand und/oder Quarzmehl ist besonders bevorzugt.

Wenn eine Mischung aus einer apolar nachbehandelten pyrogenen Kieselsäure, z.B. einer mit Polydimethylsiloxan (PDMS) nachbehandelten pyrogenen Kieselsäure, mit Quarzsand und/oder Quarzmehl verwendet wird, beträgt das Mischungsverhältnis zwischen apolar nachbehandelter pyrogener Kieselsäure und der Gesamtmenge aus Quarzsand und Quarzmehl von etwa 1:30 (w/w) bis etwa 1:10 (w/w), bevorzugt von etwa 1:25 (w/w) bis etwa 1:15 (w/w), bevorzugter etwa 1:20. Das Mischungsverhältnis zwischen apolar nachbehandelter pyrogener Kieselsäure und der Gesamtmenge an Monomer beträgt in jeder Komponente und im Gesamt-2K-System vorteilhaft von etwa 1:20 (w/w) bis etwa 1:5 (w/w), bevorzugt von etwa 1:15 (w/w) bis etwa 1:7,5 (w/w), bevorzugter von etwa 1:13 (w/w) bis 1:9 (w/w), noch bevorzugter von etwa 1:12 (w/w) bis etwa 1:10 (w/w).

In dieser Hinsicht wird Bezug genommen auf die Anmeldungen WO 02/079341 und WO 02/079293 sowie WO 2011/128061 A1, deren diesbezüglicher Inhalt hiermit in diese Anmeldung aufgenommen wird.

Ganz besonders bevorzugt ist die Mischung aus Füllstoffen, welche in den Beispielen verwendet wird, insbesondere in den darin beschriebenen Relationen zueinander und/oder zur Gesamtmenge der Monomere im 2K-System und/oder zur Monomer-Menge in Komponente A.

### (Meth)acrylat

Im Kontext der vorliegenden Erfindung sind (Meth)acrylate Verbindungen, welche eine veresterte Methacrylsäuregruppe oder Acrylsäuregruppe enthalten. Im Kontext der vorliegenden Erfindung sind Methacrylate die bevorzugten (Meth)acrylate. Methacrylate sind Verbindungen, welche eine veresterte Methacrylsäuregruppe enthalten, wie beispielsweise MMA und BDDMA.

Im Kontext der vorliegenden Erfindung sind die Methacrylate keine erfindungsgemäßen RadP-ROMP-Monomere; wenn ein Methacrylat ein gespanntes Cycloolefin-Strukturelement enthält, ist es ein erfindungsgemäßes RadP-ROMP-Monomer. Dies betrifft beispielsweise die Methacrylate Dicyclopentenyloxyethylmethacrylat, Tricyclopentadienyldimethacrylat, Dicyclopentenyloxyethylcrotonat, und 3-Methylcyclopentadienylmethacrylat.

Geeignete Methacrylate sind aliphatische (lineare, verzweigte oder cyklische) oder aromatische C₅-C₁₅-Methacrylate wie Methylmethacrylat (MMA), *tert*-Butylmethacrylat und Norbornylmethacrylat, sowie 2- oder 3-Hydroxypropylmethacrylat (HPMA), 1,2-Ethandioldimethacrylat, 1,3-Propandioldimethacrylat, 1,3-Butandioldimethacrylat, 1,4-Butandioldimethacrylat (BDDMA), Trimethylolpropantrimethacrylat, Phenethylmethacrylat, Tetrahydrofurfurylmethacrylat, Ethyltriglykolmethacrylat, *N,N*-Dimethylaminoethylmethacrylat, *N,N*-Dimethylaminomethylmethacrylat, Acetoacetoxyethylmethacrylat, Isobornylmethacrylat, 2-Ethylhexylmethacrylat, Diethylenglykoldimethacrylat, Methoxypolyethylenglykolmonomethacrylat, Trimethylcyclohexylmethacrylat, 2-Hydroxyethylmethacrylat (HEMA), Bisphenol-A-methacrylat, Novolakepoxidimethacrylat, Di-[methacryloyl-maleoyl]-tricyclo-5.2.1.0.^{2,6}-decan, 3-Methacryloyl-oxymethyl-tricylo-5.2.1,0.^{2.6}-decan, Decalyl-2-methacrylat, PEG-dimethacrylate wie beispielsweise PEG200-dimethacrylat, Tetraethylenglykoldimethacrylat, Solketalmethacrylat, Cyclohexylmethacrylat, Phenoxyethyldimethacrylat, und Methoxyethylmethacrylat.

Bevorzugt ist das Methacrylat ausgewählt aus der Gruppe bestehend aus aliphatischen (linearen, verzweigten oder cyklischen) C₅-C₁₅-Methacrylaten (wie Methylmethacrylat (MMA), *tert*-Butylmethacrylat und Norbornylmethacrylat), 2-Hydroxyethylmethacrylat (HEMA), 2- und 3-Hydroxypropylmethacrylat (HPMA), 1,2-Ethandioldimethacrylat, 1,4-Butandioldimethacrylat (BDDMA), 1,3-Butandioldimethacrylat, Trimethylolpropantrimethacrylat, Acetoacetoxyethylmethacrylat, Isobornylmethacrylat, Bisphenol-A-methacrylat, Trimethylcyclohexylmethacrylat, 2-Hydroxyethylmethacrylat, und PEG200-dimethacrylat. Besonders bevorzugt sind Methylmethacrylat (MMA), *tert*-Butylmethacrylat und Norbornylmethacrylat, 2-Hydroxyethylmethacrylat (HEMA), 2- und 3-Hydroxypropylmethacrylat (HPMA), 1,2-Ethandioldimethacrylat, 1,4-Butandioldimethacrylat (BDDMA), und 1,3-Butandioldimethacrylat. Ganz besonders bevorzugt sind Methylmethacrylat (MMA), 2-Hydroxyethylmethacrylat (HEMA), und 1,4-Butandioldimethacrylat (BDDMA). Am bevorzugtesten sind MMA und BDDMA.

Es können auch Mischungen aus zwei oder mehr der hier aufgeführten Methacrylate verwendet werden.

Darüber hinaus kann das Methacrylat neben der Methacrylsäureestergruppe noch andere reaktive Gruppen, die mit einem Radikalinitiator, wie einem Peroxid, polymerisiert werden können, enthalten, zum Beispiel reaktive Gruppen, die von der Itaconsäure, Citraconsäure und allylischen Gruppen und dergleichen abgeleitet sind, wie sie beispielsweise in der WO 2010/108939 A1 (Itaconsäureester) beschrieben sind.

Die Verwendung eines weiteren Methacrylat-Monomers zusätzlich zum erfindungsgemäßen RadP-ROMP-Monomer ist in einem erfindungsgemäßen 2K-System nicht zwingend erforderlich. Jedoch ist neben dem RadP-ROMP-Monomer in einer bevorzugten Ausführungsform zusätzlich ein aus der oben aufgeführten Gruppe von Methacrylaten ausgewähltes Monomer oder eine Mischung von zwei oder mehreren dieser Monomere im erfindungsgemäßen 2K-System vorhanden. Dieses optionale zusätzliche Methacrylat befindet sich bevorzugt in derjenigen Komponente des 2K-Systems, welche nicht den Radikalinitiator enthält.

Am bevorzugtesten sind die in den Beispielen verwendeten Methacrylate, bevorzugt etwa in den in den Beispielen angegebenen Mengen und/oder Relationen zum erfindungsgemäßen RadP-ROMP-Monomer und/oder zum Radikalinitiator.

Das Methacrylat bzw. die Methacrylate sind bevorzugt in einer Menge von 0 bis etwa 40 Gew.-%, besonders bevorzugt von etwa 1 bis etwa 30 Gew.-%, noch bevorzugter von etwa 2 bis etwa 25 Gew.-%, noch bevorzugter von etwa 5 bis etwa 20 Gew.-%, noch bevorzugter von etwa 7 bis etwa 15 Gew.-%, beispielsweise etwa 12 Gew.-%, bezogen auf die Gesamtmenge an ROMP-Monomer (wobei ROMP-Monomer hier daserfindungsgemäß vorhandene RadP-ROMP-Monomer mit einschließt), im erfindungsgemäßen 2K-System vorhanden.

Falls als (Meth)acrylat ein Acrylat statt eines Methacrylats verwendet wird, gilt für ein solches Acrylat dasselbe wie in den vorhergehenden Absätzen für das Methacrylat beschrieben, mit dem Unterschied dass die Verbindung statt einer veresterten Methacrylsäuregruppe eine veresterte Acrylsäuregruppe enthält.

### ROMP-Initiator

Grundsätzlich kann als ROMP-Initiator für das erfindungsgemäße Dual Cure-2K-System jeder ROMP-Katalysator verwendet werden, welcher zu einer Ringöffnung im verwendeten Ringsystem und anschließender Polymerisation führt, mit anderen Worten: jeder Katalysator für eine ringöffnende Olefinmetathese-Polymerisation (ROMP). Typischerweise sind dies Metall-Carben-Komplexe, beispielsweise Ruthenium-Carben-Komplexe.

In einer bevorzugten Ausführungsform ist der verwendete ROMP-Initiator ein Grubbs-Katalysator. Grubbs-Katalysatoren sind Ruthenium-Carben-Komplexe, sind in der Literatur bekannt und werden beispielsweise in Grubbs, R.H., Handbook of Metathesis, Wiley-VCH, Deutschland, 2003; Grubbs, R.H., Trnka, T.M., Ruthenium-catalyzed Olefin Metathesis, in: Ruthenium in Organic Synthesis (S.-I. Murahashi, ed.), Wiley-VCH, Deutschland, 2004, beschrieben; und Leitgeb, A. et al., Polymer 51(14):2927-2946 und Choi, T.I. und Grubbs, R.H., Angewandte Chemie International Edition 42(15):1743-1746 beschrieben. Besonders bevorzugt sind Grubbs-Katalysatoren mit einem oder mehreren Brompyridin(en) als Ligand, wie sie z.B. in Walsh, D.J: et al., Journal of the American Chemical Society 139(39):13644-13647, und insbesondere in Slugovc, C. et al., Macromol. Rapid Commun. 2004(25):475 und J. Mol. Catal. A-Chem. 2004(213):107, beschrieben werden.

Der Grubbs-Katalysator hat in einer bevorzugten Ausführungsform die folgende Formel: 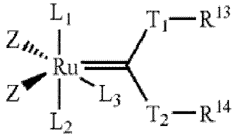 wobei
Z Cl, Br, F, I, Tosylat oder ein wie L₁, L₂, L₃ definierter Ligand ist;
L₁, L₂, L₃ unabhängig voneinander abwesend oder ausgewählt sind aus der Gruppe bestehend aus P(R¹⁵)₃ mit R¹⁵ = Phenyl, Isopropyl, Cyclohexyl, 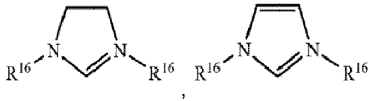 mit R¹⁶ = Mesityl (2,4,6-Trimethylphenyl; MES), unsubstituiertem Pyridin, an Position 2 oder an den Positionen 2 und 4 substituiertem Pyridin, wobei die Substituenten ausgewählt sind aus der Gruppe bestehend aus -Br, -Cl, -F, -I, -OCH₃;
R¹³, R¹⁴ unabhängig voneinander ausgewählt sind aus der Gruppe bestehend aus H, einem aromatischen oder aliphatischen, polycyclischen oder kondensierten C₆-C₂₀-Ring oder C₆-C₂₀--Ringsystem, mit 1-5 Heteroatomen im Ring, unabhängig voneinander ausgewählt aus N, S, O, und P, wobei der Ring oder das Ringsystem unsubstituiert ist oder zusätzlich substituiert ist mit 1-5 Substituenten welche unabhängig voneinander ausgewählt sind aus der Gruppe bestehend aus -Cl, -Br, -I, -F, -OR¹⁷, -CH=N-R¹⁷, -C(=O)R¹⁷, -C(=O)OR¹⁷,-OC(=O)R¹⁷, wobei R¹⁷ ein lineares oder verzweigtes acyclisches C₁-C₁₄-Alkyl oder cyclisches C₃-C₁₄-Alkyl oder ein C₆-C₁₄-Aryl ist, wobei R¹⁷ 1-5 Heteroatome enthalten kann, unabhängig voneinander ausgewählt aus der Gruppe bestehend aus N, O, Si, und S;
T₁, T₂ unabhängig voneinander abwesend oder ausgewählt sind aus der Gruppe bestehend aus -O-, -S-,, gesättigtem C₁-C₄-Alkylen, und ungesättigtem C₂-C₄-Alkylen, wobei das Alkylen 0-2 Heteroatome enthalten kann, unabhängig voneinander ausgewählt aus der Gruppe bestehend aus N, O, S, und Si.

Der als ROMP-Initiator eingesetzte Grubbs-Katalysator ist bevorzugt ausgewählt aus der Gruppe bestehend aus: 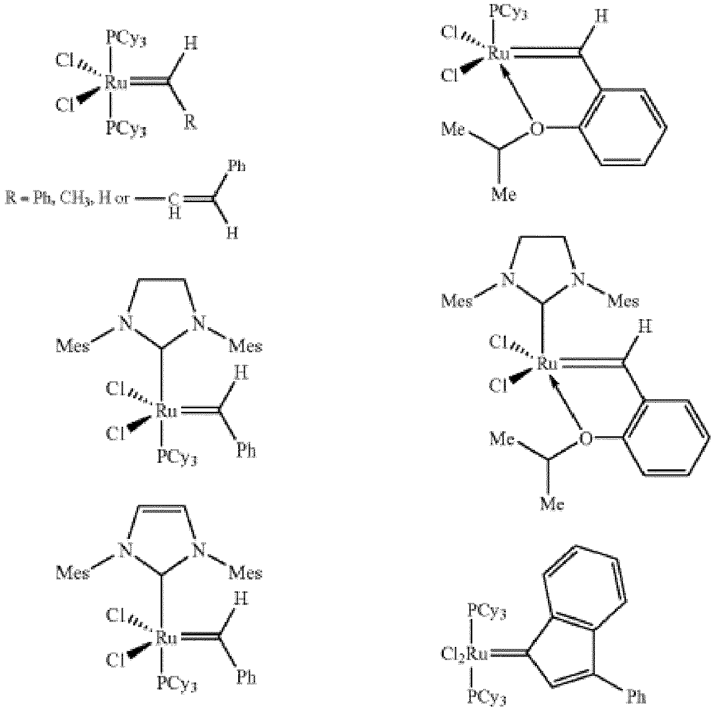 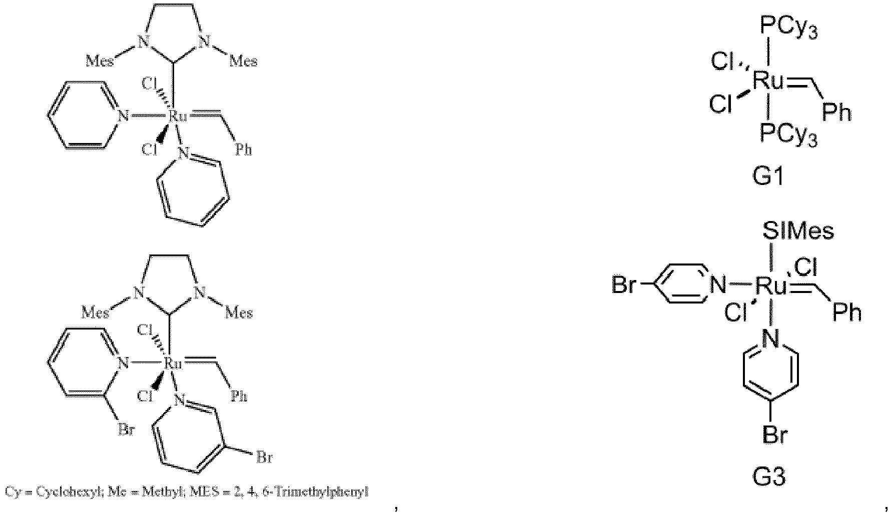 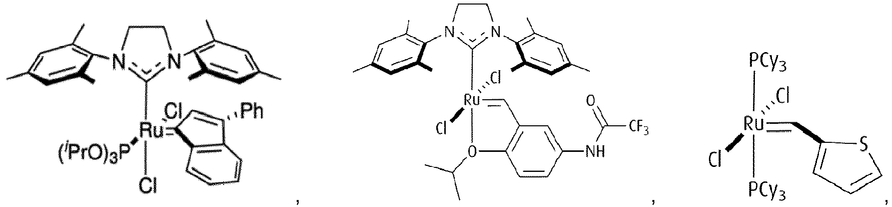 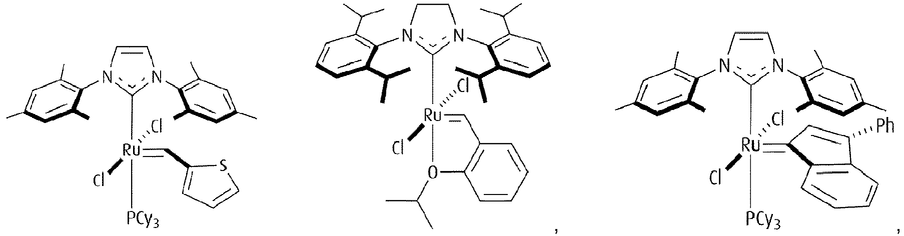 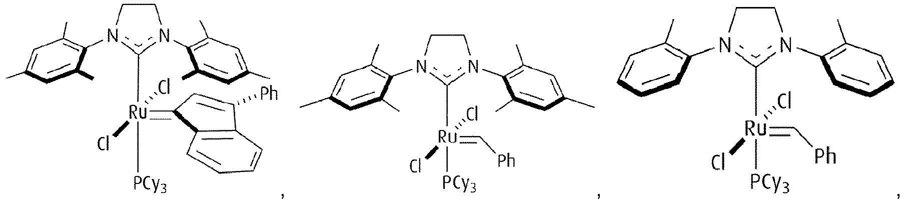 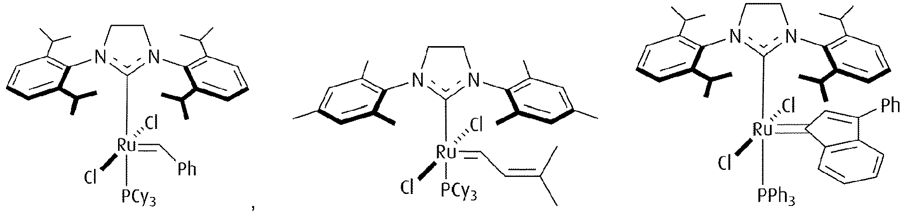 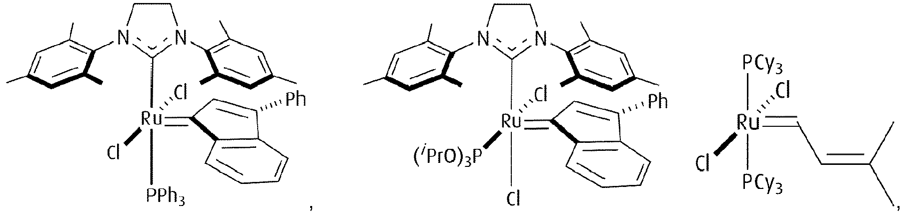 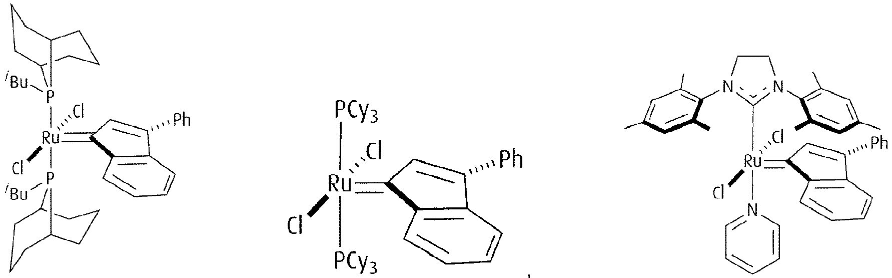 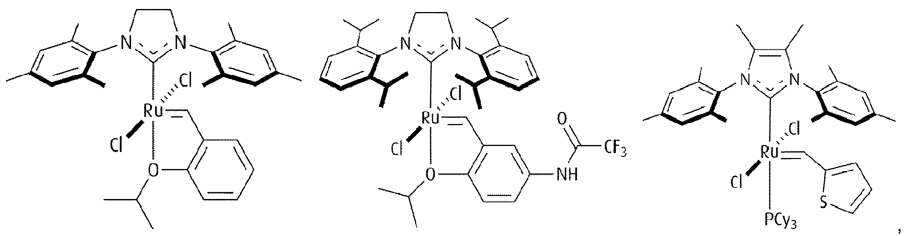 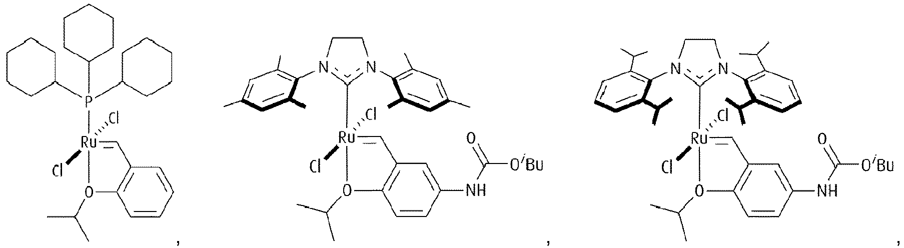 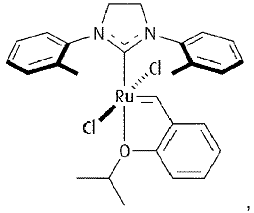 [1,3-Bis[1,3-bis(2,4,6-trimethylphenyl)-2-imidazolidinyliden]-dichloro-(3-phenyl-1H-inden-1-yliden)ruthenium(II), Bis(2,4,6-trimethylphenyl)-2-imidazolidinyliden]-dichloro-(3-phenyl-1H-inden-1-yliden)(triphenylphosphin)ruthenium(II), und [1,3-Bis(2,4,6-trimethylphenyl)-2-imidazolidinyliden]-dichloro-(3-phenyl-1H-inden-1-yliden)(pyridyl)ruthenium(II).

In einer Ausführungsform der vorliegenden Erfindung ist der Grubbs-Katalysator der Komplex G3, der Komplex M20, also [1,3-Bis(2,4,6-trimethylphenyl)-2-imidazolidinyliden]-dichloro-(3-phenyl-1H-inden-1-yliden)(triphenylphosphin)ruthenium(11), der Komplex M31, also [1,3-Bis(2,4,6-trimethylphenyl)-2-imidazolidinyliden]-dichloro-(3-phenyl-1H-inden-1-yliden)(pyridyl)ruthenium(II), oder der Hoveyda Grubbs Katalysator M722, alle beispielsweise erhältlich bei UMICORE AG & Co. KG. Bevorzugt ist der Grubbs-Katalysator M20, M31, oder M722, am bevorzugtesten als ROMP-Initiator ist M20.

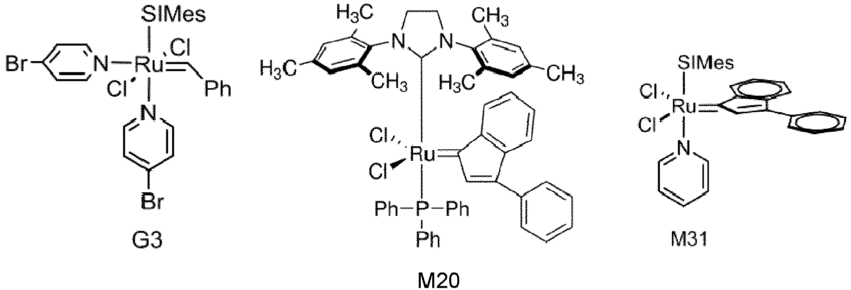

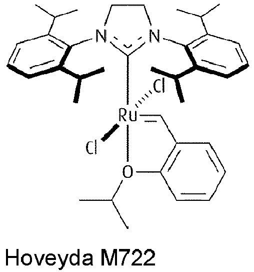

Auch Kombinationen von zwei oder mehr ROMP-Initiatoren können eingesetzt werden.

Der ROMP-Initiator liegt im erfindungsgemäßen Dual Cure-Zweikomponenten-System vorteilhafterweise getrennt vom Radikalinitiator vor. Denn der Radikalinitiator kann die Lagerstabilität des ROMP-Initiators verringern. Des Weiteren liegt der ROMP-Initiator im erfindungsgemäßen Dual-Cure-Zweikomponenten-System vorteilhafterweise in derselben Komponente wie der Radikalbeschleuniger vor, insbesondere wenn der Radikalbeschleuniger eines der hier als Radikalbeschleuniger genannten (bevorzugten) Amine ist. Dies kann die Lagerstabilität des ROMP-Initiators erhöhen. Diese Kombination wird in einer bevorzugten Ausführungsform von der Komponente B im erfindungsgemäßen Dual Cure-2K-System verwirklicht.

Der ROMP-Initiator liegt im erfindungsgemäßen Dual Cure-2K-System typischerweise in einer Menge von etwa 10 ppm bis etwa 500 ppm, bevorzugt von etwa 20 ppm bis etwa 300 ppm, bevorzugter von etwa 30 ppm bis etwa 100 ppm, noch bevorzugter von etwa 40 ppm bis etwa 70 ppm, beispielsweise etwa 50 ppm, bezogen auf die Gesamtmenge ROMP-Monomer (einschließlich des erfindungsgemäßen RadP-ROMP-Monomers), vor.

Die bevorzugtesten ROMP-Initiatoren sind die in den Beispielen verwendeten ROMP-Initiatoren, bevorzugt etwa in den in den Beispielen angegebenen Mengen und/oder Relationen zu den entsprechenden ROMP-Monomeren.

### Radikalbeschleuniger

Geeignete Radikalbeschleuniger für die Verwendung in Kombination mit einem Radikalinitiator sind dem Fachmann bekannt. Geeignete Radikalbeschleuniger sind im Kontext der vorliegenden Anmeldung zweckmäßig organische Amine.

Als Radikalbeschleuniger geeignete organische Amine sind unter folgenden Verbindungen ausgewählt, die beispielsweise in der Anmeldung US 2011/071234 A1 beschrieben sind: Dimethylamin, Trimethylamin, Ethylamin, Diethylamin, Triethylamin, *n*-Propylamin, Di-*n-*propylamin, Tri-*n*-propylamin, *Iso*-propylamin, Di-*iso*-propylamin, Tri-*iso*-propylamin, *n-*Butylamin, *Iso*-butylamin, *tert*-Butylamin, Di-*n*-butylamin, Di-*iso*-butylamin, Tri-*iso*-butylamin, *n*-Pentylamin, *Iso*-pentylamin, Di-*iso*-pentylamin, Hexylamine, Octylamine, Dodecylamine, Laurylamin, Stearylamin, Aminoethanol, Diethanolamin, Triethanolamin, Aminohexanol, Ethoxyaminoethan, Dimethyl-(2-chloroethyl)amin, 2-Ethylhexylamin, Bis-(2-chloroethyl)amin, 2-Ethylhexylamin, Bis-(2-ethylhexyl)amin, *N*-Methylstearylamin, Dialkylamine, Ethylendiamin, *N,N'*-Dimethylethylendiamin, Tetramethylethylendiamin, Diethylentriamin, Permethyldiethylentriamin, Triethylentetramin, Tetraethylenpentamin, 1,2-Diaminopropan, Dipropylentriamin, Tripropylentetramin, 1,4-Diaminobutan, 1,6-Diaminohexan, 4-Amino-1-diethylaminopentan, 2,5-Diamino-2,5-dimethylhexan, Trimethylhexamethylendiamin, *N,N-*Dimethylaminoethanol, 2-(2-Diethylaminoethoxy)ethanol, Bis-(2-hydroxyethyl)-oleylamin, Tris[2-(2-hydroxyethoxy)ethyl]amin, 3-Amino-1-propanol, Methyl-(3-aminopropyl)ether, Ethyl-(3-aminopropyl)ether, 1,4-Butandiol-bis(3-aminopropylether), 3-Dimethylamino-1-propanol, 1-Amino-2-propanol, 1-Diethylamino-2-propanol, Diisopropanolamin, Methyl-bis(2-hydroxypropyl)amin, Tris(2-hydroxypropyl)amin, 4-Amino-2-butanol, 2-Amino-2-methylpropanol, 2-Amino-2-methyl-propandiol, 2-Amino-2-hydroxymethylpropandiol, 5-Diethylamino-2-pentanon, 3-Methylaminopropionsäurenitril, 6-Aminohexansäure, 11-Aminoundecansäure, 6-Aminohexansäureethylester, 11-Aminohexansäure-isopropylester, Cyclohexylamin, *N*-Methylcyclohexylamin, *N,N*-Dimethylcyclohexylamin, Dicyclohexylamin, *N-*Ethylcyclohexylamin, *N*-(2-Hydroxyethyl)cyclohexylamin, *N*,*N*-Bis(2-hydroxyethyl)-cyclohexylamin, *N*-(3-Aminopropyl)cyclohexylamin, Aminomethylcyclohexan, Hexahydrotoluidin, Hexahydrobenzylamin, Anilin, *N*-Methylanilin, *N,N*-Dimethylanilin, *N,N*-Diethylanilin, *N,N*-Dipropylanilin, *iso*-Butylanilin, Toluidine, Diphenylamin, Hydroxyethylanilin, Bis(hydroxyethyl)anilin, Chloranilin, Aminophenole, Aminobenzoesäuren und deren Ester, Benzylamin, Dibenzylamin, Tribenzylamin, Methyldibenzylamin, β-Phenylethylamin, Xylidin, Di-*iso*-propylanilin, Dodecylanilin, Aminonaphthalin, *N*-Methylaminonaphthalin, *N,N-*Dimethylamino-naphthalin, *N,N*-Dibenzylnaphthalin, Diaminocyclohexan, 4,4'-Diaminodicyclohexyl-methan, Diamino-dimethyl-dicyclohexylmethan, Phenylendiamin, Xylylendiamin, Diaminobiphenyl, Naphthalindiamine, Toluidine, Benzidine, 2,2-Bis(aminophenyl)-propan, Aminoanisole, Aminothiophenole, Aminodiphenylether, Aminocresole, Morpholin, *N-*Methylmorpholin, *N*-Phenylmorpholin, Hydroxyethyl-morpholin, *N*-Methylpyrrolidin, Pyrrolidin, Piperidin, Hydroxyethylpiperidin, Pyrrole, *N,N*-Bisalkylarylamine, Pyridine, Chinoline, Indole, Indolenine, Carbazole, Pyrazole, Imidazole, Thiazole, Pyrimidine, Chinoxaline, Aminomorpholin, Dimorpholinethan, und [2,2,2]-Diazabicyclooctan.

Besonders bevorzugt im Kontext der vorliegenden Erfindung sind aromatischen Amine, besonders bevorzugt Aniline, Toluidine und *N,N*-Bisalkylarylamine, wie *N,N*-Dimethylanilin, *N,N*-Diethylanilin, *N,N*-Di-*iso*-propanol-*p*-toluidin (DiPpT), *N,N*-Di-hydroxyethyl-m-toluidin (DHEmT), *N,N*-Dimethyl-p-toluidin (DMpT), *N,N*-Diethyl-p-toluidin (DEpT), *N,N-*Bis(hydroxyalkyl)arylamine, *N,N*-Bis(2-hydroxyethyl)aniline, *N,N*-Bis(2-hydroxyethyl)-toluidin, *N,N*-Bis(2-hydroxypropyl)anilin, *N,N*-Bis(3-methacryloyl-2-hydroxypropyl)-*p*-toluidin, *N,N-*Dibutoxyhydroxypropyl-p-toluidin und 4,4'-Bis(dimethylamino)-diphenylmethan. Bevorzugt sind insbesondere *N,N*-Di-*iso*-propanol-*p*-toluidin (DiPpT), *N,N*-Di-hydroxyethyl-m-toluidin (DHEmT), *N,N*-Dimethyl-p-Toluidin (DMpT) und *N,N*-Diethyl-p-Toluidin (DEpT). DiPpT und DMpT sind ganz besonders bevorzugt, am bevorzugtesten ist DMpT.

Es kann ein einzelner Radikalbeschleuniger oder eine Mischung mehrerer Radikalbeschleuniger eingesetzt werden. Die Verwendung eines einzelnen Radikalbeschleunigers ist bevorzugt.

Der Radikalbeschleuniger wird typischerweise in einer Menge von etwa 0,01 bis etwa 10 Gew.-%, bevorzugt etwa 0,1 bis etwa 5 Gew.-%, bevorzugter von etwa 0,2 bis etwa 3 Gew.-%, noch bevorzugter von etwa 0,4 bis etwa 2,0 Gew.-%, noch bevorzugter von etwa 0,5 bis etwa 1,5 Gew.-%, noch bevorzugter von etwa 0,5 bis etwa 1,3 Gew.-%, noch bevorzugter von etwa 0,8 bis 1,2 Gew.-%, bezogen auf das ROMP-Monomer (wobei das ROMP-Monomer das erfindungsgemäße RadP-ROMP-Monomer einschließt), eingesetzt.

Der Radikalbeschleuniger muss zum verwendeten Radikalinitiator passen. Welche Paarungen dies sind, und in welchen Mengenverhältnissen zueinander sie eingesetzt werden können, ist dem Fachmann bekannt. Bevorzugt ist das Mengenverhältnis zwischen Radikalinitiator und Radikalbeschleuniger so, dass die Radikalpolymerisation in einer für chemische Dübel vorteilhaften Zeit vollständig ist.

In einer bevorzugten Ausführungsform ist der Radikalinitiator ein Peroxid, bevorzugt BPO. Typischerweise beträgt das Gewichtsverhältnis von Radikalbeschleuniger (der bevorzugt DMpT ist) zu Peroxid von 1:1 bis 1:10, bevorzugt von 1:3 bis 1:8, bevorzugter von 1:5 bis 1:7, beispielsweise DMpT:BPO 1:6. Bei trägergebundenem BPO wird die verwendete Menge entsprechend der korrespondierenden Menge an reinem BPO angepasst. Typischerweise beträgt das Gewichtsverhältnis von Radikalbeschleuniger (der bevorzugt DMpT ist) zu Perkadox 20S demzufolge von 1:5 bis 1:50, bevorzugt von 1:15 bis 1:40, bevorzugter von 1:25 bis 1:35, beispielsweise DMpT:Perkadox 20S 1:30.

Die bevorzugtesten Radikalbeschleuniger sind die in den Beispielen verwendeten Radikalbeschleuniger, bevorzugt etwa in den in den Beispielen angegebenen Mengen und/oder Relationen zu den entsprechenden Radikalinitiatoren.

### Inhibitor

Sowohl zur Stabilisierung als auch zur Einstellung der Reaktivität sind in einem erfindungsgemäßen Zweikomponenten-System bevorzugt ein oder mehrere Inhibitoren vorhanden. Der Inhibitor bzw. die Inhibitoren können in Komponente A oder Komponente B enthalten sein, er ist/sie sind bevorzugt nur in Komponente B enthalten.

Hierfür sind die für radikalisch polymerisierbare Verbindungen üblicherweise verwendeten Inhibitoren geeignet, wie sie dem Fachmann bekannt sind. Bevorzugt sind diese Inhibitoren unter phenolischen Inhibitoren und nicht-phenolischen Inhibitoren, insbesondere Phenothiazinen, ausgewählt.

Als phenolische Inhibitoren sind Phenole, wie 2-Methoxyphenol, 4-Methoxyphenol, 2,6-Di-tert-butyl-4-methylphenol, 2,4-Di-tert-butylphenol, 2,6-Di-tert-butylphenol, 2,4,6-Trimethylphenol, 2,4,6-Tris(dimethylaminomethyl)phenol, 4,4'-Thio-bis(3-methyl-6-tert-butylphenol), 4,4'-Isopropylidendiphenol, 6,6'-Di-tert-butyl-4,4'-bis(2,6-di-tert-butylphenol), 1,3,5-Trimethyl-2,4,6-tris(3,5-di-tert-butyl-4-hydroxybenzyl)benzol, 2,2'-Methylen-di-p-cresol, Catechole, wie Brenzkatechin, und Catecholderivate, wie Butylbrenzkatechine, wie 4-tert-Butylbrenzkatechin und 4,6-Di-tert-butylbrenzkatechin, Hydrochinone, wie Hydrochinon, 2-Methylhydrochinon, 2-tert-Butylhydrochinon, 2,5-Di-tert-butylhydrochinon, 2,6-Di-tert-butylhydrochinon, 2,6-Dimethylhydrochinon, 2,3,5-Trimethylhydrochinon, Benzochinon, 2,3,5,6-Tetrachloro-1,4-benzochinon, Methylbenzochinon, 2,6-Dimethylbenzochinon, Naphthochinon, oder Gemische von zweien oder mehreren davon, geeignet. Diese Inhibitoren sind oft Bestandteil von kommerziellen radikalisch härtenden Zweikomponenten-Systemen.

Als nicht-phenolische Inhibitoren kommen vorzugsweise Phenothiazine, wie Phenothiazin und/oder Derivate oder Kombinationen davon, oder stabile organische Radikale, wie etwa Galvinoxyl- und *N*-Oxyl-Radikale, insbesondere vom Piperidinyl-*N*-oxyl- oder Tetrahydropyrrol-*N*-oxyl-Typ, in Betracht, wie Aluminium-*N*-nitrosophenylhydroxylamin, Diethylhydroxylamin, Oxime, wie Acetaldoxim, Acetonoxim, Methylethylketoxim, Salicyloxim, Benzoxim, Glyoxime, Dimethylglyoxim, Aceton-O-(benzyloxycarbonyl)oxim, TEMPOL, TEMPO und dergleichen.

Ferner können in para-Stellung zur Hydroxylgruppe substituierte Pyrimidinol- oder Pyridinol-Verbindungen, wie sie in der Patentschrift DE 10 2011 077 248 B1 beschrieben sind, als Inhibitoren eingesetzt werden.

Als stabile *N*-Oxyl-Radikale können beispielsweise solche verwendet werden, wie sie in der DE 199 56 509 A1 und der DE 195 31 649 A1 beschrieben sind. Derartige stabile *N*-Oxyl-Radikale sind vom Typ Piperidinyl-*N*-oxyl oder Tetrahydropyrrol-*N*-oxyl oder eine Mischung daraus.

Bevorzugte stabile *N*-Oxyl-Radikale sind ausgewählt aus der Gruppe bestehend aus 1-Oxyl-2,2,6,6-tetramethylpiperidin, 1-Oxyl-2,2,6,6-tetramethylpiperidin-4-ol (ebenso als 4-Hydroxy-2,2,6,6-tetramethylpiperidinyloxyl oder TEMPOL bezeichnet), 1-Oxyl-2,2,6,6-tetramethylpiperidin-4-on (ebenso als TEMPON bezeichnet), 1-Oxyl-2,2,6,6-tetramethyl-4-carboxyl-piperidin (ebenso als 4-Carboxy-TEMPO bezeichnet), 1-Oxyl-2,2,5,5-tetramethylpyrrolidin, 1-Oxyl-2,2,5,5-tetramethyl-3-carboxylpyrrolidin (ebenso als 3-Carboxy-PROXYL bezeichnet) und Mischungen aus zwei oder mehreren dieser Verbindungen, wobei 1-Oxyl-2,2,6,6-tetramethylpiperidin-4-ol (synonym: 4-Hydroxy-2,2,6,6-tetramethyl-piperidinyloxyl oder TEMPOL) besonders bevorzugt ist.

Bevorzugt sind der oder die Inhibitoren ausgewählt aus der Gruppe bestehend aus stabilen N-Oxyl-Radikalen, Catecholen, Catecholderivaten und Phenothiazinen und ein Gemisch von zwei oder mehreren davon. Besonders bevorzugt sind der oder die Inhibitoren ausgewählt aus der Gruppe bestehend aus Tempol, Catecholen und Phenothiazinen. Noch bevorzugter ist der Inhibitor TEMPOL, bei einem Gemisch aus Inhibitoren ist bevorzugt einer davon TEMPOL.

Ganz besonders bevorzugt sind die in den Beispielen verwendeten Inhibitoren, bevorzugt etwa in den in den Beispielen angegebenen Mengen und Relationen zum RadP-ROMP-Monomer und/oder zum Radikalinitiator.

Die Inhibitoren können, abhängig von den gewünschten Eigenschaften des Zweikomponenten-Systems, entweder alleine oder als Kombination von zweien oder mehreren davon verwendet werden. Die Kombination von phenolischen und nicht-phenolischen Inhibitoren ist, wenn eine Kombination von Inhibitoren eingesetzt wird, bevorzugt.

Der Inhibitor oder die Inhibitormischung wird zugesetzt in der Fachwelt bekannten üblichen Mengen, bevorzugt in einer Menge von etwa 0,01 bis etwa 2 Gew.-%, bevorzugter von etwa 0,05 bis etwa 1 Gew.-%, bevorzugter von etwa 0,1 bis etwa 1 Gew.-%, noch bevorzugter von etwa 0,15 bis etwa 0,5 Gew.-%, beispielsweise 0,23 Gew.-% oder 0,35 Gew.-%, bezogen auf die Gesamtmenge des ROMP-Monomers (wobei das ROMP-Monomer das erfindungsgemäße RadP-ROMP-Monomer einschließt).

### Weitere optionale Inhaltsstoffe des 2K-Systems

Ein erfindungsgemäßes Zweikomponenten-System kann neben den bereits genannten Inhaltsstoffen optional noch weitere Inhaltsstoffe umfassen. Als solche optionalen Inhaltsstoffe kommen alle in 2K-Systemen üblicherweise verwendeten optionalen Inhaltsstoffe infrage, wie beispielsweise Farbstoffe, Additive (beispielsweise Verdickungsmittel oder Antioxidantien), und Reaktivverdünner.

### Reaktivverdünner

Eine oder beide Komponenten des Zweikomponenten-Systems kann - zusätzlich zu den oben beschriebenen obligatorischen Monomeren in Komponente A und B - mindestens einen Reaktivverdünner enthalten.

Bevorzugt ist der Reaktivverdünner in derjenigen Komponente enthalten, welche nicht den Radikalinitiator enthält, um keine Radikalreaktion in Gang zu setzen.

Geeignete Reaktiwerdünner sind niederviskose, radikalisch co-polymerisierbare Verbindungen, bevorzugt kennzeichnungsfreie Verbindungen. Derartige Verbindungen sind der Fachwelt bekannt.

Besonders geeignete Reaktivverdünner sind die bereits weiter oben aufgeführten Methacrylsäureester. Sie sind in bevorzugten Ausführungsformen der erfindungsgemäßen 2K-Systeme ohnedies schon obligatorisch vorhanden.

Grundsätzlich können auch andere übliche radikalisch polymerisierbare Verbindungen, allein oder im Gemisch mit den Methacrylsäureestern, als Reaktivverdünner eingesetzt werden, z. B. Styrol, α-Methylstyrol, alkylierte Styrole, wie *tert*-Butylstyrol, Divinylbenzol und Vinyl- sowie Allylverbindungen, wobei die nicht kennzeichnungspflichtigen Vertreter davon bevorzugt sind. Beispiele für derartige Vinyl- oder Allylverbindungen sind Vinylether oder -ester sowie Allylether oder -ester, beispielsweise Hydroxybutylvinylether, Ethylenglycoldivinylether, 1,4-Butandioldivinylether, Trimethylolpropandivinylether, Trimethylolpropantrivinylether, Mono-, Di-, Tri-, Tetra- und Polyalkylenglycolvinylether, Mono-, Di-, Tri-, Tetra- und Polyalkylenglycolallylether, Adipinsäuredivinylester, Trimethylolpropandiallylether und Trimethylolpropantriallylether.

Der bzw. die Reaktivverdünner ist bzw. sind bevorzugt in einer Menge von 0 bis etwa 30 Gew.-%, besonders bevorzugt von etwa 0 bis etwa 20 Gew.-%, noch bevorzugter von etwa 5 bis etwa 15 Gew.-%, bezogen auf das ROMP-Monomer (wobei das ROMP-Monomer das erfindungsgemäße RadP-ROMP-Monomer einschließt), im 2K-System vorhanden. Am bevorzugtesten ist kein weiterer Reaktivverdünner neben dem erfindungsgemäß in einigen bevorzugten Ausführungsformen enthaltenen Methacrylat vorhanden.

### Additive

Als Additive finden übliche Additive Verwendung, also Thixotropiermittel, wie gegebenenfalls organisch oder anorganisch nachbehandelte pyrogene Kieselsäure (falls sie nicht schon als Füllstoff verwendet wird), insbesondere apolar nachbehandelte pyrogene Kieselsäure (welche im Kontext der vorliegenden Erfindung bereits oben im Zusammenhang mit den Füllstoffen genauer beschrieben wurde), Bentonite, Alkyl- und Methylcellulosen, Rhizinusölderivate oder dergleichen, Weichmacher, wie Phthalsäure- oder Sebacinsäureester, Antistatikmittel, Verdickungsmittel, Antioxidantien, Flexibilisatoren, Rheologiehilfsmittel, Netzmittel, färbende Zusätze, wie Farbstoffe oder insbesondere Pigmente, beispielsweise zum unterschiedlichen Anfärben der Komponenten zur besseren Kontrolle von deren Durchmischung, oder dergleichen, oder Gemische von zwei oder mehr davon. Auch nicht reaktive Verdünnungsmittel (Lösungsmittel) können, vorzugsweise in einer Menge bis zu 30 Gew.-%, bezogen auf die Gesamtmenge der Komponente enthalten sein, wie Niederalkylketone, z.B. Aceton, Diniederalkyl-niederalkanoylamide, wie Dimethylacetamid, Niederalkylbenzole, wie Xylole oder Toluol, Phthalsäureester oder Paraffine, Wasser oder Glykole. Ferner können Metallfänger in Form von oberflächenmodifizierten pyrogenen Kieselsäuren enthalten sein. Bevorzugt ist mindestens ein Thixotropiermittel als Additiv vorhanden, besonders bevorzugt eine organisch oder anorganisch nachbehandelte pyrogene Kieselsäure, ganz besonders bevorzugt eine apolar nachbehandelte pyrogene Kieselsäure, z.B. mit Polydimethylsiloxan (PDMS) nachbehandelte pyrogene Kieselsäure. In dieser Hinsicht wird Bezug genommen auf die Anmeldungen WO 02/079341 und WO 02/079293 sowie WO 2011/128061 A1, deren diesbezüglicher Inhalt hiermit in diese Anmeldung aufgenommen wird.

### Haftvermittler

In einer Ausführungsform kann das Zweikomponenten-System zusätzlich einen Haftvermittler enthalten. Durch den Einsatz eines Haftvermittlers wird die Vernetzung der Bohrlochwand mit der Dübelmasse verbessert, so dass sich die Haftung im ausgehärteten Zustand erhöht. Dies ist für die Verwendung einer Zweikomponenten-Dübelmasse z.B. in mit einem Diamantbohrer gebohrten Bohrlöchern von Bedeutung und erhöht die Versagensverbundspannung. Geeignete Haftvermittler sind aus der Gruppe der Silane, die mit weiteren reaktiven organischen Gruppen funktionalisiert sind und in das Polymernetzwerk eingebunden werden können, ausgewählt. Diese Gruppe umfasst z.B. 3-(Meth)acryloyloxypropyltrimethoxysilan, 3-(Meth)acryloyloxypropyltriethoxysilan, 3-(Meth)acryloyloxymethyltrimethoxysilan, 3-(Meth)-acryloyloxymethyltriethoxysilan, Vinyltrimethoxysilan, Vinyltriethoxysilan, funktionalisiertes Tetraethoxysilan, funktionalisiertes Tetramethoxysilan, funktionalisiertes Tetrapropoxysilan, funktionalisiertes Ethyl- oder Propylpolysilikat, und Gemische von zwei oder mehr davon. In dieser Hinsicht wird Bezug genommen auf die Anmeldung DE 10 2009 059210, deren diesbezüglicher Inhalt hiermit in diese Anmeldung aufgenommen wird.

Der Haftvermittler ist zweckmäßig in Mengen von etwa 1 bis etwa 10 Gew.-% bezogen auf das Gesamtgewicht der jeweiligen Komponente enthalten.

### Verwendung des Zweikomponenten-Systems als chemischer Dübel

Die erfindungsgemäßen 2K-Systeme, sowohl das Dual Cure 2K-System als auch das Single Cure 2K-System, sind in vielen Bereichen, bei denen sonst üblicherweise Methacrylate Verwendung finden, einsetzbar.

Die vorliegende Erfindung betrifft jedoch insbesondere die Verwendung eines hier beschriebenen Zweikomponenten-Systems zur chemischen Befestigung eines Verankerungsmittels in einem Bohrloch, also als chemischer Dübel. Bei einer bevorzugten Ausführungsform des Zweikomponenten-Systems enthält die Komponente A und/oder die Komponente B zusätzlich zum polymerisierbaren Monomer einen Füllstoff wie hierin beschrieben. Dieser Füllstoff erhöht die Festigkeit des chemischen Dübels im Bohrloch.

Das Zweikomponenten-System umfasst die Komponente A und die Komponente B reaktionsinhibierend getrennt in unterschiedlichen Behältern, beispielsweise einer Mehrkammer-Vorrichtung, wie eine Mehrkammer-Patrone und/oder -Kartusche, aus welchen Behältern die beiden Komponenten durch Einwirkung mechanischer Presskräfte oder unter Einwirkung eines Gasdruckes ausgepresst und vermischt werden. Eine weitere Möglichkeit besteht darin, das Zweikomponenten-System als Zweikomponenten-Kapseln zu konfektionieren, die in das Bohrloch eingeführt werden und durch schlagdrehendes Setzen des Befestigungselements zerstört werden unter gleichzeitiger Durchmischung der beiden Komponenten der Mörtelmasse. Vorzugsweise wendet man ein Patronensystem oder ein Injektionssystem an, bei dem die beiden Komponenten aus den getrennten Behältern ausgepresst und durch einen statischen Mischer geführt werden, in dem sie homogen vermischt und dann über eine Düse vorzugsweise direkt in das Bohrloch ausgetragen werden.

Das erfindungsgemäße 2K-System kann in Form eines Patronen-Systems, eines Kartuschen-Systems oder eines Folienbeutel-Systems verwendet werden. Bei der bestimmungsgemäßen Verwendung des Systems werden die Komponenten entweder unter Einwirkung mechanischer Kräfte oder durch Gasdruck aus den Patronen, Kartuschen oder Folienbeuteln ausgepresst und miteinander vermischt, vorzugsweise mit Hilfe eines Statikmischers, durch den die Bestandteile hindurchgeführt werden. Die entstandene Mischung kann dann als Befestigungsmittel oder für einen anderen Zweck verwendet werden. Wird das 2K-System als chemischer Dübel verwendet, so wird die Mischung unmittelbar nach dem Vermischen in beispielsweise ein Bohrloch eingeführt, wonach die zu befestigenden Einrichtungen, wie Ankergewindestangen und dergleichen, in das mit der aushärtenden Mischung beschickte Bohrloch eingebracht und entsprechend justiert werden.

Das erfindungsgemäße Zweikomponenten-System enthält die Komponente A und die Komponente B typischerweise in einem Gewichtsverhältnis von etwa 1:1 bis etwa 20:1. Bevorzugt enthält das erfindungsgemäße Zweikomponenten-System die Komponente A und die Komponente B in einem Gewichtsverhältnis von etwa 3:1 bis etwa 15:1, mehr bevorzugt von etwa 4:1 bis etwa 12:1. Besonders bevorzugt enthält das erfindungsgemäße Zweikomponenten-System die Komponente A und die Komponente B in einem Gewichtsverhältnis von etwa 10:1. Dieselben Gewichtsverhältnis-Bereiche sind im erfindungsgemäßen Dual Cure 2K-System für das Verhältnis von ROMP-Monomer zu Methacrylat bevorzugt.

Das erfindungsgemäße 2K-System findet vor allem im Baubereich Verwendung, etwa zur Instandsetzung von Beton, als Polymerbeton, als Beschichtungsmasse oder als kalthärtende Straßenmarkierung. Besonders eignet es sich zur chemischen Befestigung von Verankerungsmitteln, wie Ankern, Gewindestangen, Bewehrungseisen, Schrauben und dergleichen, in Bohrlöchern, insbesondere in Bohrlöchern in verschiedenen Untergründen, insbesondere mineralischen Untergründen, wie solche auf der Grundlage von Beton, Porenbeton, Ziegelwerk, Kalksandstein, Sandstein, Naturstein, Glas und dergleichen, und metallischen Untergründen, wie solche aus Stahl. In einer Ausführungsform ist der Untergrund des Bohrlochs Beton, und das Verankerungsmittel besteht aus Stahl oder Eisen. In einer weiteren Ausführungsform ist der Untergrund des Bohrlochs Stahl, und das Verankerungsmittel besteht aus Stahl oder Eisen.

Die Erfindung wird im Folgenden anhand einer Reihe von Beispielen näher erläutert. Die Erfindung ist jedoch nicht auf die spezifischen in den Beispielen gezeigten Ausführungsformen beschränkt.

### BEISPIELE

Alle hier aufgelisteten Chemikalien und Bestandteile der Zusammensetzungen sind kommerziell erhältlich und wurden in der kommerziell üblichen Qualität eingesetzt, soweit nicht anders angegeben.

Der Komplex M20, also [1,3-Bis(2,4,6-trimethylphenyl)-2-imidazolidinyliden]-dichloro-(3-phenyl-1H-inden-1-yliden)(triphenylphosphin)ruthenium(II), verwendet als ROMP-Initiator, war von UMICORE AG & Co. KG.

Perkadox 20S besteht aus 20 Gew.-% BPO auf inerten Füllstoffen und ist von Akzo Nobel, Niederlande, erhältlich.

Alle in den Beispielen gemachten %- und ppm-Angaben im Zusammenhang mit Mengenangaben beziehen sich auf das Gesamtgewicht der beschriebenen Zusammensetzung als Kalkulationsbasis, soweit nicht anders angegeben.

Für Dünnschichtchromatographie wurde Silicagel 60 F254 auf Aluminium verwendet (Merck). Visualisierung wo nicht anders angegeben mit UV-Licht (254 nm).
Silicagel 60 (220-440 mesh ASTM) wurde für Säulenchromatographie verwendet.
¹H- und ¹³C-NMR-Messungen wurden bei 25°C auf einem Bruker Avarice 300 MHz durchgeführt (¹H: 300,36 MHz, ¹³C: 75,53 MHz). Die ppm-Werte sind relativ zu Tetramethylsilan (TMS).

### Beispiel 1: Synthese von norbornenhaltigen Monomeren

### Beispiel 1.1: Diels-Alter-Reaktion

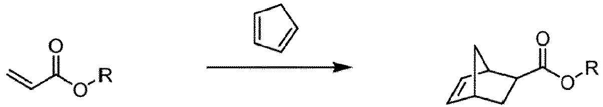

Frisch destilliertes Cyclopentadien (>1,3 eq) wurde tropfenweise zum jeweiligen eisgekühlten Acrylat (1,0 eq) zugegeben. Nach 2 Stunden wurde die Eiskühlung entfernt und die Mischung bei Raumtemperatur gerührt. Da die Reaktion exotherm verlief, wurde nochmal 15 Minuten mit Eis gekühlt. Danach wurde keine weitere Exothermie beobachtet und die Mischung wurde über Nacht bei 40°C gerührt. Vollständiger Umsatz wurde mittels ¹H-NMR-Spektroskopie bestätigt. Überschüssiges Cyclopentadien und das Nebenprodukt Dicyclopentadien wurden durch Flash-Säulenchromatographie mit Cy/EtOAc 1:0 (v/v), dann 5:1 (v/v) als Eluent entfernt. Das Verhältnis end/exo wurde mittels ¹H-NMR-Spektroskopie ermittelt.

Herstellung von Mon9 (2-Hydroxyethyl-*endo,exo*-5-norbornencarboxylat) 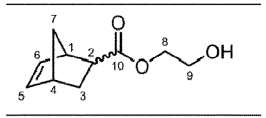 Edukte: Cyclopentadien (45,0 mL, 35,40 g, 0,535 mol, 1,3 eq), 2-Hydroxyethylacrylat (40,0 mL, 44,33 g, 0,382 mol, 1,0 eq).
Ausbeute: 66,78 g (96%), gelbliche Flüssigkeit, endo/exo: 80/20
Dünnschichtchromatographie (TLC): R_{f} = 0,62 (Cy/EtOAc:, 1:1, (v:v), Detektion: KMnO₄)
¹H-NMR (300 Hz, CDCl₃): δ = 6,21 - 6,18 (m, 1H, 6_{endo}), 6,16 - 6,08 (m, 2H, 5ₑₓₒ, 6ₑₓₒ), 5,95-5,92 (m, 1H, 5_{endo}), 4,24 - 4,21 (t, 2H, 8ₑₓₒ), 4,17 - 4,14 (t, 2H, 8_{endo}), 3,84 - 3,77 (m, 2H, 9_{endo/exo}), 3,22 (s, 1H, 1_{endo}), 3,05 (s, 1H, 1ₑₓₒ), 3,02 - 2,96 (m, 1H, 2_{endo}), 2,91 (s, 1H, 4_{endo/exo}), 2,29 - 2,24 (m, 1H, 2ₑₓₒ), 2,08 (s, b, OH), 1,96 - 1,87 (m, 1H, 3a_{endo/exo}), 1,54 - 1,51 (d, 1H, 3bₑₓₒ), 1,45 - 1,35 (m, 2H, 7_{endo/exo}), 1,29 - 1,26 (d, 1H, 3b_{endo}) ppm.
¹³C-NMR (75 Hz, CDCl₃): δ = 176,7 (1C, C_{q}, 10ₑₓₒ), 175,2 (1C, C_{q}, 10_{endo}), 138,2 (1C, 6ₑₓₒ), 137,9 (1C, 6_{endo}), 135,7 (1C, 5bₑₓₒ), 132,2 (1C, 5b_{endo}), 66,1 (1C, 8ₑₓₒ), 65,9 (1C, 8_{endo}), 61,4 (2C, 9_{endo/exo}), 49,7 (1C, 7_{endo}), 46,7 (1C, 7ₑₓₒ), 46,4 (1C, 1ₑₓₒ), 45,8 (1C, 1_{endo}), 43,3 (1C, 2_{endo}), 43,1 (1C, 2ₑₓₒ), 42,6 (1C, 4_{endo}), 41,7 (1C, 4ₑₓₒ), 30,3 (1C, 3ₑₓₒ), 29,3 (1C, 3_{endo}) ppm.

### Beispiel 1.2: Veresterungsreaktionen

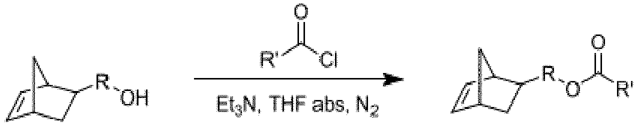

Hydroxy-funktionalisiertes Norbornen (1,0 eq), absolutes Triethylamin (1,5 eq) und absolutes Tetrahydrofuran wurden in Inertatmosphäre unter Eiskühlung gemischt. Eine vorgekühlte, farblose Säurechloridlösung (1,5 eq; z.B. Methacryloylchlorid) in THF abs. wurde über 45 Minuten hinweg tropfenweise zugegeben und die Bildung eines farblosen Präzipitats (Triethylammoniumchlorid) wurde beobachtet. Die Reaktion wurde für eine Stunde im Eisbad fortgesetzt, und dann über Nacht bei Raumtemperatur. Vollständiger Umsatz zum jeweiligen Ester wurde mit TLC überwacht. Das Lösemittel wurde im Vakuum entfernt und der Rückstand wurde in Diethylether und einer wässrigen HCI-Lösung (5 vol%) aufgenommen. Die Phasen wurden getrennt und die organische Phase nochmals mit der HCI-Lösung gewaschen. Die organische Phase wurde dann mit einer wässrigen Lösung von Natriumhydroxid (0,5 M) gewaschen, um restliche freie Säure zu entfernen, welche sich aus dem Säurechloridüberschuss während der Aufarbeitung gebildet hat. Der Restsäuregehalt wurde durch ¹H-NMR-Spektroskopie bestimmt. Die Lösung wurde über Natriumsulfat-Anhydrid getrocknet und das Lösemittel im Vakuum entfernt.

Herstellung von Mon13 (2-(Methacryloyloxy)ethyl-*endo,exo*-5-norbornencarboxylat) 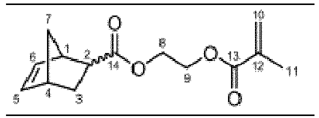 Edukte: Mon9 (97,50 g, 0,535 mol, 1,0 eq), Methacryloylchlorid (78,4 ml, 83,90 g, 0,803 mol, 1,5 eq), Triethylamin (111,25 mL, 81,22 g, 0,803 mol, 1,5 eq), Tetrahydrofuran (ca. 500 mL).
Ausbeute: 124,56 g (93%), klare, gelbliche Flüssigkeit, endo/exo: 80/20
Dünnschichtchromatographie (TLC): R_{f} = 0,71/0,79 (Cy/EtOAc, 3:1, (v:v), Detektion: KMnO₄)
¹H-NMR (300 Hz, CDCl₃): δ = 6,22 - 6,15 (m, 1H, 6_{endo}), 6,15 - 6,06 (m, 1H, 5ₑₓₒ, 6ₑₓₒ; s, 1H, 10ₜᵣₐₙₛ), 5,94 - 5,86 (m, 1H, 5_{endo}), 5,59 (s, 1H, 10_{cis}), 4,40 - 4,17 (m, 4H, 8_{endo/exo}, 9_{endo/exo}), 3,19 (s, 1H, 1_{endo}), 3,03 (s, 1H, 1ₑₓₒ), 3,00 - 2,93 (m, 1H, 2_{endo}), 2,90 (s, 1H, 4_{endo/exo}), 2,28 - 2,21 (m, 1H, 2ₑₓₒ), 1,95 (s, 3H, 11), 1,92 - 1,83 (m, 1H, 3a_{endo}), 1,55 - 1,46 (d, 1H, 3aₑₓₒ), 1,46 - 1,31 (m, 2H, _{7endo/exo}, 1H, 3bₑₓₒ), 1,31 - 1,18 (d, 1H, 3b_{endo}) ppm.
¹³C-NMR (75 Hz, CDCl₃): δ = 176,1 (1C, C_{q}, 14ₑₓₒ), 174,6 (1C, C_{q}, 10_{endo}), 167,2 (1C, C_{q}, 13), 138;2 (1C, 6ₑₓₒ), 138,0 (1C, 6_{endo}), 136,1 (1C, Cq, 12), 135,8 (1C, 5ₑₓₒ), 132,4 (1C, 5_{endo}), 126,10 (1C, 10), 62,6 (1C, 8_{endo}), 62,2 (1C, 8ₑₓₒ), 62,0 (1C, 9_{endo,exo}), 49,7 (1C, 7_{endo}), 46,8 (1C, 7ₑₓₒ), 46,5 (1C, 1ₑₓₒ), 45,8 (1C, 1_{endo}), 43,4 (1C, 2_{endo}), 43,2 (1C, 2ₑₓₒ), 42,7 (1C, 4_{endo}), 41,8 (1C, 4ₑₓₒ), 30,4 (1C, 3ₑₓₒ), 29,4 (1C, 3_{endo}), 18,4 (1C, 11) ppm.

### Herstellung von Mon14 (endo,exo-5-Norbornen-2-methylmethacrylat)

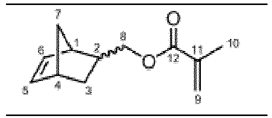

Edukte: endo,exo-5-Norbornen-2-methanol (97,37 mL, 100,0 g, 0,805 mol, 1,0 eq), Methacryloylchlorid (118,0 ml, 126,26 g, 1,208 mol, 1,5 eq), Triethylamin (167,44 mL, 122,23 g, 1,208 mol, 1,5 eq), Tetrahydrofuran (ca. 500 mL).
Ausbeute: 121,42 g (78%), klare, gelbliche Flüssigkeit, endo/exo: 76/24 Dünnschichtchromatographie (TLC): R_{f} = 0,76 (Cy/EtOAc, 5:1, (v:v), Detektion: KMnO₄) ¹H-NMR (300 Hz, CDCl₃): δ = 6,22 - 6,13 (m, 1H, 5_{endo}), 6,13 - 6,02 (m, 1H, 5ₑₓₒ, 6ₑₓₒ; s, 1H, 10ₜᵣₐₙₛ), 6,03 - 5,87 (m, 1H, 5_{endo}), 5,54 (s, 1H, 10_{cis}), 4,23 - 3,99 (m, 2H, 8ₑₓₒ), 3,99 - 3,64 (m, 2H, 8_{endo}), 2,90 (s, 1H, 1_{endo}), 2,82 (s, 1H, _{4endo/exo}), 2,72 (s, 1H, 1ₑₓₒ), 2,53 - 2,36 (m, 1H, 2_{endo}), 1,95 (s, 3H, 11), 1,91 - 1,80 (m, 1H, 3a_{endo}), 1,80 - 1,70 (m, 1H, 2ₑₓₒ), 1,51 - 1,41 (m, 1H, 7a_{endo}), 1,41 - 1,31 (m, 1H, 7a,bₑₓₒ), 1,31 - 1,24 (m, 1H, 7b_{endo}), 1,24 - 1,11 (m, 1H, 3a,bₑₓₒ), 0,65 - 0,52 (m, 1H, 3b_{endo}) ppm.
¹³C-NMR (75 Hz, CDCl₃): δ = 167,7 (1C, C_{q}, 12ₑₓₒ), 167,6 (1C, C_{q}, 12_{endo}), 137,7 (1C, 5_{endo}), 137,1 (1C, 5ₑₓₒ), 136,7 (1C, C_{q}, 11_{endo}), 136,6 (1C, C_{q}, 11ₑₓₒ), 136,4 (1C, 6ₑₓₒ), 132,3 (1C, 6_{endo}), 125,4 (1C, 10ₑₓₒ), 125,2 (1C, 10_{endo}), 68,9 (1C, 8ₑₓₒ), 68,2 (1C, 8_{endo}), 49,5 (1C, 7_{endo}), 45,1 (1C, 7ₑₓₒ), 44,1 (1C, 1_{endo}), 43,8 (1C, 1ₑₓₒ), 42,3 (1C, 4_{endo}), 41,7 (1C, 4ₑₓₒ), 38,1 (1C, 2ₑₓₒ), 38,0 (1C, 2_{endo}), 29,7 (1C, 3ₑₓₒ), 29,1 (1C, 3_{endo}), 18,5 (1C, 9) ppm.

### Beispiel 2: Single Cure RadP und Dual Cure RadP-ROMP - Schrumpftests

### Beispiel 2.1: Herstellung der Formulierungen

Die für die Schrumpftests verwendeten Formulierungen wurden wie folgt hergestellt:

### 2.1.1: Single Cure RadP-Polymerisation

Ein Ultraschallbad wurde verwendet, um BPO in der in Tabelle 1 angegebenen Menge Monomer in einem 2,5 mL Glasgefäß aufzulösen, um Komponente A zu bilden. Die restliche Menge Monomer wurde mit DMpT in einem anderen 2,5 mL Glasgefäß vermischt, um Komponente B zu bilden. Komponente A wurde mit einer Glaspipette zu Komponente B gegeben, und die Komponenten wurden durch Schütteln mit der Hand homogen durchmischt. Die verwendeten Mengen der Bestandteile der Komponenten A und B sind in Tabelle 1 zusammengefasst.

**Tabelle 1**

| | **Bestandteil** | **Menge in g** |
|---|---|---|
| **Komponente A** | Monomer | 1,00 |
| | BPO | 0,015 |
| **Komponente B** | Monomer | 0,5 |
| | DMpT | 0,0075 |

### 2.1.2: Dual Cure RadP-ROMP-Polymerisation

Ein Ultraschallbad wurde verwendet, um BPO in der in Tabelle 2 angegebenen Menge Monomer in einem 2,5 mL Glasgefäß aufzulösen, um Komponente A zu bilden. Die restliche Menge Monomer wurde mit DMpT in einem anderen 2,5 mL Glasgefäß vermischt, um Komponente B zu bilden.

Eine M20-Stammlösung wurde in Dichlormethan (50 ppm, bezogen auf die Gesamtmenge Monomer/100 µL) hergestellt. Komponente A und ein Aliquot der M20-Stammlösung (Komponente C) wurden gleichzeitig zu Komponente B gegeben, und die Komponenten wurden durch Schütteln mit der Hand homogen durchmischt.

Die verwendeten Mengen der Bestandteile der Komponenten A, B und C sind in Tabelle 2 zusammengefasst.

**Tabelle 2:**

| | **Bestandteil** | **Menge in g** |
|---|---|---|
| **Komponente A** | Monomer | 1,00 |
| | BPO | 0,015 |
| **Komponente B** | Monomer | 0,5 |
| | DMpT | 0,0075 |
| **Komponente C** | M20 | 50 ppm relativ zur Monomermenge |
| | DCM | 100 µl |

### Beispiel 2.2: Schrumpftests

Exakt 1,0 mL der Formulierung wurde mit einer Spritze in ein 3,0-mL-Glasröhrchen überführt. Das Glasröhrchen war bei 1,0 mL und 2,0 mL Füllhöhe mit einem Strich markiert. Es wurde für 24 Stunden bei Raumtemperatur ausgehärtet. Um die volumetrische Schrumpfung zu ermitteln, wurde so viel Wasser wie nötig zugegeben, um bis zur 2,0 mL Markierung aufzufüllen. Dieses Wasser wurde dann mit einer skalierten Spritze gemessen.

Tabelle 3 zeigt die eingesetzten Monomere und die damit erzielten Ergebnisse. Single Cure mit dem RadP-ROMP-Monomer Mon13 führte zu geringerem Schrumpf als Single Cure RadP mit dem Referenz-Monomer HEMA. Dual Cure RadP-ROMP führte zu geringerem Schrumpf als Single Cure RadP mit dem Referenz-Monomer HEMA.

**Tabelle 3:**

| **Monomer(e)** | **Single Cure RadP** | | **Dual Cure RadP-ROMP** | |
|---|---|---|---|---|
| | **Schrumpf in %** | **Probenzahl** | **Schrumpf in %** | **Probenzahl** |
| **HEMA** | 10,0 ± 0,0 | 5 | - | - |
| **Mon13** | 4,8 ± 1,6 | 3 | - | - |
| **Mon14** | 5,7 ± 2,2 | 6 | - | - |
| **HEMA + Mon9, Molverhältnis 1:1** | 3,8 ± 1,0 | 4 | 3,0 ± 1,6 | 5 |
| **HEMA + Mon13 Molverhältnis 1:1** | 6,0 ± 1,0 | 3 | 2,2 ± 0,8 | 5 |
| **HEMA + Mon14 Molverhältnis 1:1** | 8,5 ± 1,9 | 6 | 2,0 | 1 |

### Beispiel 3: Dual Cure RadP-ROMP-Härtungstests zur Bestimmung des Einflusses der Menge von Tempol und ROMP-Initiator

Mon13 und Mon14 wurden mit Perkadox 20S in einem 40 mL Glasgefäß gemischt und mit der Hand verrührt, um eine homogene Mischung zu erhalten, die Komponente A. Eine Stammlösung von Tempol in BDDMA wurde hergestellt, indem diese beiden Verbindungen in einem 40 mL Glasgefäß mit einem Magnetrührer gemischt wurden. Die Mischung wurde bei 50°C für 15 min gerührt. Die exakte benötigte Menge von M20 wurde in einem Aliquot der Tempol/BDDMA-Stammlösung in einem 20 ml Glasgefäß aufgelöst. DMpT wurde volumetrisch zu dieser Lösung zugegeben, was Komponente B ergab. Diese Komponente wurde mit einer Spritze aufgenommen und schnell zu Komponente A gegeben. Die beiden Komponenten wurden homogen vermischt durch Schütteln mit der Hand. Die Gewichtsanteile werden in Tabelle 4 aufgelistet; der Gelierungsprozeß wird in Tabelle 5 verfolgt. Die Viskositätsänderung und die Temperaturentwicklung wurden beobachtet.

**Tabelle 4:**

| **Komponente A** | **Bestandteil** | **Menge** | **Anteil bezogen auf die Menge Mon13/Mon14** |
|---|---|---|---|
| | Mon13/Mon14 | 10 g | 100 Gew.-% |
| | Perkadox 20S | 3 g | 30 Gew.-% |

| **Komponente B** | | | |
|---|---|---|---|
| | M20 | s. Tabelle 5 | 0-200 ppm |
| | BDDMA | 0,5 mL | 5 Gew.-% |
| | Tempol | 23-35 mg | 0,23-0,35 Gew.-% |
| | DMpT | 111 µL | 1,05 Gew.-% |

**Tabelle 5:**

| **Tempol [mg]** | **M20 [ppm (mg)]** | **Zeit ab Mischen [min]** | **Härtungsverhalten** |
|---|---|---|---|
| **Mon13 = Monomer** | | | |
| 23 | 0 (0) | 4 | Geliert |
| | | 5,5 | Fester, heiss |
| 23 | 25 (0,93) | 3,5 | Geliert |
| | | 4 | Fest |
| 23 | 50 (1,96) | 3,5 | Geliert |
| | | 4 | Fest |
| 23 | 75 (2,79) | 3,5 | Geliert |
| | | 4 | Fest |
| | | 4,5 | Heiss |
| 23 | 100 (3,72) | 3,5 | Geliert |
| | | 4 | Fest |
| 23 | 150 (5,58) | 2,5 | Geliert, 25°C |
| | | 3 | Fest, 40°C |
| | | 3,5 | Fest, heiss |
| 23 | 200 (7,44) | 2,5 | Geliert |
| | | 3 | Fest, heiss |
| 30 | 0 (0) | 6,5 | Geliert |
| | | 8 | Fest, heiss |
| 35 | 0 (0) | 9,3 | Geliert |
| | | 12 | Fest, sehr elastisch |

| **Mon 14 = Monomer** | | | |
|---|---|---|---|
| 23 | 0(0) | 16 | Geliert |
| | | 20 | Fest |
| 23 | 50 (2,42) | 1 | Erhöhte Viskosität |
| | | 4 | Hohe Viskosität |
| | | 9 | Geliert |
| | | 10,5 | Fest, heiss |
| 23 | 60 (2,91) | 0,5 | Erhöhte Viskosität |
| | | 2 | Hohe Viskosität |
| | | 7 | Geliert |
| | | 10,5 | Fest, 40°C |
| | | 11 | Heiss |
| 23 | 75 (3,63) | 2 | Hohe Viskosität |
| | | 7 | Geliert |
| | | 8,5 | Fest, heiss |
| 23 | 100 (4,84) | 0,5 | Erhöhte Viskosität |
| | | 3 | Hohe Viskosität |
| | | 7 | Fest, heiss |

Diese Ergebnisse belegen, dass das Aushärtungsverhalten durch Variation der Menge an ROMP-Initiator und Inhibitor (Tempol) einstellbar ist.

### Beispiel 4: Dual Cure RadP-ROMP verglichen mit Single Cure RadP - Geltimer-Messungen

Geltimer-Messungen wurden durchgeführt, um die Gelzeit verschiedener Formulierungen mit ("gefüllt") und ohne Füllstoffen ("ungefüllt") zu ermitteln.

### 4.1: Herstellung der Formulierungen

Die ungefüllten und gefüllten Formulierungen (Tabelle 6) wurden auf dieselbe Weise wie in Beispiel 3 hergestellt. Zur Herstellung der gefüllten Formulierungen wurde die Gesamtmenge an Füllstoffen zur Komponente A hinzugefügt. Die Zutaten wurden in einem Speedmixer homogen durchmischt und Komponente A wurde bei konstant 25°C gelagert. Dann wurde Komponente B zugegeben und erneut mit einem Speedmixer homogenisiert. Das Mischungsverhältnis war A:B:C 20:20:60 (w/w). Zur Herstellung der ungefüllten Formulierungen wurde genauso verfahren, aber die Füllstoffe wurden weggelassen.

**Tabelle 6:**

| | **Bestandteil** | **Menge in g** | **Anteil bezogen auf die Menge Mon13/Mon14** |
|---|---|---|---|
| **Komponente A** | Mon13/Mon14 | 50,00 | 100 Gew.-% |
| | Perkadox 20S | 15,0 | 30 Gew.-% |
| **Komponente B** | M20 | 0,0093 / 0,0121 | 50 ppm |
| | BDDMA | 1,023 | 2 Gew.-% |
| | Tempol | 0,175/0,115 | 0,35/0,23 Gew.-% |
| | DMpT | 0,522 | 1,05 Gew.-% |
| **Füllstoffe** | Cab-O-Sil TS-720 | 3,90 | 7,8 Gew.-% |
| | Quarzsand F32 | 48,35 | 96,7 Gew.-% |
| | Millisil W12 | 26,65 | 53,3 Gew.-% |

### 4.2: Geltimer-Messungen

Der GELNORM^{®}-Geltimer PS-1 von H. Saur Laborbedarf ("Geltimer") wurde verwendet. Die Formulierungen wurden in Glasröhrchen gefüllt, welche in einen Thermostat mit konstant 25°C gesetzt wurden (Eco RE2025 von Lauda Dr. R. Wobser GmbH & Co. KG). Das Temperaturprofil wurde mit dem Sensor PT 100 von Pico Technology gemessen. Außerdem wurde parallel in einem weiteren Glasröhrchen mit einem mechanischen Sensor (MK-B-10) gemessen.

Die Mischung wurde hierzu nach Zugabe des Initiators bis zu einer Höhe von 4 cm unterhalb des Randes in ein Teströhrchen gefüllt, wobei das Teströhrchen bei einer Temperatur von 25°C gehalten wurde (DIN 16945, DIN EN ISO 9396). Ein Glasstab oder eine Spindel wurde mit 10 Hüben pro Minuten in der Mischung auf und ab bewegt. Die Gelzeit entsprach bei der Messung des Temperaturprofils der Zeitspanne nach Zugabe des Initiators, nach der eine Temperatur von 35°C (also +10°C ausgehend von der Starttemperatur 25°C) bzw. 50°C in der Mischung gemessen wurde. Bei der mechanischen Messung wurde als Gelzeit die Zeitspanne nach Zugabe des Initiators ermittelt, nach der sich der Sensor nicht mehr bewegen ließ.

Die Ergebnisse zeigt Tabelle 7 (GTT: Gelzeit aus Temperaturprofil; GTM: Gelzeit mit mechanischem Sensor); als Referenz wurde der chemische Dübel HIT-HY150 (Komponente A enthält 1,4-Butandiol-dimethacrylat, 2-Hydroxypropyl-methacrylat und ein proprietäres Urethandimethacrylat; Komponente B enthält Dibenzoylperoxid) von Hilti verwendet:

**Tabelle 7:**

| **System** | **Monomer** | **ungefüllt/ gefüllt** | **GTT_{+10°C} [min]** | **GTT_{50°C} [min]** | **GTM [min]** |
|---|---|---|---|---|---|
| Dual Cure RadP-ROMP | Mon13 | ungefüllt | 5,83 | 6,65 | 6,35 |
| | | gefüllt | 9,57 | 10,68 | - |
| | Mon14 | ungefüllt | 3,73 | 7,21 | 7,15 |
| | | gefüllt | 11,37 | 12,15 | - |
| RadP (Referenz) | HIT-HY-150 | ungefüllt | 5,10 | 5,55 | 4,31 |
| | | gefüllt | 5,12 | 5,35 | 5,20 |

| | | | | | |
|---|---|---|---|---|---|
| "ungefüllt": ohne Füllstoffe; "gefüllt": mit Füllstoffen | | | | | |

Die Gelzeit der Dual Cure-Proben war meist etwas länger als die Gelzeit der radikalisch gestarteten Referenz. Sie befand sich jedoch im für den geplanten Einsatz als chemischer Dübel erforderlichen Zeitrahmen.

### Beispiel 5: Dual Cure RadP-ROMP und Single Cure RadP - Auszugstests und Geltimer-Messungen bei Verwendung in 10+1 Kartuschen

Gefüllte Dual Cure RadP-ROMP Formulierungen und Single Cure RadP Formulierungen (Tabelle 8) wurden getestet. Getestet wurden die Auszugskraft, die Scherfestigkeit, und die Gelzeit.

### 5.1: Herstellung der Proben

Die Zusammensetzung der Komponenten ist in Tabelle 8 gezeigt:

**Tabelle 8:**

| | **Bestandteil** | **Menge bezogen auf die gesamte Komponente** | **Anteil bezogen auf die Menge Mon13** |
|---|---|---|---|
| **Komponente A** | Mon13 | 31 Gew.-% | 100 Gew.-% |
| | Perkadox 20S | 9 Gew.-% | 30 Gew.-% |
| | Cab-O-Sil TS-720 | 3 Gew.-% | |
| | Quarzsand F32 | 37 Gew.-% | |
| | Millisil W12 | 20 Gew.-% | |
| Gesamt Komp. A | | 100 Gew.-%; ca. 300 ml | |
| | | | |
| **Komponente B** | M20 [nur bei Dual Cure] | 0,1 Gew.-% | 50 ppm |
| | BDDMA | 36 Gew.-% | 11,7 Gew.-% |
| | Tempol | 0,7 Gew.-% | 0,23 Gew.-% |
| | DMpT | 3,2 Gew.-% | 1,05 Gew.-% |
| | Cab-O-Sil TS-720 | 3 Gew.-% | |
| | Quarzsand F32 | 37 Gew.-% | |
| | Millisil W12 | 20 Gew.-% | |
| Gesamt Komp. B | | 100 Gew.-%; ca. 30 ml | |

Das jeweilige Monomer wurde mit Perkadox 20S in einem Speedmixer für 30 s vermischt. Die Füllstoffe wurden hinzugefügt und der Speedmixer wurde verwendet, um eine homogene Komponente A herzustellen. Komponente B wurde analog der Vorschrift in Beispiel 3 hergestellt, mit Speedmixerboxen statt Glasgefäßen. Die fertigen Komponenten wurden dann in die jeweilige Kammer von 2-Komponenten ("2K") 10+1-Kartuschen (Komponente A in die 10er-Kammer, Komponenten B in die 1er-Kammer) gefüllt. Diese wurden bei Raumtemperatur gelagert, außer für die Geltimer-Messung, wofür sie wenigstens eine Stunde bei konstant 25°C gelagert wurden. Die Formulierungen wurden mit einer Auspresspistole mit einer Statikmischer-Spitze vermischt und aufgebracht.

### 5.2: Auszugstests

Bohrlöcher (Durchmesser: 14 mm) in Beton (C20/25) wurden mit der Auspresspistole befüllt und eine Metall-Ankergewindestange (Durchmesser: 12 mm) wurde anschließend in 72 mm Tiefe (Einbindetiefe) hineingesteckt. Die Auszugstests wurden nach 48 h Aushärtungszeit durchgeführt, mit einer Auszugs-Vorrichtung F-100-4 von Kindsmüller mit einem Hoko 100 B Zylinder.

Zur Durchführung der Messungen wurden Ankerstangen mit Gewinde M8 mit folgender Geometrie verwendet:
Hinterschnitttiefe: 0,35 +/- 0,02 mm
Hinterschnittbreite: 2 mm

Die aus diesen Messungen ermittelte Scherfestigkeit ist definiert als der Quotient aus maximaler Kraft beim Versagen und der Scherfläche der verwendeten Ankerstange (ca. 2765 mm²).

Es wurde in trockenem ("F1ref") und feuchtem ("F1b") Beton getestet; pro getesteter Zusammensetzung wurden drei Bohrlöcher befüllt und getestet. Als Referenz-Systeme wurden das Epoxy-Amin-System HIT-RE 500 V1 und das radikalisch polymerisierte Methacrylat-System HIT-HY 200-A von Hilti verwendet.

Die Ergebnisse für Auszugskraft und Scherfestigkeit sind in Tabelle 9 gezeigt.

**Tabelle 9:**

| **System** | **Bohrloch-Zustand** | **Auszugskraft [kN]** | **Scherfestigkeit [N/mm²]** |
|---|---|---|---|
| RE 500 V1 (Epoxyamin) | F1ref | | 33,0 ± 1,5 |
| | F1b | | 28,0 ± 1,4 |
| HY 200-A (RadP) | F1ref | 90,5 ± 1,0 | 34,1 ± 0,5 |
| | F1b | 65,9 ± 6,9 | 24,5 ± 2,2 |
| Single Cure RadP Mon13 | F1ref | 38,2 ± 4,0 | 14,1 ± 1,5 |
| | F1b | 25,3 ± 2,1 | 9,0 ± 0,9 |
| Dual Cure RadP Mon13 | F1ref | 41,7 ± 4,5 | 15,3 ± 1,7 |
| | F1b | 46,5 ± 3,6 | 17,1 ± 1,3 |

Das Ergebnis zeigt, dass Single Cure RadP mit Mon13 und Dual Cure RadP-ROMP zwar nicht die Auszugskräfte und Scherfestigkeit der Referenz-Systeme erreichten; die Werte sind jedoch ausreichend für die Befestigung in Bohrlöchern. Zudem zeigt das Dual Cure-System einen wesentlichen Vorteil, der mit der schrumpfarmen ROMP zusammenhängen dürfte: die Gleichwertigkeit (oder sogar Verbesserung) der Werte von F1b gegenüber F1ref. Das bedeutet, dass dieses System gegenüber feuchten Bohrlöchern sehr robust ist. Das ist bei den anderen Rezepturen nicht der Fall, dort ist F1b stets deutlich kleiner als F1ref.

### 5.3: Geltimer-Messung

Die Geltimer-Messung wurde wie in Beispiel 4.2 beschrieben durchgeführt. Als Referenz diente wieder der chemische Dübel HIT-HY150 von Hilti. Die Ergebnisse zeigt Tabelle 10A:

**Tabelle 10A:**

| **System** | **ungefüllt/ gefüllt** | **Tempol [Gew.-%]** | **GTT_{+10°C} [min]** | **GTT_{50°C} [min]** | **GTM [min]** |
|---|---|---|---|---|---|
| Dual Cure RadP-ROMP Mon13 | ungefüllt | 0,35 | 5,83 | 6,65 | 6,35 |
| | gefüllt | | 4,78 | 5,53 | 5,00 |
| | | | 4,97 | 5,65 | 5,00 |
| HY150 | ungefüllt | 0,46 | 5,10 | 5,55 | 4,31 |
| | gefüllt | | 5,12 | 5,35 | 5,20 |

Diese Ergebnisse zeigen, dass die Gelzeit-Werte denen der Referenz vergleichbar sind.

### 5.3: Gelzeit-Messung mit einem Yokogawa-Sensor

Die Gelzeit in einem Single Cure-System wurde bei einer Umgebungstemperatur von 23°C mit einem Yokogawa-Sensor (einem Quarzglasfaser-gestörten Temperatursensor, angeschlossen an eine DAQstation D1006-3-4-2 von Yokogawa Electric Corporation) bestimmt. Als Referenz diente der chemische Dübel HIT-HY 200-A von Hilti. Die Ergebnisse zeigt Tabelle 10:

**Tabelle 10B:**

| **System** | **ungefüllt/ gefüllt** | **Tempol [Gew.-%]** | **GTT_{+10°C} [min]** | **GTT_{50°C} [min]** |
|---|---|---|---|---|
| Single Cure RadP Mon13 | gefüllt | 0,23 | 5,67 | 6,25 |
| HY 200-A | gefüllt | 0,46 | 7,55 | 7,90 |
| | | | 7,55 | 7,95 |

Diese Ergebnisse zeigen, dass die Gelzeit-Werte des Single Cure-Systems mit Mon13 denen der Referenz vergleichbar sind.

### Beispiel 6: Scherspannung und Schrumpf bei Verwendung von Mon13 und Mon14 als Monomer in HY 200-A

In einer HY 200-A-Formulierung von Hilti wurde das BDDMA, das ein Drittel des vermischten Systems ausmacht, durch Mon13 bzw. Mon14 ersetzt. Die fertigen Komponenten wurden dann in die jeweilige Kammer von 2-Komponenten ("2K") 10+1-Kartuschen (Komponente A in die 10er-Kammer, Komponenten B in die 1er-Kammer) gefüllt. Diese wurden bei Raumtemperatur gelagert. Die Formulierungen wurden mit einer Auspresspistole mit einer Statikmischer-Spitze vermischt und aufgebracht.

Die Scherspannung bei Raumtemperatur wurde mit einer Zugprüfmaschine Z050 von Zwick Roell ermittelt. Das Bohrloch wurde dabei durch eine Stahlhülse mit Innenbohrung vergleichbarer Rauigkeit wie in einem Betonbohrloch simuliert. Der Polymerisationsschrumpf wurde mit einer Distanzmessungs-Vorrichtung in einem Schwindkegel von Schleibinger Geräte, gekoppelt an einen deltaEL Lasercontroller für die Datensammlung, ermittelt, der online gemessene Schrumpf der Kegelhöhe wurde dabei automatisch auf Volumenschrumpf umgerechnet.

**Tabelle 11:**

| **System** | **Schrumpf [%]** | **Scherspannung [MPa]** |
|---|---|---|
| HY 200-A, unmodifiziert | 3,32 ± 0.08 | 28,08 ± 2,21 |
| HY 200-A mit Mon13 | 2,85 ± 0.28 | 29,79 ± 1,5 |
| HY 200-A mit Mon14 | 2,85 ± 0.12 | 24,4 ± 0,9 |

Beide Systeme, in denen BDDMA durch ein RadP-ROMP-Monomer ersetzt wurde, zeigten weniger Schrumpf als das unmodifizierte Referenzprodukt HY 200-A. Die Scherspannung war jedoch in einer vergleichbaren Größenordnung. Dies weist darauf hin, dass die Norbornen-Struktureinheit im RadP-ROMP-Monomer an der radikalischen Polymerisation teilnimmt. Wäre sie nicht beteiligt, so wäre wegen der unreagierten Norbornen-Doppelbindung mit einer verringerten Scherspannung zu rechnen.

## Patentansprüche

1. Eine Verbindung umfassend
(i) ein Strukturelement, welche ein gespanntes Cycloolefin ist, und
(ii) ein Strukturelement, welches ein Ester einer α,β-ungesättigten Carbonsäure ist.

2. Die Verbindung gemäß Anspruch 1, wobei das gespannte Cycloolefin ein monocyklisches oder bicyklisches C₃-C₁₆-Cycloolefin mit einer oder zwei Doppelbindungen ist.

3. Die Verbindung gemäß Anspruch 2, wobei das gespannte Cycloolefin ausgewählt ist aus der Gruppe bestehend aus Norbornen, Norbornadien, Tricyclo[5.2.1.0^{2,6}]deca-3,8-dien (Dicyclopentadien), Tetracyclo[6.2.1.1.0]-dodecen, und aus deren Oxa-Derivaten, in welchen ein C-Atom im gespannten Cycloolefin-Strukturelement durch O ersetzt ist.

4. Die Verbindung gemäß Anspruch 3, wobei das gespannte Cycloolefin ein Norbornen ist.

5. Die Verbindung gemäß irgendeinem der Ansprüche 1 bis 4, wobei die α,β-ungesättigte Carbonsäure ausgewählt ist aus der Gruppe bestehend aus verzweigten und unverzweigten C₃-C₂₀-α,β-ungesättigten Carbonsäuren, bevorzugter aus der Gruppe bestehend aus verzweigten und unverzweigten C₄-C₆-α,β-angesättigten Carbonsäuren.

6. Die Verbindung gemäß irgendeinem der Ansprüche 1 bis 4, welche die folgende Formel (III) hat: 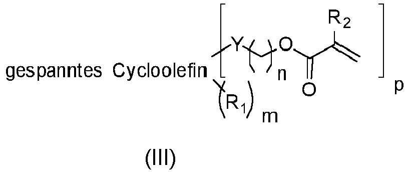 worin:
- Y ausgewählt ist aus der Gruppe bestehend aus einer Einfachbindung, -C₁-C₂₀-Alkandiyl-, -O-C₁-C₂₀-Alkandiyl-, -C₁-C₂₀-Alkandiyl-O-, -O-C₁-C₂₀-Alkandiyl-O-, -O-,-Si(Alkyl)₂-, -C₁-C₂₀-Alkandiyl-Si(Alkyl)₂-, -C₁-C₂₀-Alkandiyl-Si(Alkyl)₂-C₁-C₂₀-Alkandiyl-,-C₁-C₂₀-Alkandiyl-O-C₁-C₂₀-Alkandiyl-, -C(=O)-O-, -C₁-C₂₀-Alkandiyl--C(=O)-O-,-C(=O)-O-C₁-C₂₀-Alkandiyl-, -C(=O)-O-C₁-C₂₀-Alkandiyl-O-C(=O)-, -C₁-C₂₀-Alkandiyl-C(=O)-O-C₁-C₂₀-Alkandiyl-, -O-C(=O)-, -O-C(=O)-O-, -C₁-C₂₀-Alkandiyl-O-C(=O)-,-O-C(=O)-C₁-C₂₀-Alkandiyl-, -O-C(=O)-C₁-C₂₀-Alkandiyl-C(=O)-O-, -Aryldiyl-, -O-Aryldiyl-, -O-Aryldiyl-O-, -Aryldiyl-O-Aryldiyl-,-C(=O)-O-Aryldiyl-, -C(=O)-O-Aryldiyl-O-C(=O)-, -Aryldiyl-C(=O)-O-Aryldiyl-,-O-C(=O)-Aryldiyl-, -O-C(=O)-Aryldiyl-C(=O)-O-, -C₆-C₂₀-Arylalkandiyl-O-C₆-C₂₀-Arylalkandiyl-, -O-C₆-C₂₀-Arylalkandiyl-O-, -C₆-C₂₀-Arylalkandiyl-O-C₆-C₂₀-Arylalkandiyl-, -C(=O)-O-C₆-C₂₀-Arylalkandiyl-, -C(=O)-O-C₆-C₂₀-Arylalkandiyl-O-C(=O)-, -C₆-C₂₀-Arylalkandiyl-C(=O)-O-C₆-C₂₀-Arylalkandiyl-, -O-C(=O)-C₆-C₂₀-Arylalkandiyl-, -O-C(=O)-C₆-C₂₀-Arylalkandiyl-C(=O)-O-, -(CH₂)_{q}-C=C-(CH₂)_{q}-, worin q unabhängig voneinander eine ganze Zahl von 1 bis 20, bevorzugt von 1 bis 10, noch bevorzugter von 1 bis 2 sind, -(CH₂)_{q}-O-(CH₂)_{q}-, worin q unabhängig voneinander eine ganze Zahl von 1 bis 20, bevorzugt von 1 bis 10, noch bevorzugter von 1 bis sind, -(CH₂)_{q}-NR₄-(CH₂)_{q}-, worin q unabhängig voneinander eine ganze Zahl von 1 bis 20, bevorzugt von 1 bis 10, noch bevorzugter von 1 bis sind, und R₄ H oder ein substituiertes oder unsubstituiertes C₁-C₂₀-Alkyl ist, bevorzugter H oder ein unsubstituiertes C₁-C₂₀-Alkyl ist, noch bevorzugter H ist;
- m eine ganze Zahl von 0 bis 2 ist, bevorzugt 0 oder 1 ist; wenn m 2 ist, dann sind die beiden R₁ bevorzugt gleich;
- n eine ganze Zahl von 1 bis 10 ist, bevorzugt von 2 bis 6 ist, bevorzugter 2 ist;
- p eine ganze Zahl von 1 bis 2 ist, bevorzugt 1 ist;
- R₁ unabhängig voneinander ausgewählt sind aus der Gruppe bestehend aus -C(=O)-OH, -C(=O)-O-C₁-C₂₀-Alkyl, -O-C(=O)-C₁-C₂₀-Alkyl, -C(=O)-O-Aryl, -O-C(=O)-Aryl,-C₁-C₂₀-Alkyl, -Aryl, -C₁-C₂₀-Hydroxyalkyl, -C₁-C₂₀-Alkandiyl-O-C₁-C₂₀-Alkyl, Halogenyl, -CF₃, -CF₂-CF₃, und -SiAlkyl₃; wobei zwei an nebeneinanderliegenden Kohlenstoffatome gebundene R₁ miteinander verknüpft sein und so mit den Kohlenstoffatomen, an welche sie gebunden sind, einen Ring bilden können, bevorzugt einen Ring mit 5 bis 7 Ringatomen; oder wobei zwei R₁ zusammen eine Brücke der Formel -(CH₂)_{z}- bilden welche zwei Cycloolefin-Strukturelemente miteinander verbindet, worin z eine ganze Zahl von 1 bis 4, bevorzugt von 1 bis 3, bevorzugter von 1 bis 2, besonders bevorzugt 1 ist, oder eine Brücke der Formel -(CH₂)_{q}-Ar-(CH₂)_{q}- welche zwei Cycloolefin-Strukturelemente miteinander verbindet, worin Ar ein Aromat ist, bevorzugt Phenyl, q unabhängig voneinander eine ganze Zahl von 0 bis 2, bevorzugt von 0 bis 1, noch bevorzugter beide 0 sind, oder eine Brücke der Formel - (CH₂)_{q}-C=C-(CH₂)_{q}- welche zwei Cycloolefin-Strukturelemente miteinander verbindet, worin q unabhängig voneinander eine ganze Zahl von 0 bis 2, bevorzugt von 0 bis 1, noch bevorzugter beide 0 sind, oder eine Brücke der Formel -(CH₂)_{q}-O-(CH₂)_{q}- welche zwei Cycloolefin-Strukturelemente miteinander verbindet, worin q unabhängig voneinander eine ganze Zahl von 0 bis 2, bevorzugt von 0 bis 1, noch bevorzugter beide 0 sind, oder eine Brücke der Formel -(CH₂)_{q}-NR₄-(CH₂)_{q}- welche zwei Cycloolefin-Strukturelemente miteinander verbindet, worin q unabhängig voneinander eine ganze Zahl von 0 bis 2, bevorzugt von 0 bis 1, noch bevorzugter beide 0 sind, und R₄ H oder ein substituiertes oder unsubstituiertes C₁-C₂₀-Alkyl ist, bevorzugter H oder ein unsubstituiertes C₁-C₂₀-Alkyl ist, noch bevorzugter H ist, oder eine Brücke der Formel -C(=O)-O-(CH₂)_{z}-O-(O=)-C- welche zwei Cycloolefin-Strukturelemente miteinander verbindet, worin z eine ganze Zahl von 1 bis 4, bevorzugt von 1 bis 3, bevorzugter von 1 bis 2, besonders bevorzugt 2 ist, oder eine Brücke der Formel - C(=O)-O-, oder eine Brücke der Formel -C(=O)-O-Ar-O-(O=)-C- welche zwei Cycloolefin-Strukturelemente miteinander verbindet, worin Ar ein Aromat, bevorzugt Phenyl ist, z eine ganze Zahl von 1 bis 4, bevorzugt von 1 bis 3, bevorzugter von 1 bis 2, besonders bevorzugt 1 ist, oder eine Brücke der Formel -(CH₂)_{q}-Si(R₄)₂-(CH₂)_{q}- welche zwei Cycloolefin-Strukturelemente miteinander verbindet, worin q unabhängig voneinander eine ganze Zahl von 0 bis 2, bevorzugt von 0 bis 1, noch bevorzugter mindestens einmal 1 sind, und R₄ H oder ein substituiertes oder unsubstituiertes C₁-C₂₀-Alkyl ist, bevorzugter H oder ein unsubstituiertes C₁-C₂₀-Alkyl ist, noch bevorzugter ein unsubstituiertes C₁-C₄-Alkyl ist;
- R₂ ein C₁-C₁₇-Alkyl oder C₁-C₁₇-Hydroxyalkyl ist.

7. Die Verbindung gemäß Anspruch 6, worin
- gespanntes Cycloolefin ausgewählt ist aus der Gruppe bestehend aus Cyclopenten, Norbornen, Norbornadien, Cycloocten, Tetracyclo[6.2.1.1.0]-dodecen und aus deren Oxa-Derivaten, in welchen ein C-Atom im gespannten Cycloolefin-Strukturelement durch O ersetzt ist;
- Y ausgewählt ist aus der Gruppe bestehend aus einer Einfachbindung, -C₁-C₈-Alkandiyl-, -O-C₁-C₈-Alkandiyl-, -C₁-C₈-Alkandiyl-O-, -O-C₁-C₈-Alkandiyl-O-, -C₁-C₈-Alkandiyl-O-Ci-Cs-Alkandiyl-, -C(=O)-O-, -C₁-C₈-Alkandiyl-C(=O)-O-, -C(=O)-O-C₁-C₈-Alkandiyl-, -C(=O)-O-C₁-C₈-Alkandiyl-O-C(=O)-, -C₁-C₈-Alkandiyl-C(=O)-O-C₁-C₈-Alkandiyl-, -O-C(=O)-, -C₁-C₈-Alkandiyl-O-C(=O)-, -O-C(=O)-C₁-C₈-Alkandiyl-, -O-C(=O)-C₁-C₈-Alkandiyl-C(=O)-O-, -C₆-C₁₀-Aryldiyl-, -O-C₆-C₁₀-Aryldiyl-;
- m 0 oder 1 ist;
- n eine ganze Zahl von 2 bis 6 ist, bevorzugter 2 ist;
- p eine ganze Zahl von 1 bis 2 ist, bevorzugt 1 ist;
- R₁ ausgewählt ist aus der Gruppe bestehend aus -C(=O)-OH, -C(=O)-O-C₁-C₄-Alkyl, -O-C(=O)-C₁-C₄-Alkyl, -C₁-C₄-Alkyl, -CH₂-OH, und -CH₂-O-C₁-C₄-Alkyl; oder zwei R₁ bilden zusammen eine Brücke der Formel -(CH₂)_{z}- welche zwei Cycloolefin-Strukturelemente miteinander verbindet, worin z eine ganze Zahl von 1 bis 2, bevorzugt 1 ist, oder eine Brücke der Formel -(CH₂)_{q}-C=C-(CH₂)_{q}- welche zwei Cycloolefin-Strukturelemente miteinander verbindet,, worin q unabhängig voneinander eine ganze Zahl von 0 bis 1, bevorzugter beide 0 sind, oder eine Brücke der Formel -(CH₂)_{q}-O-(CH₂)_{q}- welche zwei Cycloolefin-Strukturelemente miteinander verbindet, worin q unabhängig voneinander eine ganze Zahl von 0 bis 2, bevorzugt von 0 bis 1, noch bevorzugter beide 0 sind, oder eine Brücke der Formel -C(=O)-O-(CH₂)_{z}-O-(O=)-C-welche zwei Cycloolefin-Strukturelemente miteinander verbindet, worin z eine ganze Zahl von 1 bis 4, bevorzugt von 1 bis 3, bevorzugter von 1 bis 2, besonders bevorzugt 1 ist, oder eine Brücke der Formel -C(=O)-O-Phenyl-O-(O=)-C- welche zwei Cycloolefin-Strukturelemente miteinander verbindet, oder eine Brücke der Formel-(CH₂)_{q}-Si(R₄)₂-(CH₂)_{q}- welche zwei Cycloolefin-Strukturelemente miteinander verbindet, worin q unabhängig voneinander eine ganze Zahl von 0 bis 1, noch bevorzugter mindestens einmal 1 sind, und R₄ H oder ein substituiertes oder unsubstituiertes C₁-C₄-Alkyl ist, bevorzugter H oder ein unsubstituiertes C₁-C₄-Alkyl ist, noch bevorzugter ein unsubstituiertes C₁-C₄-Alkyl ist;
- R₂ ausgewählt ist aus der Gruppe bestehend aus Methyl, Ethyl, n-Propyl, iso-Propyl, n-Butyl, iso-Butyl, und tert-Butyl, 2-Hydroxyethyl, 3-Hydroxy-n-propyl, 2-Hydroxyisopropyl, 4-Hydroxy-n-Butyl, 3-Hydroxy-isobutyl, und 2-Hydroxy-tert-butyl.

8. Die Verbindung gemäß Anspruch 6 oder 7, welche ausgewählt ist aus Verbindungen mit den Formeln (IV), (V), oder (VI): 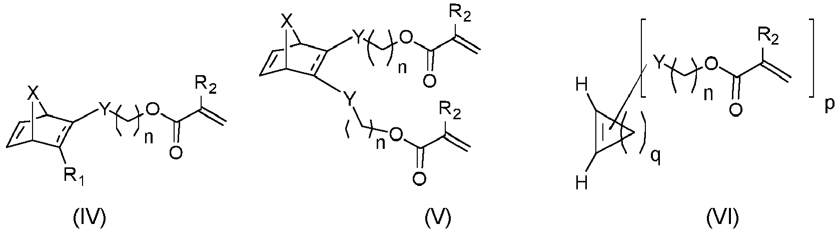 worin:
- --- eine Einfach- oder Doppelbindung, bevorzugt eine Einfachbindung ist;
- Y, n, R₁ und p wie in Anspruch 6, bevorzugt wie in Anspruch 7 definiert sind;
- X -CH₂- oder -O-, bevorzugt -CH₂- ist;
- q eine ganze Zahl ausgewählt aus der Gruppe bestehend aus 1, 2, 3, 5 und 6, bevorzugt ausgewählt aus 1, 3 und 5, besonders bevorzugt 3 ist, wobei p kleiner oder gleich q ist.

9. Die Verbindung gemäß Anspruch 6 oder 7, welche ausgewählt ist aus Verbindungen mit den Formeln (VII), (VIII), oder (IX): 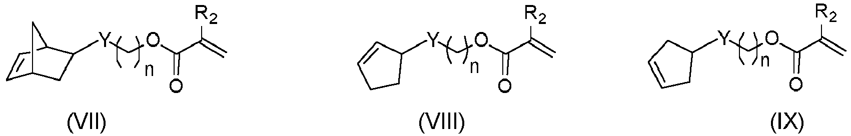 worin:
Y, n und R₂ wie in Anspruch 6, bevorzugt wie in Anspruch 7 definiert sind.

10. Die Verbindung gemäß Anspruch 9, welche die Formel (X) hat: 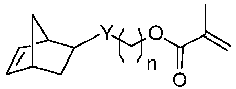 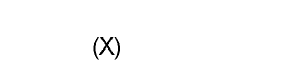 worin:
Y und n wie in Anspruch 6, bevorzugt wie in Anspruch 7 definiert sind.

11. Verwendung der Verbindung gemäß irgendeinem der Ansprüche 1 bis 10 als Monomer für die Polymerisation.

12. Ein Zweikomponenten-System für die Herstellung eines Polymers umfassend:
eine Komponente A umfassend
ein Monomer, welches eine Verbindung gemäß einem der Ansprüche 1 bis 10 ist, und
optional einen Radikalbeschleuniger, und
eine Komponente B umfassend
einen Radikalinitiator.

13. Ein Zweikomponenten-System für die Herstellung eines Polymers umfassend:
eine Komponente A umfassend
ein Monomer, welches eine Verbindung gemäß einem der Ansprüche 1 bis 10 ist, und
einen Radikalinitiator, und
eine Komponente B umfassend
ein Methacrylat,
einen Katalysator für Ring Opening Metathese Polymerisation (ROMP-Initiator), und
einen Radikalbeschleuniger.

14. Das Zweikomponenten-System gemäß Anspruch 12, wobei
(a) der ROMP-Initiator ein Grubbs-Katalysator ist; und/oder
(b) der Radikalinitiator ein Peroxid ist; und/oder
(c) das Methacrylat in Komponente B ausgewählt ist aus Methylmethacrylat, HEMA und BDDMA, und bevorzugt BDDMA ist; und/oder
(d) der Radikalbeschleuniger ein aromatisches Amin ist, bevorzugt ein aromatisches Amin ausgewählt aus der Gruppe bestehend aus *N,N*-Di-*iso*-propanol-*p*-toluidin (DiPpT), *N,N*-Dimethyl-p-Toluidin (DMpT) und *N,N-*Diethyl-p-Toluidin (DEpT).

15. Das Zweikomponenten-System gemäß irgendeinem der vorhergehenden Ansprüche 12 bis 14, wobei mindestens eine der Komponenten A oder B mindestens einen Füllstoff enthält, wobei der Füllstoff bevorzugt aus der Gruppe bestehend aus Quarz, Glas, Korund, Porzellan, Steingut, Leichtspat, Schwerspat, Gips, Talk, Kreide oder Gemischen davon ausgewählt ist, und wobei der Füllstoff in Form von Sand, Mehl, oder Formkörpern vorliegen kann.

16. Verwendung eines Zweikomponenten-Systems gemäß irgendeinem der Ansprüche 12 bis 15 zur Befestigung eines Verankerungsmittels in einem Bohrloch.
